# EUROPEAN PATENT SPECIFICATION

(11) **EP 1 853 581 B1**
(45) Date of publication and mention of the grant of the patent: **02.06.2010**
(21) Application number: 06723133.2
(22) Date of filing: 27.02.2006
(51) Int. Cl.: C07D 401/04, A61K 31/435, A61P 29/00

(54) **2-SULFINYL- AND 2-SULFONYL-SUBSTITUTED IMIDAZOLE DERIVATIVES AND THEIR USE AS CYTOKINE INHIBITORS**
2-SULFINYL- UND 2-SULFONYL-SUBSTITUIERTE IMIDAZOLDERIVATE UND DEREN VERWENDUNG ALS CYTOKININHIBITOREN
DÉRIVÉS D IMIDAZOLE DE 2-SULFINYLE ET DE 2-SULFONYLE ET LEUR UTILISATION COMME INHIBITEURS DE CYTOKINES

(30) Priority: 28.02.2005 EP 05004369; 28.02.2005 US 656389 P
(43) Date of publication of application: 14.11.2007
(73) Proprietor: MERCKLE GMBH, 89079 Ulm (DE)
(72) Inventor: ALBRECHT, Wolfgang, 89075 Ulm (DE); GREIM, Cornelia, 73235 Weilheim/Teck (DE); STRIEGEL, Hans-Günter, 89143 Blaubeuren (DE); TOLLMANN, Karola, 65611 Brechen (DE); MERCKLE, Philipp, 89143 Blaubeuren-Weiler (DE); LAUFER, Stefan, 72070 Tübingen (DE)
(74) Representative: Reitstötter - Kinzebach
(86) International application number: PCT/EP2006/001801
(87) International publication number: WO 2006/089798

(56) References cited:
- WO-A-02/066458
- WO-A-03/097633
- LAUFER ET AL.: "Tetrasubstituted Indazole inhibitors of Cytokine Release: probling Substituents in the N-1 Position" J. MED. CHEM., vol. 47, 2004, pages 6311-6325, XP002382670
- LAUFER ET AL.: "Novel substituted Pyridinyl imidazoles as Potent Anticytokine Agents with Low Activity against Hepatic Cytochrome P450 Enzymes" J. MED. CHEM., vol. 46, 2003, pages 3230-3244, XP002382671
- WAGNER ET AL.: "Identification of Regioisomers in a Series of N-Substituted Pyridin-4-yl Imidazole Derivatives by Regiospecific Synthesis, GC/MS, and 1H NMR" J. ORG. CHEM., vol. 68, 2003, pages 4527-4530, XP002382672

## Description

The present invention relates to 2-sulfinyl- and 2-sulfonyl-substituted imidazole derivatives having an immunomodulating and cytokine release-inhibiting effect, to pharmaceutical compositions which comprise the compounds, and to these compounds for use in pharmacy.

Pharmacologically active imidazole compounds having antiinflammatory activity are known, see GB 1,155,580; US 4,585,771; EP 236 628 A; EP 372 445 A; US 4,355,039; US 5,364,875; US 4,190,666; WO 02/076 951, WO 9113876; GB 1,564,184; JP 64-40467; WO 88/01167; WO 96/03387; J. Med. Chem. 1995, 38, 1067-1083; Acta Chim. 1969, 61(1), 69-77; and J. Med. Chem. 1999, 2180-2190.

Very diverse pharmaceutical effects have been described for 2-thioimidazole compounds having 4,5-diaryl and 4(5)-(heteroaryl)arylimidazole elements. Similar is also true of compounds related thereto having a substitution on N1 and/or C2 on the imidazole ring.

EP 0 043 788 A (US 4,528,298 and US 4,402,960) describe 4,5-di(hetero)aryl-imidazole derivatives which are substituted at position 2 via a thio or sulfinyl or sulfonyl group by a phenyl, pyridyl, N-oxypyridyl, pyrimidyl, thiazolyl or thienyl radical and have an antiinflammatory and antiallergic activity.

WO 00/17192 (and Angew. Chem. Int. Ed. 2002, 41, 2290-2291) relates to 4-heteroaryl-5-phenylimidazole derivatives which are substituted at position 2 by a phenylalkylthio group. They have no N1 substituent and exist in 2 tautomers. These compounds act as an antiinflammatory agent and inhibitor of cytokine release. In the compounds described in DE 35 04 678 there are sulfur-linked alkanecarboxylic acid residues in position 2 of the 1,4,5-triaryl-substituted imidazole. The 4-heteroaryl-5-phenylimidazoles described in WO 99/03837 have functionalized and nonfunctionalized alkanes, which are also linked via sulfur atoms, at position C2, and carbonyl-linked radicals in position N1.

WO 93/14081 describes 2-substituted imidazoles which inhibit the synthesis of a number of inflammatory cytokines. The compounds described in WO 93/14081 have a phosphorus-containing substituent linked via a sulfur atom, or an aryl or heteroaryl substituent in position 2. US 5,656,644 describes similar compounds. WO 91/10662 describes imidazole derivatives which inhibit acyl-coenzyme A: cholesterol 0-acyltransferase and the binding of thromboxane TxA₂. WO 95/00501 describes imidazole derivatives which can be used as cyclooxygenase inhibitors. The imidazole J. Med. Chem. 1996, 39, 3927-37 describes compounds having a 5-lipoxygenase- and cyclooxygenase-inhibiting effect, with 2-(4-methylsulfinylphenyl)-4-(4-fluorophenyl)-5-(pyrid-4-yl)imidazole also having a cytokine-inhibiting effect.
In addition, EP 004 648 and the corresponding US 4,461,770, US 4,584,310 and US 4,608,382 disclose 2-alkylthio-, 2-alkylsulfinyl and 2-alkylsulfonyl- and N1-alkylsubstituted imidazole derivatives which have in position 4 and 5 in each case a heteroaryl radical (preferably 3-pyridyl and 2-thienyl) combined with an aryl radical which is then located in the respective other ring position (preferably phenyl and 4-fluorophenyl). These compounds have an antiinflammatory effect and antinociceptive activity (rat paw edema and mouse phenylquinone writhing test) in the dose range 50-200 mg/kg orally and 100 mg/kg orally, respectively. The compounds inhibit prostaglandin synthesis from arachidonic acid (cyclooxygenase/5-lipoxygenase inhibition according to Prostaglandins 7, 123 (1974)) in the range 10 - 30 mg/L (10⁻⁴ to 10⁻⁵ M).

WO 04/018458 A1 describes 2-thio-, 2-sulfinyl- and 2-sulfonyl-substituted imidazole compounds having a cytokine-inhibiting effect which are unsubstituted on N1. The compounds substituted on N1 which are disclosed in WO 02/066458 A2 show an in vitro activity, which is improved compared with the prior art, on the main pharmacological target, the p38 MAP kinase alpha. Besides the P38 Map kinase alpha, prior art compounds influence further kinases of the cellular signal transduction cascade, e.g. the isoenzymes of p38 MAP kinase, extracellular receptor kinases, apoptotic kinases and cell cycle-regulating kinases. WO 02/066458 A2 and WO 03/097633 describe 2-thio-substituted, N1-substituted imidazole compounds having a cytokine-inhibiting effect which inhibit P38 MAP kinase alpha with high selectivity and moreover exert a smaller influence on cytochrome P450 enzyme systems. In cell assays (isolated PMNLs), the compounds show an activity which is improved by comparison with the prior art in relation to the suppression of release of the proinflammatory cytokines TNFα and IL1β after stimulation with lipopolysaccharides. However, these compounds have proved to be relatively toxic.

J. Med. Chem. 2003, 46, 3230-3244 discloses a series of polysubstituted pyridine-4-yl imidazole inhibitors of p38 MAP (mitogen-activated protein) kinase which are useful for the treatment of chronic inflammatory diseases. J. Med. Chem. 2004, 47, 6311-6325 discloses tetrasubstituted imidazole derivatives with high anti-inflammatory activity. J. Org. Chem. 2003, 68, 4527-4530 discloses a series of N-substituted pyridine-4-yl imidazole derivatives having a strong anti-inflammatory effect

Despite the numerous known compounds, therefore, there continues to be a need for compounds having an antiinflammatory effect which inhibit cytokine release and show low toxicity.

The object of the invention is to provide such compounds.

It has now surprisingly been found that certain thio-imidazole derivatives which have a sulfinyl or sulfonyl substituent in position 2 show antiinflammatory properties with, at the same time, improved solubility of their free bases and of their acid addition compounds compared with the non-oxidized bases and salts of the sulfanyl compounds. Their absorbability in vivo is improved and they are stable compounds which are easy to process and which show in vivo a high immunomodulating and/or cytokine release-inhibiting activity and overall improved pharmacokinetics. In addition, they are less toxic and therefore lead to less stress on all drug-metabolizing enzyme systems.

The present invention therefore relates to the 2-sulfinyl- and 2-sulfonyl-substitututed imidazole compounds of the formula I 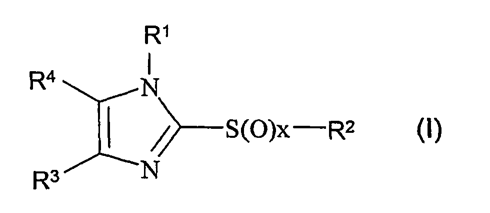 in which
R¹ is selected from:
a) C₁-C₆-alkyl which is optionally substituted by one or two groups independently of one another selected from
   hydroxy;
   C₁-C₄-alkoxy;
   C₂-C₆-alkenyloxy;
   C₂-C₆-alkynyloxy;
   CO₂H;
   CO₂-C₁-C₆-alkyl;
   CN;
   halogen;
   C₁-C₆-alkyl- SO₃;
   C₁-C₆-alkylthio;
   NR⁷R⁸, wherein R⁷, and R⁸ are independently of one another H, C₁-C₆-alkyl or hydroxy-C₁-C₆-alkyl;
   R⁹CONR¹⁰, R⁹ and R¹⁰ are independently of one another H or C₁-C₆-alkyl;
   a nonaromatic heterocyclic radical having 5 or 6 ring atoms and 1 or 2 heteroatoms, selected independently of one another from N, O and S, which heterocyclic radical may be substituted by 1, 2, 3 or 4 C₁-C₆-alkyl groups;
b) 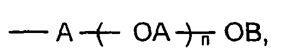 in which
   A is -CH₂CH₂-, -CH₂CH₂CH₂-, 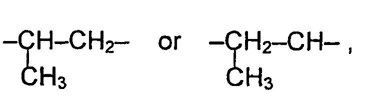 n is 1, 2, 3, 4 or 5, and B is H or C₁-C₄-alkyl;
c) C₁-C₆-oxoalkyl;
d) C₂-C₆-alkenyl
e) C₃-C₇-cycloalkyl;
f) (C₃-C₇-cycloalkyl)-C₁-C₆-alkyl;
g) aryl which is optionally substituted by one or more halogen atoms or a C₁-C₄-alkylsulfanyl group;
h) aminoaryl, where the amino group is optionally substituted by one or two C₁-C₄-alkyl groups,
i) aryl-C₁-C₆-alkyl or
j) an aromatic or nonaromatic heterocyclic radical having 5 or 6 ring atoms and 1 or 2 heteroatoms selected independently of one another from N, O and S, which heterocyclic radical is optionally substituted by 1, 2, 3 or 4 C₁-C₄-alkyl groups, an aryl or aryl-C₁-C₄-alkyl group;
R² is selected from:
a) C₁-C₆-alkyl,
b) phenyl-C₁-C₄-alkyl, where the phenyl group may have one or two substituents which are selected independently of one another from C₁-C₄-alkyl, halogen, C₁-C₄-alkylsulfanyl, C₁-C₄-alkylsulfinyl and C₁-C₄-alkylsulfonyl,
c) C₂-C₆-alkenyl,
d) C₂-C₆-alkenyl which is substituted by one or two halogen atoms and/or phenyl groups, where the phenyl group may be substituted independently by one or two C₁-C₄-alkyl or halogen atoms,
e) C₂-C₆-alkynyl,
f) C₂-C₆-alkynyl which is substituted by a phenyl group which may be optionally substituted by one or two C₁-C₄-alkyl or halogen atoms,
g) C₁-C₆-alkyl which is substituted by C₁-C₄-alkylsulfanyl, C₁-C₄-alkylsulfinyl or C₁-C₄-alkylsulfonyl;
h) C₁-C₆-alkyl which is substituted by -CO-Het wherein Het is a nonaromatic heterocyclic radical having 5 or 6 ring atoms and 1 or 2 heteroatoms which are selected independently of one another from N, O, and S, or C₁-C₄-alkylsulfanyl, C₁-C₄-alkylsulfinyl or C₁-C₄-alkylsuffonyl;
i) phenyl; and
j) phenyl which has one or two substituents which are selected independently of one another from C₁-C₄-alkyl, halogen, C₁-C₄-alkylsulfanyl, C₁-C₄-alkylsulfinyl or C₁-C₄-alkylsulfonyl; or
R¹ and R² together are -CH₂CH₂- or -CH₂CH₂CH₂-,
x is 1 or 2,
R³ is phenyl which is substituted by 1 or 2 halogen atoms or trifluoromethyl groups,
R⁴ is 4-pyridyl which has one or two substituents which are selected independently of one another from
a) amino;
b) C₁-C₈-alkylamino;
c) phenylamino, where the phenyl group may be substituted by C₁-C₄-alkyl, C₁-C₄-alkoxy, halogen, or CF₃;
d) phenyl-C₁-C₄-alkylamino;
e) C₃-C₇-cycloalkylamino;
f) (C₃-C₇-cycloalkyl)-C₁-C₈-alkylamino; and
g) R⁵CONR⁶-, wherein R⁵ is selected from
   H;
   C₁-C₈-alkyl;
   phenyl which may have one or two substituents which are selected independently of one another from C₁-C₄-alkyl, C₁-C₄-alkoxy and halogen;
   C₃-C₇-cycloalkyl;
   CF₃;
   C₂-C₆-alkenyl;
   phenyl-C₁-C₈-alkyl wherein the phenyl group may have one or two substituents which are selected independently of one another from C₁-C₄-alkyl, C₁-C₄-alkoxy or halogen;
   phenyl-C₂-C₆-alkenyl wherein the phenyl group may have one or two substituents which are selected independently of one another from C₁-C₄-alkyl, C₁-C₄-alkoxy or halogen; and
   phenyl-NR¹¹-, wherein R¹¹ is H or C₁-C₄-alkyl and the phenyl group may have one or two substituents which are selected independently of one another from C₁-C₄-alkyl, C₁-C₄-alkoxy or halogen;
R⁶ is H, C₁-C₄-alkyl, phenyl or benzyl, and
the optical isomers and physiologically tolerated salts thereof.

If the compounds of the invention have centers of asymmetry, then racemates and optical isomers (enantiomers, diastereomers and enriched forms thereof) are included. In particular, compounds in which R¹ or R² is 1-phenylethyl or R⁴ is substituted by 1-phenylethylamino or by R⁵CONR⁶ wherein R⁵ is 1-phenylethyl may exist as racemate (R,S) or enantiomers [(R) or (S)].

The sulfinyl compounds of formula I have an asymmetric center at the sulfur atom. They are obtained in the form of mixtures of the optical antipodes (racemates) which can be separated into the enantiomers by conventional methods. If further centers of asymmetry are present in the molecule, mixtures of diastereomers are formed in the oxidation and can be separated by conventional methods into the individual compounds. Conventional methods for separating said racemates and diastereomers are, e.g., fractional crystallization or chromatographic methods. The enantiomers are separated preferably by methods of adsorption chromatography on chiral supports, e.g. on modified methylstarches or methylcelluloses (Chiralcel).

The invention includes the racemates, diastereomers and the specific enantiomers and any enriched forms thereof.

The term "alkyl" (also in other groups such as phenylalkyl, alkylsulfonyl etc.) includes straight-chain and branched alkyl groups having preferably 1 to 6 or 1 to 4 C atoms, such as methyl, ethyl, n- and i-propyl, n-, i- and t-butyl, sec-butyl, n-pentyl and n-hexyl.

The term "C₁-C₆-oxoalkyl" means an alkyl group which includes a carbonyl group either in the carbon chain (ketone) or at the end thereof (aldehyde).

The term "aryl" includes aromatic ring systems such as phenyl or naphthyl.

The term "halogen" stands for a fluorine, chlorine, bromine or iodine atom, in particular a fluorine or chlorine atom.

C₃-C₇-Cycloalkyl groups are cyclopropyl, cyclobutyl, cycloheptyl and in particular cyclopentyl and cyclohexyl.

The term "alkenyl" (also in other groups such as "alkenyloxy" means a straight-chain or branched alkenyl group having 2 to 6 carbon atoms and a carbon-carbon double bond such as vinyl or allyl.

"Phenylalkenyl" is in particular styryl.

The term "alkynyl" (also in other groups such as "alkynyloxy" means a straight-chain or branched alkynyl group having 2 to 6 carbon atoms and a carbon-carbon triple bond such as acetylenyl or propargyl.

The aromatic or nonaromatic heterocyclic radicals in the compounds of the present invention have 5 or 6 ring atoms. 1 or 2 of said ring atoms are heteroatoms selected from O, N and S.

Nonaromatic heterocyclic radicals-may be saturated or unsaturated. Pyrrolidinyl, piperidinyl, piperazinyl, pyranyl, tetrahydrofuranyl or morpholinyl are preferred. The piperidinyl radical may be substituted by 1, 2, 3 or 4 C₁-C₄-alkyl groups, in particular methyl groups. A preferred piperidinyl radical is 2,2,6,6-tetramethylpiperidinyl.

Preferred aromatic heterocyclic radicals are pyridyl, especially 3- or 4-pyridyl, pyrimidinyl, pyrrolyl, imidazolyl, pyrazolyl, oxazolyl, isoxazolyl, furyl, thienyl or thiazolyl. The heterocyclic radical may be substituted as indicated above.

Phenyl-C₁-C₄-alkyl means in particular benzyl, 1-phenylethyl or 2-phenylethyl.

If R¹ is C₁-C₆-alkyl which is substituted by a nonaromatic heterocyclic radical, the latter preferably comprises at least one nitrogen atom, and the linkage to the alkyl group preferably takes place via the nitrogen atom. Preferred heterocyclic radicals are piperidinyl, 1,1,6,6-tetramethyl piperidinyl or morpholinyl.

If R¹ is an aromatic or nonaromatic heterocyclic radical, it is preferably linked via a carbon atom to the imidazole group. Preferred nonaromatic heterocyclic radicals are piperidinyl or piperidinyl which is substituted at the N-atom with C₁-C₄-alkyl or OCO-C₁-C₄-alkyl.

R¹ is preferably:
C₁-C₆-alkyl which is optionally substituted by one or two hydroxy or C₁-C₄-alkoxy groups or by a nonaromatic heterocyclic radical having 5 or 6 ring atoms and 1 or 2 heteroatoms which are selected independently of one another from N, O and S, 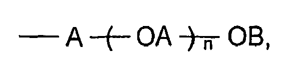 in which
   A is -CH₂CH₂-, -CH₂CH₂CH₂-, 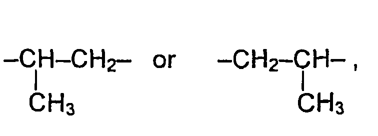
n is 1, 2, 3, 4 or 5, and B is H or C₁-C₄-alkyl,
C₂-C₆-alkenyl,
C₃-C₆-cydoalkyl,
amino-C₁-C₄-alkyl, where the amino group is optionally substituted by one or two C₁-C₄-alkyl groups,
an aromatic or nonaromatic heterocyclic radical having 5 or 6 ring atoms and 1 or 2 heteroatoms which are selected independently of one another from N, O and S, which is optionally substituted by 1, 2, 3 or 4 C₁-C₄-alkyl groups,

In particular R¹ is
C₁-C₆-alkyl which is optionally substituted by one or two hydroxy or C₁-C₄-alkoxy groups or a nonaromatic heterocyclic radical having 5 or 6 ring atoms and 1 or 2 heteroatoms which are selected independently of one another from N, O and S, or 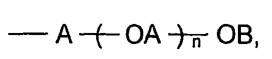 in which
A is -CH₂CH₂-, -CH₂CH₂CH-, 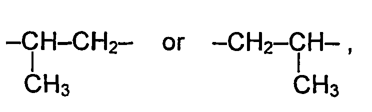 n is 1, 2, 3, 4 or 5 and B is H or C₁-C₄-alkyl.

R¹ is particularly preferably C₁-C₄-alkyl, especially methyl and ethyl or C₂-C₄-alkyl, which is substituted by one or two hydroxy or C₁-C₄-alkoxy groups, such as methoxypropyl, methoxyethyl, hydroxypropyl, hydroxyethyl, 2,3-dimethoxypropyl or 2,3-dihydroxypropyl.

R² is preferably C₁-C₆-alkyl (especially methyl, ethyl, n-propyl or i-propyl), phenyl-C₁-C₄-alkyl, especially benzyl or phenylethyl (the phenyl group in benzyl or phenylethyl is optionally substituted as indicated above), phenyl or phenyl which has one or two substituents which are selected independently of one another from C₁-C₄-alkyl and halogen. R² is particularly preferably C₁-C₆-alkyl.

R³ is preferably 4-fluorophenyl or 3-trifluoromethylphenyl.

R⁴ is preferably 4-pyridyl which is substituted by amino, C₁-C₈-alkylamino, phenylamino, phenyl-C₁-C₄-alkylamino, C₃-C₇-cycloalkylamino or R⁵CONR⁶-, where R⁵ and R⁶ have the meanings indicated above, and in particular is 4-pyridyl which is substituted by C₁-C₈-alkylamino, phenylamino, phenyl-C₁-C₄-alkylamino, C₃-C₇-cycloalkylamino or R⁵CONR⁶. If R⁴ is C₁-C₈-alkylamino, branched alkyl groups are preferred. If R⁴ is R⁵CONR⁶, R⁵ is preferably C₁-C₈-alkyl, C₃-C₇-cycloalkyl, phenyl-C₁-C₈-alkyl or phenyl-C₂-C₆-alkenyl.

R⁵ is preferably C₁-C₄-alkyl.

R⁶ is preferably H or C₁-C₄-alkyl.

The 4-pyridyl group preferably has one substituent. The substituent is particularly preferably in position 2.

A particularly preferred embodiment are the compounds of the formula I in which R¹ is C₁-C₄-alkyl, C₁-C₄-alkoxy-C₁-C₄-alkyl or hydroxy-C₂-C₄-alkyl;
R² is C₁-C₆-alkyl;
R³ is 4-fluorophenyl or 3-trifluoromethylphenyl;
R⁴ is 4-pyridyl which is substituted by C₁-C₄-alkylamino, phenyl-C₁-C₄-alkylamino, C₃-C₇-cycloalkylamino or R⁵CONR⁶-;
R⁵ is C₁-C₄-alkyl; and
R⁶ is H or C₁-C₄-alkyl.

The invention further relates to 2-thio-substituted imidazole compounds of the formula II 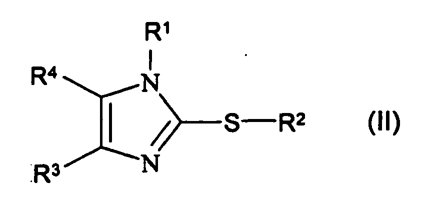 wherein R¹, R² and R³ are as defined above and R⁴ is 4-pyridyl which has one or two substituents which are selected independently of one another from C₅-C₈-alkylamino;
(C₃-C₇-cycloalkyl)-C₁-C₈-alkylamino;
C₃-C₇-cycloalkylamino;
R⁵CONR⁶, wherein R⁵ is selected from H;
C₅-C₈-alkyl;
cycloheptyl;
CF₃;
C₂-C₆-alkenyl;
phenyl-C₁-C₈-alkyl wherein the phenyl group may have one or two substituents which are selected independently of one another from C₁-C₄-alkyl, C₁-C₄-alkoxy or halogen; phenyl-C₂-C₆-alkenyl wherein the phenyl group may have one or two substituents which are selected independently of one another from C₁-C₄-alkyl, C₁-C₄-alkoxy or halogen; and
phenyl-NR¹¹-, wherein R¹¹ is H or C₁-C₄-alkyl and the phenyl group may have one or two substituents which are selected independently of one another from C₁-C₄-alkyl, C₁-C₄-alkoxy or halogen; and
R⁶ is H, C₁-C₄-alkyl, phenyl or benzyl, and
the optical isomers and physiologically tolerated salts thereof.

The invention further relates to 2-thio-substituted imidazole compounds of the formula II 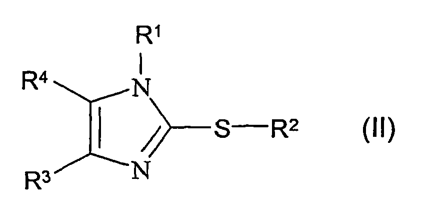 wherein R¹ is selected from:
a) C₁-C₆-alkyl which is substituted by one or two groups independently of one another selected from
   CO₂H
   CO₂-C₁-C₆-alkyl;
   CN;
   halogen;
   C₁-C₆-alkyl- SO₃;
   NR⁷R⁸, wherein R⁷ is H and R⁸ is hydroxy-C₁-C₆-alkyl or R⁷ and R⁸ are hydroxyl-C₁-C₆-alkyl;
b) 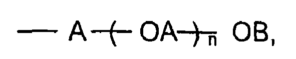 in which
   A is -CH₂CH₂-, -CH₂CH₂CH₂-, 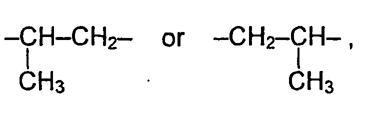 n is 1, 2, 3, 4 or 5, and B is C₁-C₄-alkyl;
c) C₁-C₆-oxoalkyl;
d) cycloheptyl; and
e) (C₃-C₇-cycloalkyl)-C₁-C₆-alkyl;
and R², R³ and R⁴ are as defined above and
the optical isomers and the physiologically tolerable salts thereof.

The compounds of formula II are intermediates for preparing the compounds of formula I. Moreover, they have like the compounds of formula I an immunomodulating and cytokine release-inhibiting effect.

The physiologically tolerated salts may in the present case be acid addition salts or base addition salts. Employed for acid addition salts are inorganic acids such as hydrochloric acid, sulfuric acid or phosphoric acid, or organic acids such as tartaric acid, citric acid, maleic acid, fumaric acid, malic acid, mandelic acid, ascorbic acid, gluconic acid and the like.

The sulfoxide and sulfonyl compounds of the invention are prepared starting from the corresponding 2-thio compounds. The starting compounds are prepared by the processes described in WO 02/066458 A2, which is incorporated in its entirety herein by reference. The 2-thio compounds in which R⁴ is amino- or amido-substituted pyridyl are prepared as shown in scheme 1.

The amino group of the starting compound 2-amino-γ-picoline (1) is protected, e.g. by introducing an acetyl group with acetic anhydride. The methyl group of compound (2) is then oxidized to the carboxyl group, e.g. with potassium permanganate in aqueous medium at 20 to 90°C.

Reaction of the resulting pyridinecarboxylic acid (3) with 4-fluorophenylacetonitrile to give compound (4) and the subsequent elimination of the nitrile group are carried out by variant 1 described in WO 02/066458. In this case, the acetyl group on the amino group of the pyridine compound is also eliminated to form compound (5).

In the next step, the amino group is protected anew, e.g. by introducing an acetyl group with acetic anhydride. The resulting compound (6) is converted into thiono compound (9) as described in WO 02/066458, variant 1 or 2 (shown for variant 1 in scheme 1). The desired radical R² is introduced into (9) as described in WO 02/066458.

In order to introduce the desired substituent into the pyridyl group, firstly the acetyl group is eliminated by hydrolysis, e.g. with aqueous acid, resulting in the amino compound (12). An acyl radical is introduced by acylation, in particular with the appropriate acid chloride R⁵COCl in an inert solvent such as an ether, e.g. tetrahydrofuran, dioxane, or a chlorinated hydrocarbon, e.g. methylene chloride or 1,2-dichloroethane etc. The acylation generally takes place in the presence of a base, e.g. triethylamine, in at least equivalent amount.

The substituted amine compounds are prepared by reacting compound (12) with one or two mole equivalents of an alkyl bromide, cycloalkyl bromide, phenylalkyl bromide or of an optionally substituted iodobenzene in an inert solvent such as dimethylformamide in the presence of a base such as sodium hydride to give the compounds (14) or (15). Alternatively, the amide compound (13) or (36) can be reduced with lithium aluminum hydride in, for example, tetrahydrofuran to compound (16).

An alternative synthesis for compounds of the formula I in which R⁴ is 4-pyridyl which is substituted by R⁵CONR⁶- in position 2 is illustrated in scheme 2.

The acetamido compound (17) is converted by hydrolysis with aqueous acids, e.g. dilute HCl, into the compound (18). (18) is treated with tetrafluoroboric acid in the presence of sodium nitrite, resulting in compound (19). This is subjected to a nucleophilic aromatic substitution with the appropriate amine to give compound (20) which is then reacted with an acylating agent such as a carboxylic anhydride or carbonyl chloride, to give compound (21). The oxidation to the sulfinyl and sulfonyl compounds is effected as described below.

Conversion into the sulfinyl derivatives and sulfonyl derivatives takes place with oxidizing agents by known methods. Especially suitable for preparing the sulfinyl compounds are in particular peroxocarboxylic acids or H₂O₂ solutions in alkanecarboxylic acids such as acetic acid. The sulfonyl compounds (x = 2) of the invention can be prepared in two substeps via the sulfinyl compound or in one step directly from the 2-thio compound (x=0) with strong oxidizing agents such as peroxo compounds in excess or at elevated temperature.

Compounds of the formula I with x = 0 can be oxidized to the corresponding sulfinyl compound with a mild and selective oxidizing agent such as m-chloroperbenzoic acid (mCPBA) in stoichiometric amounts in the cold, particularly preferably at -20°C to room temperature (RT), with hydrogen peroxide in solution in a carboxylic acid, preferably acetic acid, with tert-butyl hydroperoxide, peroxobenzoic acid etc. The sulfonyl compounds are obtained under more energetic conditions through use of excess oxidizing agent or through use of stronger oxidizing agents, e.g. potassium permanganate, see scheme 3.

Catalysts are employed to increase the selectivity, both to suppress further oxidation to sulfonyl compounds and to imidazole N-oxides or pyridine N-oxides. The catalysts are employed in conjunction with cooxidants such as sodium metaperiodate, hydrogen peroxide, atmospheric oxygen and peroxy acids for oxidation to the sulfinyl derivatives. One example of such a catalyst is methylrhenium trioxide which is employed in conjunction with H₂O₂. The oxidations can also be achieved with sodium hypochlorite in alcoholic solution or with sodium metaperiodate in 2-phase systems.

The compounds of formula I wherein R¹ and R² together are ethylene or propylene can be obtained from the thio compounds 22 shown in scheme 4. Cyclisation occurs by activating the hydroxyl group, for example by converting it to the corresponding methane sulfonate by reaction with methane sulfonic acid chloride in the presence of a base such as pyridine, at a temperature from 50 to 90 °C. Under these reaction conditions the methane sulfonate which is formed as an intermediate cyclises to the sulfanyl compound (23) which can be oxidised to the sulfinyl and sulfonyl compound as indicated above. The pyridyl substituent can be modified by subjecting compound (23), (24) or (25) to hydrolysis in aqueous acid to the amino pyridyl compound (26) or (30). The amino group is then substituted by a fluorine atom using Olah's reagent (HF 70% in pyridine) in the presence of sodium nitirite at -10 to -30 °C (Fukuhara et al.; Journal of Fluorine Chemistry, 38 (1988) 435-438, Reagent: 70% (HF)ₓ in Pyridin). The obtained sulfanyl compound (27) can then be treated with amine reagents to introduce the desired sustituent into the pyridine ring by nucleophilic substitution. Examples for this substitution are shown in scheme 5. The obtained amino substituted sulfanyl compounds can finally be converted to the sulfinyl and the sulfonyl compounds as described above.

The compounds having 2,3-dihydro-imidazo[2,1-b]thiazole and 6,7-dihydro-5H-imidazo[2,1-b][1,3]thiazine structure can be prepared from N-{4-[5-(4-fluorophenyl)-3-(3-hydroxy-propyl)-2-thioxo-2,3-dihydro-1H-imidazol-4-yl]-pyridin-2-yl}-acetamide and N-{4-[5-(4-fluorophenyl)-3-(2-hydroxyethyl)-2-thioxo-2,3-dihydro-1H-imidazol-4-yl]-pyridin-2-yl}-acetamide by activation of the hydroxyl group with methane sulfonic acid chloride in pyridine and intramolecular cyclisation. The obtained sulfanyl compounds can then be oxidised to the sulfinyl and sulfonyl compounds as described above. In order to obtain other acyl or alkyl substituted 4-[6-(4-fluorophenyl)-2,3-dihydro-imidazo[2,1-b]thiazol-5-yl]-pyridin-2-yl-amine and 4-[2-(4-Fluoro-phenyl)-6,7-dihydro-5H-imidazo[2,1-b][1,3]thiazin-3-yl]-pyridin-2-ylamine said acetamides are cleaved hydrolytically to obtain the free amines which are then alkylated or acylated with the corresponding alkylating or acylating agent.

The compounds of the invention show *in vitro* and *in vivo* an immunomodulating and cytokine release-inhibiting effect. Cytokines are proteins such as TNF-α and IL-1β which play an important part in numerous inflammatory disorders. The compounds of the invention are suitable, owing to their cytokine release-inhibiting effect, for the treatment of disorders associated with an impairment of the immune system. They are suitable for example for the treatment of autoimmune diseases, cancer, rheumatoid arthritis, gout, septic shock, osteoporosis, neuropathic pain, HIV dissemination, HIV dementia, viral myocarditis, insulin-dependent diabetes, periodontal disorders, restenosis, alopecia, T-cell depletion in HIV infections or AIDS, psoriasis, acute pancreatitis, rejection reactions with allogenaic transplants, allergy-related inflammation of the lungs, arterosclerosis, multiple sclerosis, cachexia, Alzheimer's disease, stroke, jaundice, inflammatory bowel diseases such as ulcerative colitis and Crohn's disease, reperfusion damage, ischemia, congestive heart failure, pulmonary fibrosis, hepatitis, glioblastoma, Guillain-Barré syndrome, systemic lupus erythematosus, adult respiratory distress syndrome (ARDS) and respiratory distress syndrome.

The compounds of the invention can be administered either as single therapeutic active ingredients or as mixtures with other therapeutic active ingredients. The compounds can be administered alone, but they are generally dosed and administered in the form of pharmaceutical compositions, i.e. as mixtures of the active ingredients with suitable pharmaceutical carriers or diluents. The compounds or compositions can be administered orally or parenterally, and they are preferably given in oral dosage forms.

The nature of the pharmaceutical composition or carrier or of the diluent depends on the desired administration form. Oral compositions may be for example in the form of tablets or capsules and comprise conventional excipients such as binders (e.g. syrup, acacia, gelatin, sorbitol, tragacanth or polyvinylpyrrolidone), fillers (e.g. lactose, sugars, corn starch, calcium phosphate, sorbitol or glycine), lubricants (e.g. magnesium stearate, talc, polyethylene glycol or silicon dioxide), disintegrants (e.g. starch) or wetting agents (e.g. sodium lauryl sulfate). Liquid oral products may be in the form of aqueous or oily suspensions, solutions, emulsions, syrups, elixirs or sprays and the like. They may also be in the form of a dry powder which is prepared for reconstitution with water or another suitable carrier. Liquid products of this type may comprise conventional additives, for example suspending agents, flavorings, diluents or emulsifiers. Solutions or suspensions with conventional pharmaceutical carriers can be employed for parenteral administration.

The compounds or compositions of the invention can be adminstered to a mammal (human or animal) in a dose of about 0.5 mg to 100 mg per kg of body weight per day. They can be given in a single dose or in a plurality of doses. The range of effects of the compounds as inhibitors of cytokine release was investigated by means of the following test systems as described by Donat C. and Laufer S. in Arch. Pharm. Pharm. Med. Chem. 333, Suppl. 1, 1-40, 2000.

The pharmacological properties of the compounds of the invention are distinguished by comparison with compounds of the closest prior art disclosed in WO 02/066458 A2 by a number of advantages such as greater metabolic stability, increased oral bioavailability and slower systemic elimination, little inhibition of cytochrome P-450 enzymes and, derived therefrom, less hepatotoxicity, improved selectivity for inhibition of p38 MAP kinase, whereby the occurrence of unpredictable adverse effects is reduced and lower cardiotoxicity.

### In vitro metabolism

The test substances were incubated in standard experiments with rat liver microsomes (phosphate buffer with pH 7.4, NADPH, 37°C). The biotransformations were stopped after 0, 15, 30 and 60 min by adding acetonitrile and were centrifuged, and the protein-free supernatant was analyzed by HPLC. Resulting metabolites were approximately quantified via the peak areas. The result of the biotransformation of Example 12 and of the corresponding sulfanyl compound, which is representative in terms of the sulfinyl/sulfanyl substitution comparison, is described by way of example. The sulfanyl compound was rapidly converted into a number of metabolites and, after 30 min, less than 1% of the pharmacologically active starting substance remained. Under identical experimental conditions, about 70% of the initial amount of Example 12 were still present after 30 min, and more than 50% were still present after 60 min.

### Investigation of the pharmacokinetics in rats

The test substances were ground in a mortar and suspended in 1% aqueous methylcellulose. The suspensions were administered by gavage to the animals.

Immediately before and at fixed times after dosage, blood samples were taken from the animals via a catheter previously implanted in a vein, and were collected in a heparin-coated sample vessel and then centrifuged, and the supernatant plasma was removed. The active ingredient concentration in the plasma was determined by a validated bioanalytical method (liquid chromatography coupled to a tandem mass spectrometer). A concentration/time course was constructed from the plasma concentrations measured at various times after dosage, and the pharmacokinetic parameters were calculated therefrom. The primary characteristics determined were the maximum plasma concentration (= Cₘₐₓ) and the area under the concentration/time curve (= AUC).

The results obtained for the compounds of Examples 1 and 3 and their corresponding sulfanyl compounds are summarized in the table below. The data show that, besides a general improvement in the oral bioavailability, there is also a distinct reduction in the sex-specific differences observed for the sulfanyl compounds.

**Table 1**

| Compound | | rel. Cₘₐₓ | Cₘₐₓ (f/m) | rel. AUC | AUC (f/m) |
|---|---|---|---|---|---|
| Sulfanyl compound | m | 1.00 | 2.03 | 1.00 | 2.65 |
| | f | 0.49 | | 0.38 | |
| Example 3 | m | 1.77 | 1.44 | 2.10 | 1.37 |
| | f | 1.23 | | 1.53 | |
| | | | | | |

| Compound | | rel. Cₘₐₓ | Cₘₐₓ (f/m) | rel. AUC | AUC (f/m) |
|---|---|---|---|---|---|
| Sulfanyl compound | m | 1.00 | 2.70 | 1.00 | 2.65 |
| | f | 0.37 | | 0.38 | |
| Example 1 | m | 4.47 | 1.40 | 2.07 | 1.35 |
| | f | 3.19 | | 1.53 | |

| | | | | | |
|---|---|---|---|---|---|
| m: male animals; f: female animals | | | | | |

### Investigation of the pharmacokinetics in dogs

The test substances are ground in a mortar, and the required amount is suspended in 1 % aqueous methylcellulose and administered to the animals. Immediately before the dosage and at fixed times after dosage, blood samples were taken from the animals. For this purpose, about 0.5 ml of blood was taken from a thigh vein and collected in a heparin-coated sample vessel. The sample was centrifuged, and the supernatant plasma was removed and deep-frozen until the analytical investigation. The active ingredient concentration in the plasma was determined by a validated bioanalytical method (liquid chromatography coupled to a tandem mass spectrometer). A concentration/time course was constructed from the plasma concentrations measured at various times after dosage, and the pharmacokinetic parameters were calculated therefrom. The primary characteristics are the maximum plasma concentration (= Cₘₐₓ) and the area under the concentration/time curve (= AUC).

The results summarized in Table 2 were obtained for Example 3 and its corresponding sulfanyl compound. The data obtained with the same animals, and thus not influenced by interindividual variations, show that in this case too administration of the sulfinyl compound leads to a distinctly increased systemic exposure.

**Table 2**

| Compound | | rel. Cₘₐₓ | Cₘₐₓ (f/m) | rel. AUC | AUC (f/m) |
|---|---|---|---|---|---|
| Sulfanyl compound | m | 1.0 | 2.0 | 1.0 | 1.3 |
| | f | 2.0 | | 1.3 | |
| Example 3 | m | 4.8 | 0.6 | 5.5 | 0.6 |
| | f | 2.9 | | 3.4 | |

| | | | | | |
|---|---|---|---|---|---|
| m: male animals; f: female animals | | | | | |

### Inhibition of cytochrome P450 enzymes

The influence of the test substances on the activity of the human cytochrome P-450 isoenzymes 1A2, 2C9, 2C19, 2D6 and 3A4 was investigated by a standard method. Test systems were microsomes from baculovirus-infected insect cells, each of which expresses one of the cytochrome P-450 isoenzymes. The microsomes cytochrome P-450 isoenzymes catalyze the biotransformation of substrates, and the products of this transformation have fluorescent properties. The intensity of the fluorescence is thus a measure of the activity of cytochrome P-450 enzyme. The investigations took place in 96-well plates. The test substance dissolved in DMSO was diluted with phosphate buffer to the test concentration of 10 µM. A mixture of NADP, glucose 6-phosphate and the enzyme glucose-6-phosphate dehydrogenase was then added. The reaction was started by adding the microsomes and the substrate. The volume of the test mixtures was 0.2 ml. After a predetermined incubation time, the reaction was stopped by adding 75 µl of acetonitrile/ 0.5 M Tris base (80/20), and the fluorescence intensity was quantified using a plate scanner.

The results are shown as % inhibition compared with the control mixtures without inhibitor. The inhibitors known for the respective cytochrome P-450 isoenzymes were always included as positive controls.

**Table 3**

| | | CYP 1A2 | CYP 2C9 | CYP 2C19 | CYP 2D6 | CYP 3A4 |
|---|---|---|---|---|---|---|
| Concentration of test substance | | 10 µM | 10 µM | 10 µM | 10 µM | 1µM |
| Substrate | | 2.5 µM CEC*¹ | 50 µM MFC*² | 6 µM CEC | 0.5 µM AMMC*³ | 50 µM BFC*⁴ |
| Product | | 7-OH-3-CC*¹ | 7-OH-TFC | 7-OH-3-CC*¹ | DM-AMMC | 7-OH-TFC |
| Detection | | | | | | |
| | λₑₓ | 410 nm | 420 nm | 410 nm | 390 nm | 420 nm |
| | λₑₘ | 460 nm | 530 nm | 460 nm | 460 nm | 530 nm |
| Positive control | | Furafylline | Sulphaphenazole | Tranylcypromine | Quinidine | Ketoconazole |
| Incubation time | | 15 min | 45 min | 30 min | 30 min | 30 min |

| | | | | | | |
|---|---|---|---|---|---|---|
| *1: CEC (3-cyano-7-ethoxycoumarin) → 7-OH-3-CC (7-hydroxy-3-cyanocoumarin) *2: MFC (7-methoxy-4-trifluoromethylcoumarin) → 7-OH-TFC (7-hydroxy-4-trifluoromethylcoumarin) *3: AMMC (3-[2-(N,N-diethyl-N-methylamino) ethyl]-7-methoxy-4-methylcoumarin) → DM-AMMC (3-[2-(N,N-diethylamino) ethyl]-7-hydroxy-4-methylcoumarin) *4: BFC (7-benzyloxy-4-trifluoromethylcoumarin) → HFC (7-hydroxy-4-trifluoromethylcoumarin) | | | | | | |

### Inhibition of protein kinases

The influences of the test substances on the activity of the protein kinases listed below was investigated by a standard method. The protein kinase, the test substance and the substrate is introduced into reaction mixtures with a total volume of 25 µl. The reaction is started by adding γ-³³P-ATP. After incubation for a defined period, the reaction is stopped and an aliquot of the reaction mixture is placed on a filter. After the filter has been washed and dried, the radioactivity bound to the filter is quantified in a scintillation counter. The percent influence on the protein kinase activity is found by comparing the measured radioactivity in a controlled experiment without added test substance.

**Table 4**

| Enzyme | c-Raf (h) | CaMK II (r) | GSK3β | Lck | | MEK1 | | PKA | | PKBα | |
|---|---|---|---|---|---|---|---|---|---|---|---|
| Substrate | MBP | Peptide. | Peptide (P-GS2) | Peptide (Cdc2) | | MAPK2 (m) | | Kemptide | | Peptide | |
| | | | | | | | | | | | |

| Enzyme | PKCα | ROCK-II | p38α-δ | ERK1 | ERK2 | | JNK1α | | JNK2α | | JNK3 |
|---|---|---|---|---|---|---|---|---|---|---|---|
| Substrate | Histone H1 | Peptide | MBP | MAPK1 (h) | MBP | | ATF-2 | | ATF-2 | | Peptide |

### Inhibition of cytokine release in stimulated PBMCs (peripheral blood mononuclear cells) and stimulated human whole blood

Solutions of the substances to be tested are prepared in DMSO (PBMC experiments) or Cremophor EL/ethanol 70/30 (experiments with whole blood). 0.01 vol. of test substance solution is added to whole blood mixed with anticoagulant or to PBMCs isolated from whole blood, and preincubated with the test substances for 15 min. In this case, 3-5 different test substance concentrations are investigated in one series of experiments. The cells are then stimulated to produce and release cytokines by adding bacterial lipopolysaccharide (LPS). After an incubation time of 4 h, the reaction is stopped and the test mixture is centrifuged. The inhibition of cytokine release is quantified by including control mixtures to which only the diluting medium (DMSO or Cremophor EL/ethanol) has been added. A series of experiments with the reference substance SB-203580 as positive control is also included. The concentrations of the cytokines interleukin-1β (IL-1β) and tumor necrosis factor α (TNFα) in the cell culture supernatant and plasma are determined by ELISA. Complete test sets from various manufacturers, e.g. R&D or Beckman Coulter Immunotech, can be used for this. The percent inhibition of IL-1β and TNFα production is found from the concentration ratio in the test mixtures and control mixtures. The concentration at which cytokine release is reduced by 50% (= IC₅₀) compared with the control group is found from the concentration/effect relation. To compare the results of experiments on different days, a relative activity compared with SB-203580 is determined (relative activity = IC₅₀ (SB-203580) / IC₅₀ (test substance)).

### Inhibition of TNFα formation in mice after treatment with LPS

The test substances are suspended in 1% tragacanth and administered by gavage to male Balb/c mice (time t=0 h). To induce TNFα-release, 500 mg/kg galactosamine (GAlN) and 1.5 mg/kg LPS are administered intraperitoneally at time t=1 h. At time t=2.5 h, the animals are anesthetized by intravenous administration of 150 mg/kg Narkoren and 0.8 mg/kg heparin, and a blood sample is taken by cardiac puncture. After centrifugation, the plasma concentration of TNFα is quantified by ELISA. A control group is included to determine the activity of the test substances administered orally. These animals receive a 1% tragacanth suspension at time t=0h. The percent inhibition of cytokine release is calculated by comparing the TNFα concentrations in animals from the test group and control group. It is possible where appropriate, by administering different dosages, to determine the dosage at which the TNFα is reduced by 50% (= ED₅₀) compared with the control group.

### EXAMPLES

### General conditions and analyses:

| | |
|---|---|
| Melting points: | Mettler FP 5 |
| IR spectroscopy: | Thermo Nicolet Avatar 330 FT-IR, with Smart Endurance |
| NMR spectroscopy: | Varian Mercuryplus 400, 5 mm PFG IDP (1H: 400 MHz, 13C:100 MHz) |
| GC-MS: | Agilent GC 6890plus + MSD 5973 + ALS 7683 |
| | He-(5% phenyl methyl silicone, 15 m 250µm, 0.25 µm |
| LC-(MS): | HP1090 DAD: Thermo Hypersil Keystone, 150 mm x 4.6, Betasil C8, 5µm and |
| | HP1100 Phenomenex, Synergi 250 mm x 2.00 mm Polar-RP 80A, 4µ, DAD+MS (Bruker Esquire HCT-IonTrap) |
| Prep HPLC: | Varian PrepStar 2 SD1: 250 ml/min. column head |
| | Column C18, 21.4 mm x 250 mm, 8 µm |
| | CSP: Chiralpac AD |

TLC adsorbents and plates:
Polygram SIL G/UV, Macherey-Nagel, Düren.
Polygram ALOX N/UV, Macherey-Nagel, Düren.

Adsorbents for column chromatography:
Aluminum oxide ICN-Alumina TSC, No. 04511, ICN Biomedicals.
Silica gel SiO2 60 (0.063 mm), No. 7734, Merck, Darmstadt.
Silica gel Geduran Si 60 (0.063-0.200 mm), No. 110832, Merck, Darmstadt.

Deuterated solvents for NMR spectroscopy:
CDCl3, (99.96%), 0.03%TMS Euriso-top (C.E.Saclay, Gif-sur-Yvette, France)
[D6]-DMSO (99.9%), 0.05% TMS Cambridge Isotop Laboratories (CIL), Andover MA, USA.
[D4]-Methanol: (99.8%)0.05% TMS Cambridge Isotop Laboratories (CIL), Andover MA, USA.

### Anhydrous (absolute or abs.) solvents

The solvents were purchased (from Fluka, Neu-Ulm), stored over molecular sieves and used without additional post-drying method. Anhydrous solvents and apparatuses employed with exclusion of water were blanketed with dry argon and kept under a gentle stream of dry argon.

### EI-MS

EI mass spectra were recorded from GC/MSD systems at 70 eV. The samples were dissolved in tetrahydrofuran (THF) or methanol, volume injected 1 µl, ALS split ratio 1:50, and measured using helium as carrier gas on a 5% phenyl-methylsilicone quartz capillary column. The temperature was in the range from 120 or 160°C to 280°C.

### NMR spectroscopy

The signals (chemical shifts) in the NMR spectra are reported relative to tetramethylsilane as internal standard (δ= 0 ppm). The chemical shift is reported in ppm (delta scale), and coupling constants are reported in Hz, ignoring the sign. Abbreviations used for first-order signals: s = singlet, d = doublet, dd= double doublet, t = triplet, q = quartet, qui = quintet,
for 2nd-order signals: A or B, A,B or X
no assignment is made for higher order signals: m = multiplet,

### Infrared spectroscopy:

IR spectra are recorded in a diamond ATR system between 4000 cm⁻¹ and 550 cm⁻¹ in absorption mode directly from solids or crystals.
Wave numbers (cm⁻¹) are recorded for the 10 - 20 most intense signals, together with the observed intensities in some examples.

Melting points are calibrated and corrected. The reference substances used are vanillin, phenacetin and caffeic acid standards.

The molecular weight and the molecular composition was calculated from the structure or the molecular formula.
The molecular composition is determined for carbon, hydrogen, nitrogen, sulfur and, if necessary, for halogen.

The compounds are named according to IUPAC rules.

Purity is determined as area percent (area % = proportion of the total of the integrated peak areas) measured via UV absorption at 230 nm for samples containing about 1.0 mg/ml, dissolved in dry MeOH (Uvasol, HPLC purity) and with a volume of 5-10 µl injected.

### Abbreviations:

- HPLC: high performance liquid chromatography
- m.p., mp: melting point
- RT: retention time
- THF: tetrahydrofurane
- MeOH: methanol
- EA, EtOAc: ethyl acetate
- DMF: dimethylformamide
- TLC: thin layer chromatography
- DCM: dichloromethane

### Preparation of the starting compounds:

The compounds of the invention described in the examples were obtained by reacting the compounds to which WO 02/066458A2 relates and which were prepared by the processes described therein:

### (The numbers of the compounds refer to scheme 1)

### a) 2-Acetamido-4-methylpyridine (2)

200.0 g of 2-aminopicoline (1) are mixed with 400 ml of acetic anhydride and with 100 mg of 4-dimethylaminopyridine and refluxed for 5 h. After cooling, the excess acetic anhydride is substantially distilled off, and the residue is poured onto ice and neutralized with aqueous ammonia solution. The precipitate of (2) which separates out during this is filtered off and dried in vacuo over P₂O₅.
Yield: 209.0 g (75%)

### b) 2-Acetamidopyridine-4-carboxylic acid (3)

214.0 g of (2) are introduced in portions with stirring into an aqueous solution of 160 g of potassium permanganate at 50°C. A further 360 g of potassium permanganate are added in portions over the course of one hour. The temperature of the reaction mixture should not exceed 90°C during this. The mixture is then stirred for 1.5 h and filtered hot, and the filtrate is adjusted to pH 3-4 with conc. HCl. The white precipitate of (3) which separates out is filtered off and dried in vacuo over P₂O₅.
Yield: 108.0 g (42%)

### c) 2-Cyano-2-(4-fluorophenyl)-1-(2-acetamido-4-pyridyl)ethanone (4)

18.0 g of (3) are taken up in 50 ml of abs. dimethylformamide (DMF) and, after addition of 17.0 g of carbonyldiimidazole (CDl), stirred at room temperature for 45 min. Then 14.9 g of 4-fluoroacetonitrile and 14.6 g of potassium tert-butanolate are added, and the reaction mixture is heated at 120°C for 2 h. After cooling, the mixture is stirred at room temperature overnight. Ice is then added to the solution, and it is neutralized with conc. HCl. The precipitate of (4) which separates out is filtered off and dried in vacuo over P₂O₅.
Yield: 18.1 g (65%)

### d) 2-(4-Fluorophenyl)-1-(2-amino-4-pyridyl)ethanone (5)

27.9 g of (4) are mixed with 150 ml of 48% strength hydrobromic acid, and the reaction mixture is kept at a gentle boil for 30 h. After cooling, the mixture is poured onto ice and neutralized with concentrated ammonia. The precipitate of (5) which separates out is filtered with vigorous suction, washed several times with petroleum ether and cold diethyl ether and dried.
Yield: 11.7 g (55%)

### e) 2-(4-Fluorophenyl)-1-(2-acetamido-4-pyridyl)ethanone (6)

12.0 g of compound (5) are suspended in 100 ml of acetic anhydride and, after addition of a spatula tip of 4-dimethylaminopyridine, the reaction mixture is refluxed for 5 h. The excess acetic anhydride is substantially distilled off, and the residue is hydrolyzed and adjusted to pH 7 with conc. ammonia. The pale precipitate of (6) which separates out is filtered off and dried in vacuo over P₂O₅.
Yield: 13.5 g (94%)

### f) 2-(4-Fluorophenyl)-1-(2-acetamido-4-pyridyl)-α-hydroxyiminoethanone (7)

2.1 g of sodium methoxide solution (30% in methanol) are mixed with 30 ml of methanol and added to a solution of 1.2 g of isoamyl nitrite in 20 ml of methanol. While stirring, 3.0 g of (6) are added in portions, and then stirring is continued at room temperature for 2 h. The solvent is distilled off, and the solid residue is taken up in water and adjusted to pH 7 with 10% strength HCl. The pale precipitate of (7) which separates out is filtered off and dried in vacuo over P₂O₅.
Yield: 1.8 g (54%)

### g) Preparation of compounds (8):

(7) is dissolved together with twice the amount of the appropriate triazine in absolute ethanol and refluxed until the precursor has completely reacted. After cooling, ethanol is removed in a rotary evaporator. The partly oily residue solidifies on addition of diethyl ether. The precipitate of compounds (8) is filtered off and dried in vacuo.

| | |
|---|---|
| Yields: | R¹= -CH₃ : 74% |
| | R¹= -C₃H₇: 62% |
| | R¹= 2,2,6,6-tetramethylpiperidin-4-yl: 81 % |
| | R¹= N-morpholinopropyl-: 72% |
| | R¹= 3-hydroxypropyl-: 56% |

### h) Preparation of compounds (9)

Compound (8) is dissolved in CHCl₃, and the reaction mixture is cooled in an ice bath. An equimolar solution of 2,2,4,4-tetramethyl-cyclobutan-1,3-dithione in CHCl₃ is slowly added dropwise, and the mixture is then stirred in the ice bath for 30 min. The ice bath is removed and stirring is continued at room temperature for 1 h. The solvent is then removed in a rotary evaporator, and the solid residue is stirred in diethyl ether. The precipitate of (9) is filtered off and dried in vacuo.

| | |
|---|---|
| Yields: | R¹= -CH₃ : 96% |
| | R¹= -C³H₇: 74% |
| | R¹=2,2,6,6-tetramethylpiperidin-4-yl: 61% |
| | R¹= N-morpholinopropyl-: 82% |
| | R¹= 3-hydroxypropyl-: 71% |

### i) Preparation of compounds (10)

Compound (9) is suspended in abs. ethanol under protective gas, and the equimolar amount of methyl iodide is added. After addition of a spatula tip of Na₂CO₃, the reaction mixture is refluxed until the precursor has completely reacted. After cooling, the inorganic salts are filtered off, and the solvent is removed in a rotary evaporator. The crude product (10) is purified by column chromatography.

### j) 4-(4-Fluorophenyl)-1-methyl-5-(2-acetamido-4-pyridyl)-2-methylthioimidazole

R¹= -CH₃: yield 63%
NMR (CDCl₃, ppm): 8.75 (bs, 1H), 8.26-8.24 (m, 2H), 7.46- 7.39 (m, 2H), 6.97- 6.88 (m, 3H), 3.53 (s, 3H), 2.71 (s, 3H), 2.23 (s, 3H)
IR (1/cm): 1669, 1607, 1543, 1505, 1416, 1268, 1218, 843

### k) 4-(4-Fluorophenyl)-1-n-propyl-5-(2-acetamido-4-pyridyl)-2-methylthioimidazole

R¹ = -C₃H₇: yield 28%
NMR (CDCl₃, ppm): 8.28- 8.25 (m, 2H), 7.44-7.37 (m, 2H), 6.96-6.88 (m, 2H), 3.85 (t, 2H, J = 7.7 Hz), 2.73 (s, 3H), 2.24 (s, 3H), 1.65-1.57 (m, 2H), 0.83 (t, 3H, J = 7.4 Hz) IR (1/cm): 3303, 1674, 1544, 1501, 1416, 1264, 1213, 845

### l) 4-(4-Fluorophenyl)-1-(2,2,6,6-tetramethylpiperidin-4-yl)-5-(2-acetamido-4-pyridyl)-2-methylthioimidazole

R¹ = 2,2,6,6-Tetramethylpiperidin-4-yl: yield 23%
NMR (CDCl₃, ppm): 10.62 (s, 1H), 8.38-8.35 (m, 2H), 8.01 (s, 1H), 7.33-7.26 (m, 2H), 7.04-6.95 (m, 3H), 4.19- 4.03 (m, 1H), 2.61 (s, 3H), 2.00 (s, 3H), 1.87 -1.81 (m, 2H), 1.52-1.47 (m, 2H), 0.93 (s, 6H), 0.78 (s, 6H)
IR (1/cm): 2976, 1699, 1533, 1407, 1255, 838

### m) 4-(4-Fluorophenyl)-1-[3-(N-morpholino)propyl]-5-(2-acetamido-4-pyridyl)-2-methylthioimidazole

R¹= N-morpholinopropyl-: yield 52%
NMR (CDCl₃, ppm):8.29 (m, 1H), 8.12 (s, 1H), 7.42-7.35 (m, 2H), 6.96-6.87 (m, 3H), 4.08-3.92 (m, 6H), 3.17-3.00 (m, 6H), 2.74 (s, 3H), 2.41-2.34 (m, 2H), 2.24 (s, 3H)

### n) 4-(4-Fluorophenyl)-1-(3-hydroxypropyl)-5-(2-acetamido-4-pyridyl)-2-methylthioimidazole

R¹= 3-Hydroxypropyl: yield 32%
NMR(CDCl₃, ppm):8.69 (bs, 1H), 8.23-8.19 (m, 2H), 7.44-7.37 (m, 2H), 6.98-6.86 (m, 3H), 4.04 (t, 2H, J= 7.9 Hz), 3.70 (t, 2H, J= 7.2 Hz), 2.74 (s, 3H), 2.25 (s, 3H), 2.13-2.05 (m, 2H)

### o) 4-(4-Fluorophenyl)-1-methyl-5-(2-amino-4-pyridyl)-2-methylthioimidazole

Compound j) is dissolved in 10% strength HCl and refluxed for 14 h. After cooling, 20% strength NaOH is used to neutralize. The pale precipitate which separates out is filtered off and dried in vacuo over P₂O₅.
Yield: 82%
NMR (CDCl₃, ppm): 8.16-8.13 (m, 1H), 7.50-7.43 (m, 2H), 6.98-6.89 (m, 2H), 6.60-6.57 (m, 1H), 6.41 (s 1H), 4.60 (bs, 2H,) 3.46 (s, 3H), 2.70 (s, 3H)
IR (1/cm): 1629, 1542, 1509, 1215, 837, 814

### Compounds p) to r)

Compound o) is dissolved in dry tetrahydrofuran (THF), and 1.2 times the amount of triethylamine is added. The reaction mixture is cooled in an ice bath. While stirring, 1.2 times the amount of the acid chloride is added dropwise, and stirring is continued until precursor is no longer present. The reaction mixture is filtered and the filtrate is concentrated to dryness. The crude product is purified by column chromatography.

### p) 4-(-Fluorophenyl)-1-methyl-5-[2-(4-methoxybenzamido)-4-pyridyl]-2-methylthioimidazole

Yield: 62%
NMR (CDCl₃, ppm): 8.66 (s, 1H), 8.44 (s, 1H), 8.30 (s, 1H), 8.29-8.28 (m, 1H), 7.94-7.89 (m, 2H), 7.49-7.42 (m, 2H), 6.95- 6.90 (m, 4H), 3. 90 (s, 3H), 3.58 (s, 3H), 2.72 (s, 3H)
IR (1/cm): 3410, 1674, 1500, 1412, 1253, 1175, 840, 759

### q) 4-(-Fluorophenyl)-1-methyl-5-(2-cyclopropylamido-4-pyridyl)-2-methylthioimidazole

Yield: 24%
NMR (CDCl₃, ppm): 8.67-8.62 (m, 1H), 7.63-7.38 (m, 3H), 6.98-6.85 (m, 3H), 3.90 (s, 3H), 2.73 (s, 3H), 2.05-1.98 (m, 1H), 1.26-1.14 (m, 2H), 1.21-1.14 (m, 2H)

### r) 4-(-Fluorophenyl)-1-methyl-5-(2-cyclopentylamido-4-pyridyl)-2-methylthioimidazole

Yield: 53%
NMR (CDCl₃, ppm): 8.28-8.22 (m, 3H), 7.46-7.39 (m, 2H), 6.97-6.87 (m, 3H), 3.54 (s, 3H), 2.69 (s, 3H), 1.97-1.67 (m, 8H)

### Compounds s) to u)

1.2 eq of NaH are suspended in DMF, compound o) is slowly added, and the reaction mixture is stirred at room temperature for 1 h. The benzyl bromide or phenylethyl bromide is then added in equimolar amount and refluxed until precursor is no longer present. The reaction mixture is diluted with water, and the precipitate which separates out is filtered off. The crude product is purified by column chromatography.

### s) 4-(4-Fluorophenyl)-1-methyl-5-(2-benzylamino-4-pyridyl)-2-methylthioimidazole

Yield: 13%
NMR (CDCl₃, ppm): 8.12-8.16 (m, 1H), 7.47- 7.26 (m, 7H), 6.95- 6.86 (m, 2H), 6.53-6.50 (m, 1H), 6.24 (s, 1H), 5.30 (bs, 1H), 4.47 (d, 2H, J= 5.8 Hz), 3.32 (s, 3H), 2.68 (s, 3H)
IR(1/cm): 3241, 1610, 1507, 1219, 839, 813, 737, 698

### t) 4-(4-Fluorophenyl)-1-methyl-5-[2-(2-phenylethyl)amino-4-pyridyl]-2-methylthioimidazole

Yield: 54%
NMR (CDCl₃, ppm): 8.12-8.10 (m, 1H), 7.41- 7.19 (m, 7H), 6.92- 6.84 (m, 2H), 6.46-6.43 (m, 1H), 6.06 (s, 1H), 5.18 (d, 1H, J= 6.3 Hz), 4.63-4.57 (m, 1H), 3.11 (s, 3H), 2.70 (s, 3H),
IR (1/cm): 1605, 1505, 1432, 1219, 839, 701
If the benzyl bromide is added in 2.5 times the amount, the nitrogen is bisubstituted (15).

### u) 4-(4-Fluorophenyl)-1-methyl-5-(2-dibenzylamino-4-pyridyl)-2-methylthioimidazole

Yield: 81%
NMR (CDCl₃, ppm): 8.27-8.24 (m, 1H), 7.45- 7.19 (m, 12 H), 6.95- 6.86 (m, 2H), 6.51-6.48 (m, 1H), 6.31 (s, 1H), 4.80 (s, 4H), 3.17 (s, 3H), 2.66 (s, 3H)
IR (1/cm): 1598, 1496, 1427, 1219, 840, 831, 734, 702

### Compounds v) to y)

The compound of Examples j), k) and l) is dissolved in THF and, while stirring, a 10-fold excess of LiAlH₄ is added. The reaction mixture is then heated for 2 h. After cooling, water is slowly added. The mixture is extracted several times with CH₂Cl₂, and the combined organic phases are dried over Na₂SO₄. The desiccant is filtered off, and the solvent is removed. The crude product is purified by column chromatography.

### v) 4-(4-Fluorophenyl)-1-methyl-5-(2-ethylamino-4-pyridyl)-2-methylthioimidazole

Yield: 70%
NMR (CDCl₃, ppm): 8.17-8.15 (m, 1H), 7.53-7.46 (m, 2H), 6.98-6.89 (m, 2H), 6.52-6.49 (m, 1H), 6.27-6.26 (m, 1H), 4.59 (t, 1H, J= 6.0 Hz), 3.47 (s, 3H), 3.29-3.23 (m, 2H), 2.70 (s, 3H), 1.23 (t, 3H, J= 7.1 Hz)
IR (1/cm): 3235, 1604, 1562, 1506, 1435, 1221, 844, 806

### w) 4-(4-Fluorophenyl)-1-n-propyl-5-(2-ethylamino-4-pyridyl)-2-methylthioimidazole

Yield: 25%
NMR (CDCl₃, ppm): 8.17-8.14 (m, 1H), 7.51-7.43 (m, 2H), 6.98-6.87 (m, 2H), 6.53-6.50 (m, 1H), 6.27 (s, 1H), 4.61 (t, 1H, J= 2.8 Hz), 3.79 (t, 2H, 7.7 Hz), 3.28- 3.22 (m, 2H), 2.71 (s, 3H), 1.66-1.54 (m, 2H), 1.24 (t, 3H, J= 7.2 Hz), 0.83 (t, 3H, J= 7.4 Hz)
IR (1/cm): 3275, 2930, 1607, 1525, 1507, 1219, 846, 813

### x) 4-(4-Fluorophenyl)-1-(2,2,6,6-tetramethylpiperidin-4-yl)-5-(2-ethylamino-4-pyridyl)-2-methylthioimidazole

Yield: 52%
NMR (CDCl₃, ppm): 8.10- 8.07 (m, 2H), 7.47- 7.40 (m, 2H), 7.12-7.03 (m, 2H), 6.44-6.41 (m, 1H), 6.37 (s, 1H), 4.30-4.14 (m, 1H), 3.27- 3.21 (m, 2H), 2.66 (s, 3H), 2.11-1.91 (m, 2H), 1.59-1.52 (m, 2H), 1.12-1.01 (m, 9H), 0.90 (s, 6H)
IR (1/cm):3325, 2959, 1603, 1516, 1499, 1217, 1158, 849, 812

### y) 4-(4-Fluorophenyl)-1-(3-N-morpholinopropyl)-5-(2-acetamido-4-pyridyl)-2-(4-methylsulfinylbenzyl)thioimidazole

4-(4-Fluorophenyl)-1-(3-N-morpholinopropyl)-5-(2-acetamido-4-pyridyl)-imidazole-2-thione is suspended in abs. ethanol under protective gas, and the equimolar amount of 4-methylsulfinylbenzyl chloride is added. After addition of a spatula tip of Na₂CO₃, the reaction mixture is refluxed until the precursor has completely reacted. After cooling, the inorganic salts are filtered off, and the solvent is removed in a rotary evaporator. The crude product is purified by column chromatography.
Yield: 27%
NMR (CDCl₃, ppm): 8.67 (bs, 1H), 8.28-8.25 (m, 2H), 8.12 (s, 1H), 7.64-7-49 (m, 4H), 7.44-7.37 (m, 2H), 6.98-6.86 (m, 3H), 4.45 (s, 2H), 3.81-3.65 (m, 6H), 2.72 (s, 3H), 2.54-2.52 (m, 6H), 2.22 (s, 3H), 1.85-1.73 (m, 2H)

### General preparation method 1 - sulfoxides:

In a typical embodiment, 10-20 mmol (3.5 g-7 g) of the appropriate thio compound (e.g. compound a) to y)) is dissolved (30-100 ml, ~ 10 ml / g of precursor) or suspended in glacial acetic acid, and the suspension or solution is cooled in an ice bath to 0 -10°C and then stoichiometric amounts of a 35% strength aqueous hydrogen peroxide solution are added in slight excess (1.1 : 1.2 equivalents, 1-2 g) in 2-3 portions. The progress of the reaction is monitored by thin-layer chromatography, high pressure liquid chromatography or gas chromatography. If precursor is still detectable after the usual reaction time of 4-6 hours has elapsed, the reaction time can be extended to several hours (16-72 h), or the excess of hydrogen peroxide is raised to 2-3 equivalents.

If the sample shows no starting material left, the reaction mixture is poured into ice-water (300-700 ml) and neutralized with 12.5 to 25% strength aqueous ammonia solution until pH 8 is reached, after which the product crystallizes out of the aqueous phase or separates as an oil, which crystallizes on standing in the cold.
The deposited solids are collected on a Büchner funnel and dried and, if necessary, purified by recrystallization from ethyl acetate or diethyl ether or by chromatography with ethyl acetate, ethyl acetate/methanol, ethyl acetate/THF or ethyl acetate/DMF (dimethylformamide) on silica gel or alumina. Substance fractions which elute early are discarded. There are obtained successively unreacted precursor in 5-10% yield and sulfone in 5-20% yield. The sulfoxide is present in the fractions which elute late.

The yield of sulfoxide is typically 50-60% after column chromatography and 85-90% after recrystallization.

The acid addition salts are prepared by dissolving the imidazole bases in a suitable solvent such as ethyl acetate, THF, methanol, ethanol, isopropanol etc. This solution is then added to solutions of stoichiometric amounts of acids, e.g. gaseous HCl in ethanol, diethyl ether, isopropanol or aqueous HCl. The salts are then isolated in a conventional way.

### General preparation method 1a - sulfoxides:

1 equivalent of the corresponding [4-(3-alkyl or substituted alkyl-2-alkylsulfanyl-5-phenyl or 5-substituted phenyl-3H-imidazol-4-yl)-pyridin-2-yl]- alkyl-,cycloalkyl, aryl- or acyl-amine compound is taken up in a water-miscible solvent such as THF, dioxane, glyme, acetone and butanone or isopropyl-methylketone or mixtures thereof or mixtures of acetone and lower alcohols such as methanol, ethanol, isopropanol (~ 10 mL / g educt). An aqueous solution of the oxidation agent sodium metaperiodate is added to the water-miscible phase in one volume or in aliquots. To selectively obtain the sulfoxides stoichiometric amounts up to a small molar excess of periodate may be used in general. The educts may also be present in suspension. The suspension or solution is in general heated to the boiling temperature of the mixture (reflux) and the reflux is maintained for several hours to several days. The progress of the reaction is controlled by thin layer chromatography, HPLC or gas chromatography. If after the normal reaction time of 4 to 6 hours educt can be detected, the reaction time can be extended (16-72 h). An excess of sodium metaperiodate does in general not enhance the reaction but may result in increased formation of the corresponding sulfone. Advantageously, the reaction is terminated at 90-95% conversion. The selectivity for sulfoxide formation versus sulfone formation is then in general > 95%. Due to the lower polarity of the sulfanyl starting materials as compared to the sulfoxides traces of educts can be removed by extraction with lipophilic solvents (ethyl acetate, acetone, THF, diethylether) or by recrystallization from semi-polar organic solvents.

If the progress of the reaction is as desired (at most 0.5-1 % sulfone), the low-boiling organic components are evaporated. Unreacted starting materials and sulfones precipitate as solids. If required water may be added to dissolve undesired inorganic precipitates. The precipitated solids are then slurried with warm water, isolated by filtration and washed with cold water and dried. The solid material is purified by extraction with or recrystallization from ethyl acetate, acetone, THF or diethyl ether. Alternatively, the crude sulfoxides can be purified by chromatography on silica gel or aluminium oxide with ethyl acetate, ethyl acetate-methanol, ethyl acetate-THF or ethyl acetate-DMF as eluent.

The oxidation of the thio compounds results in racemates of the sulfoxides which can be resolved into the pure enantiomers by enantiomer separation. The pure enantiomers are isolated from the racemates preferably by preparative (high pressure) column chromatography with high enantiomeric purity (ee > 95%) by use of chemically modified celluloses and chemically modified starches, such as, for example, Chiralpak OD, Chiralpak AD, Chiralpak OJ, as stationary phase (chiral stationary phase = CSP). The eluent particularly preferably used comprises isopropanol-aliphatic hydrocarbon mixtures as eluent with an isopropanol content of 10-90%, particularly preferably under isocratic conditions with an isopropanol content of 60-80%.

A further possibility for separating into the enantiomers consists of salt formation and crystallization with enantiopure acids such as, for example, dextrorotatory L-(+)-lactic acid L-(+)-mandelic acid, (1 R)-(-)-camphor-10-sulfonic acid or (1S)-(+)-camphor-10-sulfonic acid.

Oxidation of chiral precursor compounds to sulfoxides results in mixtures of diastereomers which can be separated in a conventional way, e.g. by crystallization.

### General preparation method 2 - sulfones:

1 mmol (~ 0.3-0.4 g) of the appropriate thio compound is suspended (30-100 ml, - 10 ml / g of precursor) or dissolved in glacial acetic, acid, and this suspension or solution is heated to 40-50°C in a heating bath. An excess of 35% strength aqueous hydrogen peroxide solution (3 to 9 equivalents; 0.3 g-1 g) is added in one portion, and the process of the reaction is monitored by thin-layer chromatography, high pressure liquid chromatography or gas chromatography. If precursor is still detectable after the usual reaction time of 4-6 hours has elapsed, the reaction time can be extended to several hours (16-72 h), or the reaction temperature is raised further to 60-70°C. If the sample shows no starting material left, the reaction mixture is poured onto ice-water (300-700 ml) and neutralized with 12.5 to 25% aqueous ammonia solution until pH 8 is reached. The product crystallizes on standing or separates out as oil from the aqueous phase. The deposited solids are collected on a Büchner funnel, dried and, if necessary, purified by recrystallization from ethyl acetate or diethyl ether or by chromatography with ethyl acetate, ethyl acetate/methanol, ethyl acetate/THF or ethyl acetate/DMF on silica gel or alumina. There are obtained successively imidazole N-oxide sulfoxides in 10-30% yield and sulfones in 50-60% yield. Small amounts of sulfoxides are obtained from fractions which elute late, typically <10% yield.

### General preparation method 3 - salts:

### 3.1 Preparation of methane sulfonates

A solution of methane sulfonic acid in THF (1 M) is added to an approximately 2.5% by weight solution of the compound in THF (prepared by gentle warming) in stoichiometric amount. Upon cooling colorless crystals are formed after 5 to 10 minutes. Crystallization is completed by cooling to 3-5 °C for several hours. The precipitated salt is isolated by filtration and washed with a small amount of diisopropylether (2 x 1 ml) and dried for several hours uder vacuum at 40 to 50 °C.

### 3.2 Preparation of hydrochlorides

A 1.25 M solution of HCl in isopropanol is added to an approximately 2.5% by weight solution of the compound in THF (prepared by gentle warming) in stoichiometric amount. The salt is then isolated as given under 3.1.

### 3.3 Preparation of the hydrobromides

A1M solution of HBr in THF is added to an approximately 2.5% by weight solution of the compound in THF (prepared by gentle warming) in stoichiometric amount. The salt is then isolated as given under 3.1.

### 3.4 Preparation of the hydrogensulfates and sulfates

A 1 M solution of H₂SO₄ (96%) in THF is added to an approximately 2.5% by weight solution of the compound in THF (prepared by gentle warming) in stoichiometric amount. The solvent is evaporated under vacuum and the residue is suspended in diisopropylether, the crystalline solid is filtered off, washed with diisopropylether and dried.

For the preparation of the sulfates 0.5 equivalents of sulphuric acid are used.

### General preparation method 4 - fluoropyridyl-sulfanyl- and fluoropyridyl-sulfinyl-imidazole precursors for nucleophilic replacement by amines

General procedure for introduction of fluorine in place of amino function of 4-5-aryl-sulfanyl-imidazol-4-yl]-pyridin-2-ylamine precursors by nitrosation and diazonium replacement with HF or HBF₄.

The amino compounds (11) prepared according to the methodology of WO02 /066458 were obtained by acidic hydrolysis from the acetamido-pyridyl precursors (10) following the sequence in reaction scheme 1.

As long as there is no other functional group present in the amino-precursor molecule, which is sensitive to the strong acidic conditions (acetals), to fluoride (silans) or sensitive against nitrosation and diazotation (primary and secondary amines), the amino group may be transformed to the diazonium group by introducing the alkali nitrite under aqueous conditions to the HBF₄ acidic solution of these precursors. This solution is made by dissolving the aminopyridyl base in an aqueous or methanol solution of tetra fluoro boric acid (HBF₄) or by dissolving the base directly in Olah's reagent (70% HF in pyridine). Diazotizing with nitrous acid esters (i.e. isoamyl and isobutyl nitrite) under non-aqueous conditions is possible when Olah's reagent is used to dissolve the amino pyridyl base.

Without isolating any diazonium tetrafluoro borate the fluorination is accomplished directly under the reaction conditions. In analogous manner direct fluorination is observed with Olah's reagent under aqueous and non-aqueous conditions.

### Preparation of 2-fluoro-4-5-aryl-sulfanyl-3H-imidazol-4-yl-pyridines from 2-(4-fluorophenyl)-1-(2-fluoro-pyridin-4-yl)-ethanone

In cases where fluoride sensitive or acid sensitive functional groups are present or where primary or secondary (even tertiary) amino groups are present (R¹ and R²) a modified and optimised strategy is necessary to make these precursors available. The 1-(2-amino-pyridin-4-yl)-2-(4-fluoro-phenyl)-ethanone (5, scheme 1), which was prepared according to WO 02/066458, can be converted to the 2-(4-fluoro-phenyl)-1-(2-fluoro-pyridin-4-yl)-ethanone (5', Scheme 6), either with NaNO₂ under aqueous conditions with tetrafluoro boric acid solutions (methanol or water) or with isoamyl nitrite. More easily and with higher yields said conversion can be performed with Olah's reagent and sodium nitrite. Nitrosation of the CH-acidic α-carbon position of the diaryl-ethanone was never observed. This makes it necessary to perform the nitrosation/oximation of this intermediate 2-(4-fluoro-phenyl)-1-(2-fluoro-pyridin-4-yl)-ethanone as a separate step. According to WO 2004/ 018458 the nitrosation/ oximation was achieved analogously in glacial acetic acid with sodium nitrite in place of isoamyl nitrite in the presence of sodium methoxide.

In accordance with scheme 1 of WO 02 /066458 the 1-(4-fluoro-phenyl)-2-(2-fluoropyridin-4-yl)-ethane-1,2-dione 1-oxime (7') can be condensed with diverse (R¹ substituted) hexahydro-triazins, which are readily available from paraformaldehyde and corresponding amines, to obtain 3-oxo-imidazol intermediates (8'). For the preparation of the thion intermediate (9') *Mlostons* procedure (Mloston G.; Gendek, T.; Heimgartner, H.; Helv. Chim. Acta. 1998. 81; (9): 1585-1595) was applied to the fluoropyridin-imidazol-N-oxid derivatives. R² can then be introducee by alkylation with iodides or sulfonates in the presence of alkali hydrogen carbonate or alkali carbonate to obtain the 2-sulfanyl-substituted starting materials (10') which can be used for subsequent amination reaction.

Amino-fluoro-replacement reaction can also be performed on the sulfinyl level by first oxidizing the fluoropyridin-sulfanyl compounds according to the above general methods, either with H₂O₂/glacial acetic acid or with the NalO₄ method, and then proceeding as described above.

### General preparation method 5 - Acylation of the 2-aminopyridyl group with acid chlorides

The 2-aminopyridyl compound (1 equivalent) is dissolved in abs. pyridine and the corresponding acid chloride (1 equivalent) is added dropwise. The reaction is completed with stirring at 55 °C (control by TLC)). The pyridine is then removed under vacuum; the residue is taken up in ethyl acetate and washed several times with water. The organic phase is dried with anhydrous sodium sulfate and the ethyl acetate is removed under vacuum. The crude product was purified by column chromatography.

### General preparation method 6 - Acylation of the 2-aminopyridyl group with carboxylic acids

The carboxylic acid (1 equivalent) is dissolved in 50 ml abs. THF under argon atmosphere at room temperature. Carbonyldiimidazole (CDI, 1 equivalent) is then slowly added. After the gas evolution has ceased (1.5 h) pyridyl amine (1 equivalent) is added. The reaction mixture is then stirred at room temperature until completion of the reaction. THF is evaporated; ethyl acetate is added to the residue and washed several times with water. The organic phase is dried with anhydrous sodium sulfate and the ethyl acetate is removed under vacuum. The crude product was purified by column chromatography by means of MPLC (RP-18, acetonitrile : water = 6 : 4).

### Example 1

### {4-[5-(4-Fluorophenyl)-2-methanesulfinyl-3-methyl-3H-imidazol-4-yl]pyridin-2-yl}(1-phenylethyl)amine

### Prepared by general method 1 from {4-[5-(4-fluorophenyl)-2-methanesulfanyl-3-methyl-3H-imidazol-4-yl]pyridin-2-yl}(1-phenylethyl)amine: 76.4% yield (94.9% purity)

C₂₄H₂₃FN₄OS = 434.53951
C 66.34% H 5.34% F 4.37% N 12.89% O 3.68% S 7.38%
¹H-NMR (CDCl₃):δ [ppm]: 8.07-8.03(d,H); 7.36-7.19(m,9H); 7.22-6.88(m,2H); 6.45-6.42(m,2H); 6.13-6.11 (d,H); 3.42-3.417(d,CH₃); 3.20(d,CH₃); 1.61-1.58(d,CH₃)
MS(EI, 70eV): m/z [rel Int. %] = 436 (8), 435 (30), 434 (100), 421 (8), 420 (25), 419 (82), 417 (16), 405 (18), 404 (60), 403 (58), 387 (10), 372 (8), 355 (10), 342 (7), 330 (7), 329 (7), 315 (18), 314 (10), 313 (15), 301 (5), 300 (10), 299 (26), 298 (8), 281 (10), 267 (8), 252 (5), 241 (5), 226 (5), 210 (12), 209 (13), 186 (10), 121 (12), 120 (86), 106 (8), 105 (65), 103 (20), 79 (20), 77 (18)

### Example 2

### {4-[5-(4-Fluorophenyl)-2-methanesulfonyl-3-methyl-3H-imidazol-4-yl]pyridin-2-yl}(1-phenylethyl)amine

### Prepared by general method 1 from {4-[5-(4-Fluorophenyl)-2-methanesulfanyl-3-methyl-3H-imidazol-4-yl]pyridin-2-yl}(l-phenylethyl)amine: 3% yield (80% purity)

C₂₄H₂₃FN₄O₂S = 450.54
C 63.98% H 5.15% F 4.22% N 12.44% O 7.10% S 7.12%
MS(EI, 70eV): m/z (rel Int.%) = 452 (10), 451 (30), 450 (100), 449 (14) 437 (10) 436 (28), 435 (98), 418 (5), 373 (7), 357 (7), 355 (8), 346 (14), 345 (18), 331 (7), 301 (9), 281 (4), 267 (6), 253 (4), 239 (4), 225 (12), 210 (5), 207 (7), 186 (4), 121 (8), 120 (70), 106 (6), 105 (56), 104 (14), 79 (15), 77 (12)

### Example 3

### N-{4-[5-(4-Fluorophenyl)-2-methanesulfinyl-3-methyl-3H-imidazol-4-yl]pyridin-2-yl}acetamide

### Prepared by general method 1 from N-{4-[5-(4-Fluorophenyl)-2-methanesulfanyl-3-methyl-3H-imidazol-4-yl]pyridin-2-yl}acetamide: 80% yield (99.8%)

C₁₈H₁₇FN₄O₂S = 372.42
C 58.05% H 4.60% F 5.10% N 15.04% O 8.59% S 8.61%
¹H-NMR: d (ppm) 8.42-8.28(m, 3H); 7.44-70.26 (m,2H); 7.01-6.91(m, 4H); 3.90-3.85 (s,-SO-CH₃); 3.26 (s,-NC-CH₃); 2.24 (s, NO-CH₃)
¹H-NMR (MeOD): δ [ppm]: 2.17 (s, 1H, CO-CH₃); 3.25 (s, 1H, SO-CH₃); 3.83 (s, 1H, N-CH₃); 6.99-7.03 (m, 2H, C3/5-H, 4F-Ph); 7.08-7.09 (m, 1H, C5-H, Py); 7.44-7.47 (m, 2H, C2/6-H, 4F-Ph); 8.15 (s, br, 1H, C3-H, Py); 8.40-8.42 (m, 1H, C₆-H, Py); exchangeable proton not visible;
¹³C-NMR (MeOD): δ [ppm] 23.96 (CO-CH₃); 32.72 (N-CH₃); 38.28 (SO-CH₃); 116.24 (d, 2C, 2J(C,F)=21.8 Hz, C3/5, 4F-Ph); 116.64 (C3, Py); 122.18 (C5, Py); 130.52 (d, 2C, 3J(C,F)=8.3 Hz, C2/6, 4F-Ph); 130.61; 132.02; 140.34; 140.50; 147.99 (C6, Py); 154.04; 163.81 (d, 1J(C,F)=244.0 Hz, C4, 4F-Ph); 172.34 (C=O)
MS(EI, 70eV): m/z (rel Int. %) = 372 (<1), 348 (7), 347 (21), 346 (100) 345 (21), 283 (4), 281 (8), 268 (5), 267 (9), 266 (7), 265 (6), 252 (5), 250 (5), 241 (7), 240 (6), 238 (4), 225 (5), 224 (4), 212 (4), 211 (5), 210 (8), 199 (5), 134 (8), 93 (8), 66 (8).

Solubility in water 0.07 mg/ml.

According to the general preparation method 3, the following salts of the title compound were prepared:
a) methane sulfonate - from 0.125 g of the title compound 0.140 g of the methane sulfonate were obtained. M.p. 137 °C; yield 99.6%; purity 99.4% (HPLC); solubility in water 0.89 mg/ml.
b) hydrochloride - from 0.125 g of the title compound 0.120 g of the hydrochloride were obtained. M.p. 220.6 °C; yield 98%; purity 99.4% (HPLC); solubility in water 1.12 mg/ml.
c) hydrobromide - from 0.125 g of the title compound 0.120 g of the hydrobromide were obtained. M.p. 220.6 °C; yield 98%; purity 99.4% (HPLC); solubility in water 1.05 mg/ml.
d) hydrogensulfate - from 0.125 g of the title compound 0.140 g of the hydrogensulfate were obtained. M.p. 199.4 °C; yield 99%; purity 99.4% (HPLC); solubility in water 0.39 mg/ml.
e) sulfate - from 0.125 g of the title compound 0.110 g of the sulfate were obtained. M.p. 203.4 °C; yield 88%; purity 99% (HPLC); solubility in water 0.31 mg/ml.

### Example 4

### N-{4-[5-(4-Fluorophenyl)-2-methanesulfonyl-3-methyl-3H-imidazol-4-yl]pyridin-2-yl}acetamide

### Prepared by general method 1 from N-{4-[5-(4-Fluorophenyl)-2-methanesulfanyl-3-methyl-3H-imidazol-4-yl]pyridin-2-yl}acetamide: 17% yield

C₁₈H₁₇FN₄O₃S = 388.42
C 55.66% H 4.41% F 4.89% N 14.42% O 12.36% S 8.25%
MS(EI, 70eV): m/z (rel Int.%) = 390 (5), 389 (20), 388 (100), 373 (10), 347 (10) 346 (50) 345 (60), 283 (5), 281 (7), 268 (5), 267 (12), 266 (10), 265 (10), 252 (5), 250 (7), 240 (5), 238 (6), 225 (6), 224 (5), 211 (7), 210 (8), 199 (5), 43 (30)

### Example 5

### N-{4-[5-(4-Fluorophenyl)-2-methanesulfinyl-3-(2-methoxyethyl)-3H-imidazol-4-yl]pyridin-2-yl}acetamide

### Prepared by general method 1 from N-{4-[5-(4-Fluorophenyl)-2-methanesulfanyl-3-(2-methoxyethyl)-3H-imidazol-4-yl]pyridin-2-yl}acetamide: 30% yield (99% purity)

C₂₀H₂₁FN₄O₃S = 416.48
C 57.68% H 5.08% F 4.56% N 13.45% O 11.52% S 7.70%
¹H-NMR (CDCl₃): δ (ppm): 9.10(H, N-H); 8.408(2H, from N-H and arom.); 7.403-7.267(2H arom.); 6.990-6.908(4H arom.); 4.58-4.41(2H from CH₂); 4.392-4.106(2H from CH₂); 3.373-3.135(S,6H from -SO-CH₃; and -O-CH₃); 2.279-2.047(s, 3H from -NO-CH₃)

### Example 6

### N-{4-[5-(4-Fluorophenyl)-2-methanesulfonyl-3-(2-methoxyethyl)-3H-imidazol-4-yl]pyridin-2-yl}acetamide

### Prepared by general method 1 from N-{4-[5-(4-Fluorophenyl)-2-methanesulfanyl-3-(2-methoxyethyl)-3H-imidazol-4-yl]pyridin-2-yl}acetamide: 7% yield

C₂₀H₂₁FN₄O₄S = 432.48
C 55.55% H 4.89% F 4.39% N 12.95% O 14.80% S 7.41%
1H-NMR (CDCl₃): δ (ppm): 8.315-6.610 (7H from aromat. moiety); 5.497(1H from N-H); 4.56-4.186 (4H from 2XCH₂); 3.261-3.128 (6H from -O-CH₃, -SO₂-CH₃); 1.249-1.116 (3H from -CO-CH₃).

### Example 7

### N-{4-[3-Ethyl-5-(4-fluorophenyl)-2-methanesulfinyl-3H-imidazol-4-yl]-pyridin-2-yl}-acetamid

### Prepared by general method 1 from N-{4-[3-Ethyl-5-(4-fluorophenyl)-2-methanesulfanyl-3H-imidazol-4-yl]-pyridin-2-yl}-acetamid: 72% yield (98.9% purity)

C₁₉H₁₉FN₄O₂S = 386.45
C 59.05% H 4.96% F 4.92% N 14.50% O 8.28% S 8.30%
MS(EI, 70eV): m/z (rel Int. %) = 120.0(6); 225.0 (6); 252.0 (5) ; 297.0(12); 301.0 (20); 302.0 (5); 311.00 (8); 325.00 (7); 339.10 (9); 343.0 (12); 371.0 (100); 372.0 (31); 373.1 (26); 374.1 (6); 389.1 (57); 390.1 (14); 391.1 (5)

### Example 8

### N-{4-[5-(4-Fluorophenyl)-2-methanesulfinyl-3-(3-methoxypropyl)-3H-imidazol-4-yl]pyridin-2-yl}acetamide

### Prepared by general method 1 from N-{4-[5-(4-Fluorophenyl)-2-methanesulfanyl-3-(3-methoxypropyl)-3H-imidazol-4-yl]pyridin-2-yl}acetamide: 58% yield (>99% purity)

C₂₁H₂₃FN₄O₃S = 430.50
C 58.59% H 5.39% F 4.41% N 13.01% O 11.15% S 7.45%
MS (EI, 70eV, Scan 1143/8.379 min.): m/z (rel Int.) = 341.0 (10); 430.1(30); 415.1 (28); 414.1 (77); 399.0 (11); 369.0 (15); 368.1 (26); 367.1 (100); 357.0 (12); 355.0 (21); 336.1 (10); 335.1 (37);325.0 (11); 323.1 (14); 309.0 (21); 293.0 (13); 283.0(11); 282.0 (16); 281.0 (14); 268.0 (10); 267.00 (33); 240.0 (12); 206.9 (16); 121.0 (12); 73.0 (18); 71.0 (76); 45.0 (49); 44.0 (10); 43.0 (25); 41.0 (10); 32.0 (11)

### Example 9

### {4-[5-(4-Fluorophenyl)-2-methanesulfinyl-3-(2-methoxyethyl)-3H-imidazol-4-yl]pyridin-2-yl}-(1-phenylethyl)amine

### Prepared by general method 1 from {4-[5-(4-Fluorophenyl)-2-methanesulfanyl-3-(2-methoxyethyl)-3H-imidazol-4-yl]pyridin-2-yl}-(1-phenylethyl)amine: 71 % yield (>99% purity)

C₂₆H₂₇FN₄O₂S = 478.59
C 65.25% H 5.69% F 3.97% N 11.71% O 6.69% S 6.70%
IR (ATR), A [cm⁻¹] = 2928, 1606, 1502, 1448, 1219, 1116, 1047, 1015,957, 839, 812, 763,7001,658,593
MS (EI, 70eV, Scan 1659 / 11.445 min.): m/z (rel Int. %) = 103.0 (11); 105.0 (38); 120.0 (44); 343.0 (13); 357.1 (11); 447.1 (51); 448.1 (18); 461.2 (13); 462.2 (100); 463.1 (46); 464.2 (14); 479.2 (10); 478.2 (33);

### Hydrochloride

### Mixture of RR/SS and RS/SR diastereomeric enantiomer pairs of {4-[5-(4-fluorophenyl)-2-methanesulfinyl-3-(2-methoxyethyl)-3H-imidazol-4-yl]pyridin-2-yl}(1-phenylethyl)amine hydrochloride

Prepared by general method 1: 65% yield (>99% purity)
C₂₆H₂₈ClFN₄O₂S = 515.05
C 60.63% H 5.48% Cl 6.88% F 3.69% N 10.88% O 6.21 % S 6.23%
¹H-NMR (DMSO-d6):δ [ppm] = 9.6 (b, 1H, NH+); 8.05 (b, 1H, NH+); 7.5-7.4 (m, 6H); 7.4-7.3 (m, 2H) 7.3-7.25 (m, 1H); 7.2-7.1 (m, 3H), 6.82-6.78 (m, 1H), 3.123 (s, 3H, NCH₃); 3.09 (s, 3 H, SOCH₃); 1.540 (d, 3H, J=6.8 Hz, CH₃CH-)

### Example 10

### {4-[5-(4-Fluorophenyl)-2-methanesulfinyl-3-(3-methoxypropyl)-3H-imidazol-4-yl]pyridin-2-yl}(1-phenylethyl)amine

### Prepared by general method 1 from {4-[5-(4-Fluorophenyl)-2-methanesulfanyl-3-(3-methoxypropyl)-3H-imidazol-4-yl]pyridin-2-yl}(1-phenylethyl)amine: 76% yield (96% purity)

C₂₇H₂₉FN₄O₂S = 492.62
C 65.83% H 5.93% F 3.86% N 11.37% O 6.50% S 6.51%
IR (ATR), λ [cm⁻¹] = 3250, 2978, 1606, 1548, 1500, 1437, 1219, 1118, 1047, 957, 839, 812, 762, 700, 605
¹H-NMR (DMSO-d6):δ (ppm) : (Gu1671 10mg/0.8ml); 8.07 (d, 1H, J=4.8Hz), 7.42 (AB; 2H, J=5.6Hz), 7.34-7.17(m; 7H), 7.12 (t, 2H, J=8.8Hz) 6.47 (AB, 1H, J=1.2Hz); 5.0(t; 1H, J=6.8Hz), 4.11-4.02 (m, 2H), 3.19-3.12(m; 5H), 3.416/3.375 (br,2H, CH2-); 3.07(s;CH3), 1.75-1.70(m; 2H), 1.43(d, CH3, J=7.2Hz)
MS(EI, 70eV): m/z (rel Int. %) = 492(30), 476(100), 461(40), 430

### Example 11

### {4-[5-(4-Fluorophenyl)-2-methanesulfinyl-3-(3-methoxypropyl)-3H-imidazol-4-yl]pyridin-2-yl}isopropylamine

### Prepared by general method 1 from {4-[5-(4-Fluorophenyl)-2-methanesulfanyl-3-(3-methoxypropyl)-3H-imidazol-4-yl]pyridin-2-yl}isopropylamine: 40% yield (95% purity)

C₂₂H₂₇FN₄O₂S = 430.55
C 61.37% H 6.32% F 4.41% N 13.01% O 7.43% S 7.45%
IR (ATR, A = [cm⁻¹]) = 2965.2; 2926.1; 2869.3; 1605.5; 1546.8; 1520.3; 1500.9; 1479.9; 1463.0; 1382.8;1363.4; 1282.9; 1220.9; 1173.6; 1157.2; 1118.6; 1036.4 ; 970.1; 956.5; 839.3; 810.0; 743.2; 605.3
¹H-NMR: δ (ppm) = (10 mg/0.8ml DMSO-d6): 8.12(d;1H, J=5.2Hz), 7.49-7.46(m; 2H), 7.18-7.13(m; 2H), 6.58(d; 1H; J=7.6Hz), 6.48(d;1H, J=4.8Hz), 6.42(s; NH), 4.16(t; 2H, J=8.0Hz), 4.02-3.97(m; CH), 3.27-3.20(m; 2H), 3.16(s; CH₃), 3.09(s; CH₃), 1.87-1.80(m; 2H), 1.15(s; CH₃), 1.13(s; CH₃)
MS(EI, 70eV): m/z (rel Int. %) = 430(60), 414(100), 399(44), 367(63), 309(26)

### Example 12

### {4-[5-(4-Fluorophenyl)-2-methanesulfinyl-3-(2-methoxyethyl)-3H-imidazol-4-yl]pyridin-2-yl}isopropylamine

### Prepared by general method 1 from {4-[5-(4-Fluorophenyl)-2-methanesulfanyl-3-(2-methoxyethyl)-3H-imidazol-4-yl]pyridin-2-yl}isopropylamine: 89% yield (>99% purity)

C₂₁H₂₅FN₄O₂S = 416.52
C 60.56% H 6.05% F 4.56% N 13.45% O 7.68% S 7.70%
IR (ATR, cm⁻¹) = 3350, 2970, 1739, 1613, 1521, 1501, 1456, 1367, 1345, 1218, 1040,841,829,812,662,594
¹H-NMR: (10mg/0.8mlDMSO): δ (ppm) = 8.11 (d; 1H, J=6.0Hz); 7.49-7.46(m; 2H), 7.18-7.13(m; 2H), 6.57(d; 1H, J=7.6Hz); 6.48(d; 1H, J=6.8Hz), 6.42(t; NH, J=0.8Hz); 4.27(m; 1H), 4.20-4.16(m; 1H), 3.14(s; CH₃), 3.13(s; CH₃), 1.15(s; CH₃), 1.13(s; CH₃)
MS(EI, 70eV): m/z (rel Int. %) = 146(11); 369.10(17); 385.1(55); 293.(15); 386.1(20); 311.0(14); 400.1(100); 312.0(11); 401.1(67); 327.0(23); 402.1(17); 328.0(15); 416.1(42); 343.0(18) 357.1(23)

### Example 13

### {4-[5-(4-Fluorophenyl)-2-methanesulfonyl-3-(3-methoxypropyl)-3H-imidazol-4-yl]pyridin-2-yl}isopropylamine

### Prepared by general method 2 from {4-[5-(4-Fluorophenyl)-2-methanesulfanyl-3-(3-methoxypropyl)-3H-imidazol-4-yl]pyridin-2-yl}isopropylamine: 50% yield (77% purity)

C₂₂H₂₇FN₄O₃S = 446.55
C 59,18% H 6.09% F 4.25% N 12.55% O 10.75% S 7.18%
IR (ATR, cm⁻¹) = 3298.5 2960.1; 2913.6; 1615.2; 1545.2; 1524.1; 1502.7; 1484.2; 1404.8; 1380.8; 1324.1; 1296.5; 1286.5; 1213.8; 1150.1; 1126.9; 1092.4; 1022.2; 976.0; 924.4; 879.2; 843.8; 812.9; 773.1; 741.1; 719.3; 608.1; 570.4
MS(EI, 70eV): m/z (rel Int. %) Scan 670 (4.703 min): 447.10 (27); 446.1 (100); 432.1 1 (26); 431.0 (85); 368.0 (21); 367.0 (79); 351.0 (22); 326.0 (22); 325.0 (68); 310.0 (27); 309.0 (33); 295.0 (24); 293.1 (27); 281.0 (34); 267.0 (41); 207.0 (48)

### Example 14

### N-{4-[3-Ethyl-5-(4-fluorophenyl)-2-methanesulfonyl-3H-imidazol-4-yl]pyridin-2-yl}acetamide

### Prepared by general method 1 from N-{4-[3-Ethyl-5-(4-fluorophenyl)-2-methanesulfanyl-3H-imidazol-4-yl]pyridin-2-yl}acetamide: 2% yield (96% purity)

C₁₉H₁₉FN₄O₃S = 402.45
C 56.71% H 4.76% F 4.72% N 13.92% O 11.93% S 7.97%
MS(EI, 70eV Scan 1638 (11.823 min:): m/z (rel Int. %) = 309.0 (13); 337.1 (33); 355.0 (17); 369.0 (18); 370.0 (100); 371.0 (24)

### Example 15

### Cyclohexyl-{4-[5-(4-fluorophenyl)-2-methanesulfinyl-3-methyl-3H-imidazol-4-yl]pyridin-2-yl}amine

### Prepared by general method 1 from Cyclohexyl-{4-[5-(4-fluorophenyl)-2-methanesulfanyl-3-methyl-3H-imidazol-4-yl]pyridin-2-yl}amine: 98% yield (85% purity)

C₂₂H₂₇FN₄O₃ = 446.55,
C 64.05% H 6.11% F 4.61% N 13.58% O 3.88% S 7.77%
IR (ATR) A [cm⁻¹] = 3250,2928,2852,1739,1605,1500,1448,1366,1218,1156, 1048,971,839,811,658,589,562
¹H-NMR (10mg/0.8mlDMSO-d6): δ [ppm]: 8.10(d; 1H, J=4.8Hz); 7.49-7.46(m; 2H); 7.18-7.14(m; 2H), 6.58(d; 1H, J=7.6Hz), 6.48-6.41(m; 2H), 3.66(s; CH), 3.32(s; 3H), 3.15(s; 3H), 1.92-1.89(m;2H), 1.71-1.57(m;3H), 1.32-1.16 (m;5H)
MS(EI, 70eV): m/z [rel Int. %] = 412, 353, 339, 327, 313(100), 299, 266

### Example 16

### Cyclopentyl-{4-[5-(4-fluorophenyl)-2-methanesulfinyl-3-methyl-3H-imidazol-4-yl]pyridin-2-yl}amine

### Prepared by general method 1 from Cyclopentyl-{4-[5-(4-fluorophenyl)-2-methanesulfanyl-3-methyl-3H-imidazol-4-yl]pyridin-2-yl}amine: 90% yield (96% purity)

C₂₁H₂₃FN₄OS = 398.51
C 63.29% H 5.82% F 4.77% N 14.06% O 4.01% S 8.05%
IR (ATR) λ [cm⁻¹] (int.) = 1613.2 (0.204); 1522.3 (0.226);1501.7 (0.287); 1484.9 (0.196); 1381.7 (0.118); 1356.8 (0.138); 1299.1 (0.107); 1213.4 (0.228); 1161.5 (0.134); 1034.1 (0.242); 1015.2 (0.122); 972.2 (0.164); 873.5 (0.181); 811.4 (0.282); 742.0 (0.155); 715.3 (0.119); 659.2 (0.158); 589.4 (0.234)
¹H-NMR (10mg/0.8mlDMSO-d6): δ [ppm]: 8.11 (d;1H; J=5.2Hz); 7.48 (AB,2H, J=5.2Hz); 7.16 (t; 2H, J=8.4Hz); 6.72 (d; 1H, J=6.4Hz); 6.48 (d; 1H, J=6.4Hz), 6.41 (s,1H); 4.08(q; 1H; J=6.4Hz); 3.67 (s;2H); 3.32 (s; 6H); 3.15 (s; 3H) 1.89 (quin, 2H; J=6.4Hz) 1.67-1.64(m, 2H) 1.55-1.51 (m, 2H), 1.44-1.39 (m; 2H)
MS(EI, 70eV): m/z [rel Int. %] = 389(14), 382(84), 353(14), 329(18); 313(100), 299(15), 241 (13); 206(13)

### Example 17

### Cycloheptyl-{4-[5-(4-fluorophenyl)-2-methanesulfinyl-3-methyl-3H-imidazol-4-yl]pyridin-2-yl}amine

### Prepared by general method 1 from Cycloheptyl-{4-[5-(4-fluorophenyl)-2-methanesulfanyl-3-methyl-3H-imidazol-4-yl]pyridin-2-yl}amine: 94% yield (96% purity)

C₂₃H₂₇FN₄OS = 426.56
C 64.76% H 6.38% F 4.45% N 13.13% O 3.75% S 7.52%
IR (ATR), λ [cm⁻¹] (int.) =: 1601.6 (0.331); 1570.6 (0.292); 1549.7 (0.318); 1498.9 (0.369); 1437.1 (0.339); 1377.3 (0.260); 1249.7 (0.213); 1218.2 (0.490); 1053.3 (0.488); 1011.6 (0.218); 949.1 (0.247); 847.3 (0.562); 823.5 (0.320); 813.9 (0.481); 740.7 (0.216); 707.3 (0.326); 685.6 (0.200); 659.7 (0.367); 618.4 (0.267); 588.7 (0.444)
¹H-NMR (DMSO) δ [ppm] : 8.11 (d; 1H; J=5.2HZ); 7.47(q; 2H, J=5.6Hz); 7.16(t, 2H, J=8.8Hz); 6.61 (d; 1H, J=8.0Hz); 6.47 (d, 1H, J=4.8Hz), 6.39(s; 1H); 3.70 (s; 3H); 3.15 (s; 2H); 1.88-1.86(m; 3H) 1.62-1.42 (m, 13H)
MS(EI, 70eV): m/z [rel Int. %] = 426(29); 410(55), 355(43), 339(58), 329(52), 313(100), 281 (39), 208(18), 132(30)

### Example 18

### Cyclohexyl-{4-[5-(4-fluorophenyl)-2-methanesulfinyl-3-(2-methoxyethyl)-3H-imidazol-4-yl]pyridin-2-yl}amine

### Prepared by general method 1 from Cyclohexyl-{4-[5-(4-fluorophenyl)-2-methane-sulfanyl-3-(2-methoxyethyl)-3H-imidazol-4-yl]pyridin-2-yl}amine: 61% yield (>99% purity)

C₂₄H₂₉FN₄O₂S = 456.59
C 63.14% H 6.40% F 4.16% N 12.27% O 7.01% S 7.02%
¹H-NMR (CDCl₃) δ [ppm]: 8.145 (d, 1H, J=5.2 Hz); 7.515-7.47 (m, 2H); 6.97-6.92 (m, 2H); 6.5135 (d, 1H J=5.2 Hz); 6.333 (s,1H), 4.872 (d, 1H, J=7.6Hz); 4.54-4.46 (m, 1H), 4.28-4.215 (m, 1H); 3.63-3.485 (m, 2H); 3.45-3.38 (m 1H); 3.264 (s, 3H); 3.232 (s, 3H); 1.99-1.95 (m, 2H); 1.77-1.72 (m, 2H); 1.650-1.61 (m, 1H); 1.40-1.15 (m, 5H)
MS(EI, 70eV, Scan 1810 10.854 min): m/z (rel Int.%) = 457.2 (27); 456.2 (95); 455.2 (12); 442.2 (10); 441.2 (34); 440.2 (48); 399.1 (29); 393.2 (11); 384.1 (18); 383.1 (17); 381.1 (11); 375.1 (15), 374.1 (54); 373.1 (100); 367.1 (17); 359.1 (39); 358.1 (26); 357.1 (51); 343.0 (10); 327.1 (14); 326.0 (14); 316.0 (13); 313.0 (14); 312.1 (22); 311.1 (93); 301.0 (21); 291.0 (11); 281.0 (10); 277.0 (11); 267.0 (23); 98.1 (13)

### Example 19

### Cyclopentyl-{4-[5-(4-fluorophenyl)-2-methanesulfinyl-3-(2-methoxyethyl)-3H-imidazol-4-yl]pyridin-2-yl}amine

### Prepared by general method 1 from Cyclopentyl-{4-[5-(4-fluorophenyl)-2-methane-sulfanyl-3-(2-methoxyethyl)-3H-imidazol-4-yl]pyridin-2-yl}amine: 67% yield (>99% purity)

C₂₃H₂₇FN₄O₂S = 442.56
C 62.42% H 6.15% F 4.29% N 12.66% O 7.23% S 7.25%
¹H-NMR (CDCl₃) δ [ppm]: 8.147 (d, 1H, J=4.8 Hz); 7.52- 7.47 (m, 2H); 6.98- 6.91 (m, 2H); 6.526 (d, 1H J=4.4 Hz); 6.391 (s,1H), 5.053 (d, 1H, J=6Hz); 4.54-4.465 (m, 1H), 4.285- 4.220 (m, 1H); 3.91- 3.85 (m, 1H); 3.63- 3.45 (m 2H); 3.262 (s, 3H,); 3.234 (s, 3H); 2.01 -1.94 (m, 2H); 1.79- 1.695 (m, 2H); 1.67-1.58 (m, 2H); 1.51 (m, 2H)
MS(EI, 70eV): m/z (rel Int. %) = 442(90%), 426(100%), 357(95%), 311(78%), 267(25%), 301(25%), 327(18%),397(14%)

### Example 20

### Cycloheptyl-{4-[5-(4-fluorophenyl)-2-methanesulfinyl-3-(2-methoxyethyl)-3H-imidazol-4-yl]pyridin-2-yl}amine

### Prepared by general method 1 from Cycloheptyl-{4-[5-(4-fluorophenyl)-2-methane-sulfanyl-3-(2-methoxyethyl)-3H-imidazol-4-yl]pyridin-2-yl}amine: 42% yield (95% purity)

C25H31FN4O2S = 470.61
C 63.81% H 6.64% F 4.04% N 11.91% O 6.80% S 6.81%
IR (ATR), λ [cm⁻¹] (int.) =: 3392, 2931, 2355, 1613, 1523, 1499, 1104, 1053, 848
1H-NMR (CDCl3) δ [ppm]: 8.07-8.06 (d,2H); 7.49-7.45(m,2H); 6.99-6.39 (t,2H); 6.56-6.54 (d,1H); 6.39(s,1H); 5.75 (b,NH); 4.51-4.49 (m,1H,CH2) 4.28-4.25 (m,1H,CH2); 3.63-3.54 (m,2H,CH2); 3.27 (s,3H,OCH3); 3.22 (s,3H,SOCH3); 1.93-1.89 (m,2H,CH2 cyclohept) 1.66-1.55 (m,10H,cyclohept.)

The compounds listed in Table 5 were obtained in an analogous manner from the corresponding sulfanyl compounds:

**Table 5**

| Example No. | Compound | m.p. (°C) |
|---|---|---|
| 21 | 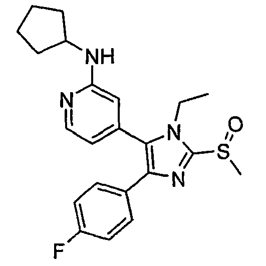 | 158 |
| 22 | 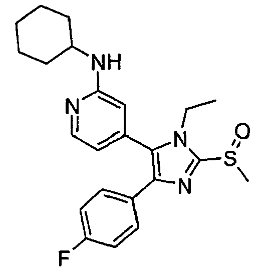 | 141 |
| 23 | 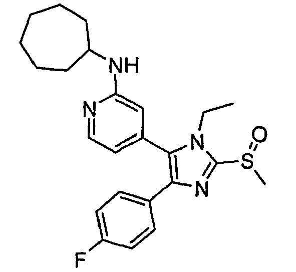 | |
| 24 | 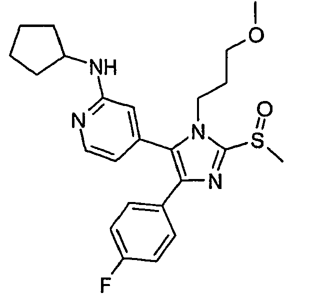 | 110 |
| 25 | 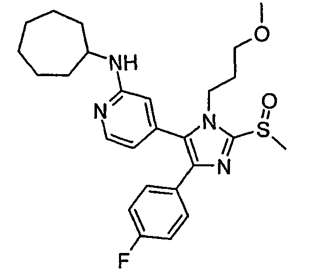 | 134 |
| 26 | 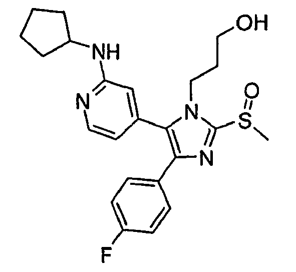 | 148 |
| 27 | 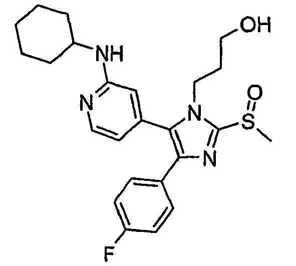 | 145 |
| 28 | 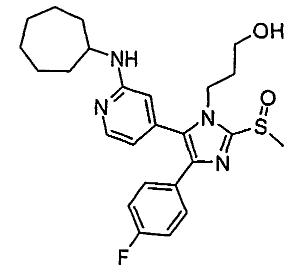 | |

### Example 29

### (+)-N-{4-[5-(4-Fluorophenyl)-2-methanesulfinyl-3-methyl-3H-imidazol-4-yl]pyridin-2-yl}acetamide (1)

### (-)-N-(4-[5-(4-Fluorophenyl)-2-methanesulfinyl-3-methyl-3H-imidazol-4-yl]pyridin-2-yl)acetamide (2)

The racemate obtained in Example 3 was fractionated into the enantiomers under the following experimental conditions:
Chiralpak AD 10 µm; isocratic hexane-isopropanol 30/70, 0.5 ml/min.; analytical column 250 mm x 4.6; amount injected 0.05 ml (resolution = 1.11)
RT1=16.113; (RT = retention time)
RT2=17.486

### Example 30

### Cyclohexyl-{4-[5-(4-fluorophenyl)-2-methanesulfinyl-3-(2-methoxypropyl)-3H-imidazol-4-yl]pyridin-2-yl}amine

Prepared by general method 1:
0.44 g 66% yield (98% purity)

| | | | | | | | | | | | |
|---|---|---|---|---|---|---|---|---|---|---|---|
| C25H31FN4O2S = 470.61 | | | | | | | | | | | |
| C | 63.81% | H | 6.64% | F | 4.04% | N | 11.91% | O | 6.80% | S | 6.81% |

### A) 4-[5-(4-Fluorophenyl)-3-(3-methoxypropyl)-2-methylsulfanyl-3H-imidazol-4-yl]pyridin-2-ylamine

N-{4-[5-(4-fluorophenyl)-3-(3-methoxypropyl)-2-methylsulfanyl-3H-imidazol-4-yl]pyridin-2-yl}acetamide (37.3 g, 0.09 mol) is dissolved or suspended in 10% strength HCl (760 ml) and, after heating to 70°C, completely hydrolyzed in 4 h. The reaction is monitored by thin-layer chromatography (TLC: SiO₂/EA-n-hexane 8:2) The mixture is cooled to 0°C in an ice bath, neutralized with NaOH (32%) and extracted twice with ethyl acetate. The ethyl acetate extracts are washed with water, dried over Na₂SO₄ and, after filtration, concentrated in vacuo. Crystalline product separates out of the oily residue from evaporation which has been taken up in diisopropyl ether. The crystals (31.7 g) which have been filtered off with suction and washed are dried in vacuo. Yield 95.5% (100% purity).

| | | | | | | | | | | | |
|---|---|---|---|---|---|---|---|---|---|---|---|
| C19H21FN4OS = 372.47 | | | | | | | | | | | |
| C | 61.27% | H | 5.68% | F | 5.10% | N | 15.04% | O | 4.30% | S | 8.61% |

IR (ATR), λ= [cm⁻¹]: 3389.1 (0.111); 3313.9 (0.0462); 3144.5 (0.105); 2924.9 (0.0842); 1649.3 (0.143); 1616.6 (0.213); 1564.4 (0.163); 1544.4 (0.274); 1507.5 (0.431); 1485.5 (0.224); 1460.2 (0.175); 1441.3 (0.265); 1405.6 (0.193); 1390.5 (0.158); 1370.5 (0.147); 1343.2 (0.144); 1325.4 (0.107); 1300.4 (0.134); 1290.7 (0.118); 1256.6 (0.151); 1213.2 (0.347); 1156.4 (0.188); 1127.9 (0.162); 1106.7 (0.347); 1075.9 (0.172); 1025.9 (0.11); 998.9 (0.122); 978.1 (0.142); 905.4 (0.058); 882.9 (0.195); 864.3 (0.138); 840.5 (0.369); 821.4 (0.173); 809 (0.329); 754.1 (0.092); 731.8 (0.175); 709.7 (0.201); 687.9 (0.174); 612.6 (0.308); 572.1 (0.295)
1H-NMR (CDCl3): δ [ppm] : 8.088 (d, 1H,arom.); 7.459-7.423(m, 2H, 4-F-ph.); 6.962-6.910 (m, 2H, 4-F-ph.); 6.597-6.579 (d, 1H,arom.); 6.469 (d, 1H,arom.); 4.907(s, 1H, NH); 3.974-3.936 (m, 2H, CH₂); 3.293-3.271 (m, 2H, CH₂); 3.234 (s, 3H, CH₃); 2.717(s, 3H, CH₃); 1.871-1.803 (m, 2H, CH₂)
MS(EI, 70eV): m/z [rel Int. %] = 372(100), 357(13), 325(14), 267(28), 240(18)

### B) 2-Fluoro-4-[5-(4-fluorophenyl)-3-(3-methoxypropyl)-2-methylsulfanyl-3H-imidazol-4-yl]pyridine

4-[5-(4-Fluorophenyl)-3-(3-methoxypropyl)-2-methylsulfanyl-3H-imidazol-4-yl]pyridin-2-ylamine (7.45 g, 0.02 mol) is taken up in tetrafluoroboric acid (30.03 g , 50% strength, 0.17 mol). The solution of sodium nitrite (1.62 g, 0.033 mol) in water (2.5 ml) is added dropwise to the suspension of the tetrafluoroborate salt at -10 to -15°C. The mixture is then warmed and stirred at this temperature for 1 h. Cooling to -10°C is followed by neutralization with 10% strength NaOH and extraction with 2 volume aliquots of ethyl acetate, and the extracts which have been washed with water and dried over Na₂SO₄ are concentrated. Purification took place by column chromatography on SiO₂ with ethyl acetate as eluent. 1.1 g were obtained. Yield 14.6% (99.6% purity).
It was possible to recover 3.9 g of precursor (52%) from the column with MeOH.

| | | | | | | | | | | | |
|---|---|---|---|---|---|---|---|---|---|---|---|
| C₁₉H₁₉F₂N₃OS = 375.44 | | | | | | | | | | | |
| C | 60.78% | H | 5.10% | F | 10.12% | N | 11.19% | O | 4.26% | S | 8.54% |

IR (ATR), λ = [cm⁻¹]: 1616 (0.131); 1604.9 (0.147); 1541.6 (0.157); 1507.8 (0.256); 1466.3 (0.104); 1441.1 (0.124); 1399.2 (0.282); 1384.5 (0.185); 1365 (0.137); 1333.5 (0.159); 1317.1 (0.0931); 1261.3 (0.153); 1217.4 (0.283); 1187.4 (0.218); 1161.5 (0.172); 1114.9 (0.298); 1032.1 (0.0931); 1018 (0.0974); 996.2 (0.102); 984.2 (0.126); 907.4 (0.0936); 876.6 (0.405); 843.9 (0.541); 815.6 (0.207); 774.5 (0.126); 756 (0.113); 733.3 (0.175); 710.4 (0.153), 697.2 (0.137); 666.3 (0.177); 610.7 (0.258); 573.2 (0.107); 559.7 (0.294)
¹H-NMR (CDCl₃): δ [ppm] : 8.2815 (d, J=5.2 Hz, 1H, 2-F-py); 7.402-7.349 (m, 2H, 4-F-ph.); 7.124-7.107 (m, 1H, 2-F-py) ; 6.981-6.930 (m, 2H, 4-F-ph.); 6.902-6.893 (m, 1H, 2-F-py); 4.010-3.972 (m., 2H,CH₂); 3.264 (t, 2H,CH₂); 3.206 (s, 3H,CH₃); 2.768 (s, 3H,CH₃); 1.873 - 1.807 (2H,CH₂)
MS(EI, 70eV): m/z [rel Int. %] = 377.2 (8); 376.2 (26); 375.2 (100); 361.2 (4); 360.2 (16); 331.1 (4) 330.1 (19); 329.2 (5); 328.2 (20); 317.1 (13); 316.1 (11); 302.1 (11)296.1 (12); 285.1 (5); 284.1 (26); 271 (9); 270.1 (22); 244.1 (7); 243.1 (17); 221.1 (10); 216.1 (13); 215.1 (6); 189.1 (9); 123.1 (7); 122.0 (7); 121.1 (12)

### C) Cyclohexyl-{4-[5-(4-fluorophenyl)-3-(3-methoxypropyl)-2-methylsulfanyl-3H-imidazol-4-yl]pyridin-2-yl}amine

2-Fluoro-4-[5-(4-fluorophenyl)-3-(3-methoxypropyl)-2-methylsulfanyl-3H-imidazol-4-yl]pyridine (1.5 g, 4 mmol) is taken up in cyclohexylamine (4.05 g 40 mmol), and the mixture is heated at 130°C for 16 h. After cooling, the reaction mixture is poured into water and extracted with ethyl acetate. The ethyl acetate phase is washed with water until free of amine, dried over Na₂SO₄ and concentrated in vacuo. The residue is separated by column chromatography on silica gel with ethyl acetate-n-hexane (1:1). The initially oily main fraction (1.4 g, 77%) crystallizes from n-hexane. 1 g of title compound is obtained in a yield of 55% and in >99% purity.

| | | | | | | | | | | | |
|---|---|---|---|---|---|---|---|---|---|---|---|
| C₂₅H₃₁FN₄OS = 454.61 | | | | | | | | | | | |
| C | 66.05% | H | 6.87% | F | 4.18% | N | 12.32% | O | 3.52% | S | 7.05% |

IR (ATR), λ = [cm⁻¹]: 3227.0 (0.104); 2924.7 (0.164); 2853.2 (0.115); 2820.9 (0.0789); 1605.7 (0.183); 1568 (0.388); 1504 (0.399); 1439.4 (0.31); 1398 (0.134); 1381.7 (0.141); 1367.4 (0.181); 1337 (0.197); 1302.8 (0.126); 1290.9 (0.151); 1248.7 (0.179); 1215.5 (0.357); 1156.4 (0.185); 1129.5 (0.226); 1110.7 (0.366); 1093.5 (0.251); 1027.3 (0.118); 1005.8 (0.107); 976.1 (0.201); 905.9 (0.104); 885.9 (0.191); 833.1 (0.549); 810.1 (0.338); 779.4 (0.114); 750.4 (0.107); 729 (0.192); 711.1 (0.223); 690 (0.212); 675.4 (0.231); 610.8 (0.277); 588.8 (0.178)
¹H-NMR (CDCl₃): δ [ppm] : 8.1085 (d, J=5.2 Hz, 1H,py.); 7.474-7.461 (m, 2H, 4-F-ph.); 6.929-6.886 (m, 2H, 4-F-ph.); 6.467 (d, J= 4Hz, 1H, py.); 6.220 (s., 1H, py.); 4.586-4.567(d, J=7.6 Hz,1H,NH); 3.972-3.9356(m, 2H,CH₂); 3.397-3.378(m, 1H,cyclohexyl); 3.280-3.252 (m, 2H,CH₂); 3.214 (s, 3H,CH₃); 2.701 (s, 3H,CH₃); 1.947-1.593(m, 8H,cyclohexyl); 1.351-1.116 (m, 4H, cyclohexyl)
MS(EI, 70eV): m/z [rel Int. %] = 454(100), 371 (66); 397(22); 325(29)

### D) Cyclohexyl-{4-[5-(4-fluorophenyl)-2-methanesulfinyl-3-(3-methoxypropyl)-3H-imidazol-4-yl]pyridin-2-yl}amine

Reaction by general method 1 (H₂O₂ / glacial acetic acid). Crystallizes from diisopropyl ether: 0.44 g, 62% yield (HPLC: 98%-hplc: file 050120_E001)
IR (ATR), λ = [cm⁻¹]: 3316.9 (NH), 2926.1 (CH), 2852.1 (CH); 1605.2 (0.265); 1546.0 (0.193); 1520.0 (0.255); 1500.1 (0.280); 1479.9 (0.199); 1448.0 (0.167); 1408.3 (0.144);1363.4 (0.156); 1220.7 (0.231); 1157.0 (0.169); 1117.6 (0.265); 1042.2 (0.287); 971.8 (0.214); 957.5 (0.169); 881.6 (0.145);839.2 (0.377); 809.6 (0.257); 742.9 (0.153); 708.3 (0.163); 688.8 (0.161); 658.2 (0.166); 606.3 (0.275)
¹H-NMR (CDCl₃): δ [ppm]: 8.17 (d, 4.8 Hz, 1H, Py,); 7.493-7.458 (m, 2H, 4-F-ph.); 6.975-6.931 (m, 2H , 4-F-ph.); 6.4895 (d, J=3.6 Hz., Py.); 6.247 (s, 1H, py.) ; 4.679 (d, br. J=7.2 Hz, 1H, NH) ; 4.439-4.365/4.275-4.202 (m, 2H, CH₂, AB-(2); 3.442 -3.36 (m, 1H, cyclohexyl); 3.364-3.272 (m, 2H, CH₂) 3.250 (s, 3H, CH₃); 3.210 (s, 3H, CH₃); 2.1-1.8 (m, 5H, cyclohexyl + H₂O); 1.8-1.7 (m, 2H, cyclohexyl); 1.7-1.6 (m, 1H, cyclohexyl); 1.4-1.1 (m, 6H, cyclohexyl)
MS(EI, 70eV): m/z [rel Int. %] = m/z=470 (30), 454(78), 407(23), 371(56), 325 (100)

### Example 31

### Cyclohexyl-{4-[6-(4-fluoro-phenyl)-1-oxo-2,3-dihydro-1H-1λ⁴-imidazo[2,1-b]thiazol-5-yl]-pyridin-2-yl}-amine

### A) N-{4-[6-(4-Fluorophenyl)-2,3-dihydro-imidazo[2,1-b]thiazol-5-yl]-pyridin-2-yl}-acetamide

N-{4-[5-(4-Fluorophenyl)-3-(2-hydroxy-ethyl)-2-thioxo-2,3-dihydro-1H-imidazol-4-yl]-pyridin-2-yl}-acetamide (0.37 g) obtained according to WO 02/066458 was dissolved at 60°C in dry pyridine (6.5 mL). Then, methane sulfonylchloride (0.12 mL) was added. The reaction was kept at this temperature until conversion of the methane sulfonate formed as intermediate into the cyclisation product was complete (3 h). Subsequently, the pyridine solution was added dropwise to ice water (30 mL) and the obtained white voluminous solid was suction filtered and washed several times with water. The material was dried under vacuum over P₂O₅. 200 mg (56%) of the title compound with purity of 99.6% (HPLC) were obtained.

### B) 4-[6-(4-Fluorophenyl)-2,3-dihydro-imidazo[2,1-b]thiazol-5-yl]-pyridin-2-ylamine

N-{4-[6-(4-Fluorophenyl)-2,3-dihydro-imidazo[2,1-b]thiazol-5-yl]-pyridin-2-yl}-acetamide obtained in step A) (1.06 g) was heated to reflux in 10% aqueous HCl (25 mL) until hydrolysis was complete (16 h). The reaction mixture was then cooled in an ice bath and neutralized with ice cold NaOH (10% ig) to pH 8. The precipitate was filtered off, washed with water until the wash water was free of electrolytes and dried under vacuum (45 °C, 20 bar) for 16 h.
Yield: 0.9 g (96%); purity (HPLC): 96%
¹H-NMR: ppm(DMSO); 8.0 (d; 1H, J=4Hz); 7.41 (q; 2H, J=6.0Hz); 7.09 (t; 2H, J=8Hz), 6.47(d; 1H, J=4Hz), 6.37 (s; 1H), 6.06(s; 1H), 3.80 (t; 2H, J=4.0Hz), 3.21 (t; 2H, J=4Hz), 2.19 (m; 2H).

### C) 6-(4-Fluorophenyl)-5-(2-fluoro-pyridin-4-yl)-2,3-dihydro-imidazo[2,1-b]thiazole

The amine obtained in step B) (0.15 g) was taken up in 0.5 mL Olah's Reagenz (HF 70% in Pyridin) at -20 °C in a Falcon tube. NaNO₂ (about 0.05 g) was added in 2 small portions.The reaction mixture was maintained at -20 °C for 2 additional hours and then warmed to room temperature.
The reaction mixture was then added into a two-phase system of water and CH₂Cl₂ (1:1; 80 mL). The phases were vigorously shaken and after separation the organic phase was washed with water (40 mL). The organic phase was then dried over Na₂SO₄, filtered and concentrated. The compound was recrystallized from isopropanol. 0.08 g (52%) of the title compound were obtained.
MS(EI, 70eV): m/z (rel Int. %) = 315 (100); 286 (10); 165 (10); 121 (18).

### D) Cyclohexyl-{4-[6-(4-fluorophenyl)-2,3-dihydro-imidazo[2,1-b]thiazol-5-yl]-pyridin-2-yl}-amine

According to the general method for the preparation of aminopyridines from fluoropyridines, 6-(4-fluorophenyl)-5-(2-fluoropyridin-4-yl)-2,3-dihydro-imidazo[2,1-b]thiazole of step C) (0.48 g) was heated to130°C in cyclohexylamine (1.7 mL). After about 3 h at this temperatur in cyclohexylamine only a small amount of starting compound was detected (97% conversion). The dark oily reaction mixture which contained a precipitate was cooled and the precipitate was suction filtered. The precipitate was washed with diethyl ether/MeOH (9:1; 5 mL) and dried. 0.26 g of colorless crystals of high purity (HPLC: 99.6%) were obtained. Upon repeated addition of diethyl ether to the mother liquor a second and third crop of crystals was obtained: 0.1 g and 0.08 g of 99% and 98% purity.
Total yield: 0.44 g (74%).
The combined precipitates were recrystallized from isopropanol.

### E) Cyclohexyl-{4-[6-(4-fluorophenyl)-1-oxo-2,3-dihydro-1H-1λ⁴-imidazo[2,1-b]thiazol-5-yl]-pyridin-2-yl}-amine

The conversion of the sulfanyl to the sulfinyl compound was carried out according to the general preparation method 1: Yield 0.34 g (82%); purity (HPLC): 93%.

### Example 32

### N-{4-[6-(4-Fluorophenyl)-1-oxo-2,3-dihydro-1H-1λ⁴-imidazo[2,1-b]thiazol-5-yl]-pyridin-2-yl}-acetamide

The title compound was obtained from the compound of example 31, step A), according to the general preparation method 1.
Yield: 0.04 g (22%), purity: (HPLC): 99.4%
¹H-NMR: ppm(DMSO): 10.66 (s; 1H), 8.41 (d; 1H, J=4.8Hz); 8.15 (s; 1H); 7,52-7,48 (m; 2H); 7.21-1.17 (m; 3H); 4.77-4,70(m; 1H) 4.40-4.35 (m;1H), 4.08-4.01 (m; 1H), 3.75-3.7 (m;1H), 2.01 (s;CH₃),
¹³C-NMR: ppm(DMSO): 170.2, 154.6, 153.5, 149.6, 144.3, 139.3, 131.3, 130.5, 130.0, 129.9, 127.8, 126.6, 119.5, 116.4, 116.2, 113.5, 56.1, 43.9, 24.6.

### Example 33

### N-{4-[2-(4-Fluorophenyl)-8-oxo-5,6,7,8-tetrahydro-8λ⁴-imidazo[2,1-b][1,3]thiazin-3-yl]-pyridin-2-yl}-acetamide

The title compound was obtained from N-{4-[5-(4-fluorophenyl)-3-(3-hydroxy-propyl)-2-thioxo-2,3-dihydro-1H-imidazol-4-yl]-pyridin-2-yl}-acetamide (prepared as described in WO 02/066458) in analogous manner as decribed in examples 31 and 32.
Yield: 78%; purity (HPLC): 95.7%
¹H-NMR: ppm(DMSO): 10.7 (s;1H); 8.46 (d; 1H, J=4.8Hz); 8.1 (s;1H), 7.45-7.41 (m; 2H); 7.17-7.12 (m; 3H); 3.98-3.86 (m; 2H) 3.35-3.32 (m; 2H); 2.65-2.61 (m,1H); 2.24-2.21 (m; 1H); 2.01 (s; CH₃)
¹³C-NMR: ppm(DMSO): 170.3, 164.4, 156.8, 153.6, 151.3, 149.8, 144.3, 139.4, 138.2, 130.2, 129.3, 129.2, 128.6, 121.1, 116.3, 116.1, 114.9, 45.2, 44.8, 24.6, 14.2.

### Example 34

### {4-[6-(4-Fluorophenyl)-2,3-dihydro-imidazo[2,1-b]thiazol-5-yl]-pyridin-2-yl}-phenyl-amine

6-(4-Fluorophenyl)-5-(2-fluoro-pyridin-4-yl)-2,3-dihydro-imidazo[2,1-b]thiazole (0.25 g) was stirred at 110°C in dry freshly distilled aniline (1 mL). After 2 h no starting compound could be detected. A crystalline precipitate formed in the reaction mixture which was filtered off and washed with little ether and then ether/methanol 9:1 (1 mL). The substance was dried under vacuum over P₂O₅; 0.250 g (90.4%) of the title compound was obtained. M.p. 298.7 °C and 99.6% purity (HPLC, RT=6.1 min.).
GC-MS: 70 eV EI-MS: m/z (rel. Int.) 388(100); 327.
The title compound can be converted to the sulfinyl or sulfonyl compound using the general method 1 or 2.

### Example 35

### (+/-)-(1,2-Dimethyl-propyl)-{4-[6-(4-fluorophenyl)-1-oxo-2,3-dihydro-imidazo[2,1-b]thiazol-5-yl]-pyridin-2-yl}-amine and (+/-)-(1,2-Dimethyl-propyl)-{4-[6-(4-fluoro-phenyl)-1,1-dioxo-2,3-dihydro-imidazo[2,1-b]thiazol-5-yl]-pyridin-2-yl}-amine

### A) (+/-)-(1,2-Dimethyl-propyl)-{4-[6-(4-fluoro-phenyl)-2,3-dihydro-imidazo[2,1-b]thiazol-5-yl]-pyridin-2-yl}-amine

6-(4-Fluorophenyl)-5-(2-fluoro-pyridin-4-yl)-2,3-dihydro-imidazo[2,1-b]thiazole (0.95 g) was heated in 1,2-dimethylpropylamine (7.25 mL) to 80 °C. The proportion of educt decreased to about 30% within 7 days. Upon cooling a fine precipitate formed (0.75 g) which contained 75% product. Recrystallization from ethyl acetate and ether did not improve the quality. The mixture of educt and product was again heated in 1,2-dimethylpropylamine (5 mL) to 80 °C and maintained at this temperaure for additional 4 days. An HPLC showed 88% conversion. The crystalline mass of 0.48 g (41% yield) obtained upon cooling had an HPLC purity of > 99%.

According to the same method 6-(4-fluorophenyl)-5-(2-fluoro-pyridin-4-yl)-2,3-dihydro-imidazo[2,1-b]thiazole (0.64 g) was reacted with the pure enantiomer (S)-(+)-3-methyl-2-butylamine (2.53 mL) (96% conversion after 3 days). 0.6 g (90%) of (S)-(1,2-dimethyl-propyl)-{4-[6-(4-fluoro-phenyl)- 2,3-dihydro-imidazo[2,1-b]thiazol-5-yl]-pyridin-2-yl}-amine were obtained.

### B) (+/-)-(1,2-Dimethyl-propyl)-{4-[6-(4-fluorophenyl)-1-oxo-2,3-dihydro-imidazo[2,1-b]thiazol-5-yl]-pyridin-2-yl}-amine

In a 1: 1 mixture of THF (2.3 mL) and acetone (3.7 mL) (+/-)-(1,2-dimethyl-propyl)-{4-[6-(4-fluoro-phenyl)-2,3-dihydro-imidazo[2,1-b]thiazol-5-yl]-pyridin-2-yl}-amine (0.28 g, 95.6%) was dissolved to give a clear solution (if required by warming). An aqueous sodium metaperiodate solution (0.26 g in 2.8 mL) was added and the reaction mixture was heated to 60 °C. After 4 h an HPLC showed about 70% conversion. After an additional 4 h at 60°C the conversion was 80%, after further 16 h at room temperature conversion was 93%. The formed precipitate was filtered off and discarded (NalO₃). The filtrate was evaporated to dryness and the residue was extracted with n-hexane at elevated temperaure. Upon cooling the product (0.29 g) crystallized in 90% purity from the hexane extracts. The product was purified using column chromatography (SiO₂ / ethylester-MeOH 9:1). A total of 150 mg (52%) of the product having a purity of 98.8% was obtained.

### Example 36

### {4-[5-(4-Fluorophenyl)-2-methanesulfinyl-3-methyl-3H-imidazol-4-yl]-pyridin-2-yl}-phenyl-amine and {4-[5-(4-fluorophenyl)-2-methanesulfonyl-3-methyl-3H-imidazol-4-yl]-pyridin-2-yl}-phenyl-amine

### A) {4-[5-(4-Fluoro-phenyl)-2-methanesulfanyl-3-methyl-3H-imidazol-4-yl]-pyridin-2-yl}-phenyl-amine

Sodium hydride (0.55 g 55% ig in paraffin oil, 12.5 mmol) was suspended in diethylene glycol dimethylether. After addition of aniline (0.876 g, 9.4 mmol) the reaction was heated to 70°C until hydrogen development decreased. 2-Fluoro-4-[5-(4-fluoro-)-3-methyl-2-methylsulfanyl-3H-imidazol-4-yl]-pyridine (1.02 g; 3.2 mmol) was added with stirring and the reaction maintained at 70°C for 4 hours. Thereafter, no starting material could be detected. The reaction mixture was taken up in dichloromethane (70 mL). The organic phase was decanted from the insoluble solid, washed with water (2 x 50 mL) and concentrated under vacuum (45°C, 60 mbar). The oily residue (with diglyme and aniline) was taken up in ethyl acetate, washed with water (2 x 50 mL), dried over anhydrous Na₂SO₄, filtered, concentrated under vacuum (45°C, 60 mbar) and dried under high vacuum. The semi-solid residue was then treated with 3 aliquots of warm n-hexane (30 mL) to remove the adhering paraffin oil. The remaining crystalline solid was taken up in little diisopropyl ether, filtered and washed with diisopropyl ether. The substance was dried under vacuum over anhydrous CaCl₂ giving 0.20 g (16.3%) of the compound.
¹H-NMR: ppm (CDCl₃): 8.269-8.256 (1H,arom.); 7.438-7.447 (2H,arom.); 7.260-7.220 (2H,arom.); 7.158-7.137 (2H,arom.); 7.060-6.953 (4H, arom.); 6.716-6.714 (1NH,arom.); 6.673-6.658 (1H, arom.); 3.470-3.467 (3H,NCH₃); 2.685-2.683 (3H,SCH₃);
IR: (λ (cm⁻¹): 3280, 3050, 1616, 1599, 1548, 1527, 1497, 1479, 1440, 1398, 1371, 1309, 1296, 1270, 1221, 1159, 976, 842, 815, 757, 739, 694, 579.
GC-MS (70 eV EI-MS) m/z (rel. Int. [%] =) 390(100)357(37), 316(24), 194(11), 341(8).

Reaction of 2-fluoro-4-[5-(4-fluorophenyl)-3-methyl-2-methylsulfanyl-3H-imidazol-4-yl]-pyridine (1.9 g; 6 mmol) in excess aniline (5.476 g; 59 mmol) at 135 °C without activation by sodium hydride gave after 20 h the product in 60% yield (1.4 g) having a purity of 93%. The crude product obtained after pre-extraction of aniline with n-hexane was recrystallized from diisopropyl ether.

### B) {4-[5-(4-Fluorophenyl)-2-methanesulfinyl-3-methyl-3H-imidazol-4-yl]-pyridin-2-yl}-phenyl-amine

{4-[5-(4-Fluorophenyl)-2-methanesulfanyl-3-methyl-3H-imidazol-4-yl]-pyridin-2-yl}-phenyl-amine (0.195 g, 0.5 mmol) was dissolved in a 1:1 (V/V) mixture of THF / acetone. To the clear mixture 2.25 mL deionised water was added. Sodium metaperiodate (0.18 g, 0.8 mmol) is dissolved in water (1.5 mL) and added to the reaction mixture which was then heated under reflux. After 7 h the same amount of oxidation agent was added and the reaction mixture was heated under reflux for 14 h. THF and acetone were evaporated under vacuum and the aqueous residue was extracted with ethyl acetate. The ethyl acetate phase was washed with water, dried over Na₂SO₄, filtered and concentrated. The residue was purified using column chromatography (SiO₂/ethyl acetate). In fraction 1 the sulfone obtained as a byproduct was isolated : Yield 0.04 g (19%), HPLC-purity (RT= 7.6) 97.5%.
In the following main fraction the sulfoxide is obtained: 0.1 g (49.2%), HPLC-purity (RT= 6.86) 99.3%.
C₂₂H₁₉FN₄OS=406.49;
¹H-NMR: ppm(DMSO): 9,19 (1H,arom.); 8,327-8,314 (1H,arom.); 7,65-7,63 (2H,arom.); 7,509-7,474 (2H,arom.); 7,277-7,160 (4H, arom); 6,927-6,891 (1H,arom.); 6,829-6,794 (1H,arom;1H,NH); 3,747(3H,SOCH₃); 3,161 (3H,NCH₃);
IR: (λ (cm⁻¹): 3301, 3053, 2923, 1731, 1609, 1594, 1545, 1525, 1458, 1441, 1373, 1343, 1270, 1220, 1156, 1029, 996, 975, 957, 839, 813, 753, 693, 654, 591, 580 GC-MS (70 eV EI-MS) m/z (rel. Int. [%] =) m/z=406(20), 390 (100), 357 (30), 316 (22), 194 (13), 341 (9), 158 (7).

### {4-[5-(4-Fluorophenyl)-2-methanesulfonyl-3-methyl-3H-imidazol-4-yl]-pyridin-2-yl}-phenyl-amine

C₂₂H₁₉FN₄O₂S=422.48
GC-MS (EI, 70eV): m/z (rel Int. %) 421(100), 343(14), 287(11), 194(6).

### Example 37

### Cyclohexyl-{4-[6-(4-fluorophenyl)-1-oxo-2,3-dihydro-1H-1λ⁴-imidazo[2,1-b]thiazol-5-yl]-pyridin-2-yl}-amine and Cyclohexyl-{4-[6-(4-fluorophenyl)-1,1-dioxo-2,3-dihydro-1H-1λ⁴-imidazo[2,1-b]thiazol-5-yl]-pyridin-2-yl}-amine

### Cyclohexyl-{4-[6-(4-fluoro-phenyl)-2,3-dihydro-imidazo[2,1-b]thiazol-5-yl]-pyridin-2-yl}-amine

According to the general method for preparing aminopyridines from fluoropyridines, 6-(4-fluorophenyl)-5-(2-fluoropyridin-4-yl)-2,3-dihydro-imidazo[2,1-b]thiazole (0.48 g) was heated in cyclohexylamine (1.7 mL) to 130°C. After about 3 h at this temperature an HPLC showed only a small amount of the educt (97% conversion). The dark oily reaction mixture in which a precipitate had formed was cooled and the precipitate was filtered off. The precipitate was washed with diethyl ether/MeOH (9:1; 5 mL) and dried to give 0.26 g of the title product as colorless crystals of high purity (HPLC: 99.6%).
Upon repeated addition of diethyl ether a second and third crop of crystals was obtained: 0.1g and 0.08 g of 99% and 98% purity.
Total yield: 0.44 g (74%).
The product was recrystallized from isopropanol.

### Cyclohexyl-{4-[6-(4-fluorophenyl)-1-oxo-2,3-dihydro-1H-1λ⁴-imidazo[2,1-b]thiazol-5-yl]-pyridin-2-yl}-amine

The conversion of the above sulfanyl compound to the sulfinyl compound was carried out according to the general method 1 a. Yield: 0.34 g (82%); purity 93% (HPLC).

### Cyclohexyl-{4-[6-(4-fluorophenyl)-1,1-dioxo-2,3-dihydro-1H-1λ⁴-imidazo[2,1-b]thiazol-5-yl]-pyridin-2-yl}-amine

The sulfonyl compound was obtained according to general method 2.

### Example 38

### (1,2-Dimethylpropyl)-{4-[5-(4-fluorophenyl)-2-methanesulfinyl-3-(2-methoxyethyl)-3H-imidazol-4-yl]-pyridin-2-yl}-amine

### A) (1,2-Dimethylpropyl)-{4-[5-(4-fluorophenyl)-2-methanesulfanyl-3-(2-methoxyethyl)-3H-imidazol-4-yl]-pyridin-2-yl}-amine

2-Fluoro-4-[5-(4-fluorophenyl)-3-(2-methoxyethyl)-2-methylsulfanyl-3H-imidazol-4-yl]-pyridine (0.9 g) was dissolved in (+/-)-3-methyl-2-butylamine (3.17 mL) and heated in an closed inox reactor for 3 days at 130 °C internal temperature. After this time a sample from the reaction mixture showed by HPLC a 93% conversion of starting material. The reactor was cooled in a cooling bath and allowed to cool down to 30-40°C. When this temperature was reached, the reactor was vented. The fine crystalline mass which appeared after treatment with n-hexane/diethyl ether was collected on a Buchner funnel. The crystals were washed with diisopropyl ether. A highly pure pruduct (> 99%) with a total yield of 0.99 g (92.4%) was obtained. GC-MS /70 eV EI-MS: m/z (rel. Int. [%]): 428 (25), 385 (100), 358 (9);

### B) (1,2-Dimethylpropyl)-{4-[5-(4-fluorophenyl)-2-methanesulfinyl-3-(2-methoxyethyl)-3H-imidazol-4-yl]-pyridin-2-yl}-amine

The sulfoxide was prepared from the above starting material (0.29 g) according to the general oxidation methode 1 a with NalO₄ (0.26 g) in a solvent mixture of acetone, water, and THF (3.7 mL, 2.8 mL, 2.3 mL) at 60 °C, reaction time 16 h.
After filtration from salts the volatiles of the filtrate were evaporated and the residue was recrystallized from n-hexane. The material obtained by suction filtration (0.29 g) was 90% pure.
Purification was achieved by column chromatography: SiO₂/ethyl acetate elution gave fraction 1 (starting material), elution with ethyl acetate/MeOH 9:1 gave fraction 2: title compound (0.15 g, 52 %) of high purity (99.8% HPLC).

### Example 39

### (1,2-Dimethylpropyl)-{4-[5-(4-fluorophenyl)-2-methanesulfinyl-3-methyl-3H-imidazol-4-yl]-pyridin-2-yl}-amine

### A) (1,2-Dimethylpropyl)-{4-[5-(4-fluorophenyl)-3-methyl-2-methylsulfanyl-3H-imidazol-4-yl]-pyridin-2-yl}-amine

2-Fluoro-4-[5-(4.fluorophenyl)-2-methanesulfanyl-3-methyl-3H-imidazol-4-yl]-pyridine (0.8 g) was dissolved in (+/-)-3-methyl-2-butylamine (3.0 mL) and heated in an sealed glass tube for 3 days at 75°C. After 5 days a sample from the reaction mixture showed by HPLC a 53% conversion of starting material. The reaction mixture was transferred into an inox reactor and brought to 120°C. After 24 hours the reactor was cooled in a cooling bath, a sample was drawn for HPLC analysis: 86% conversion was detected. The reactor was brought to 150 °C for further 24 h and then a sample showed 93% conversion. The reactor was allowed to cool down to 30-40°C and was vented. From the reaction mixture the volatile components were evaporated and the residue was treated with a diethyl ether/ethyl acetate mixture. The crystals were collected: 0.71 g (57.7%) highly pure material (>99%, RT = 5.9 min.). A second fraction was obtained from the mother liquor after removal of solvent and recrystallization from diisopropyl ether/n-hexane: 0.2 g (17%, 97.3% purity).
GC-MS /70 eV EI-MS: m/z (rel. Int. [%]): 384 (20), 369 (4), 341 (100), 326 (5), 313(8), 293 (8), 170 (4);

### B) (1,2-Dimethylpropyl)-{4-[5-(4-fluorophenyl)-2-methanesulfinyl-3-methyl-3H-imidazol-4-yl]-pyridin-2-yl}-amine

The sulfoxide was prepared from the above starting material (0.23 g) according to the general oxidation method 1 a with NalO₄ (0.24 g) in a solvent mixture of acetone, water, and THF (3.7 mL, 2.8 mL, 2.3 mL) at 60 °C, reaction time 24 h (89% conversion, RT = 4.7 min).
After filtration from salts on a Buchner funnel, the salts were rinsed on the funnel with aliquots of ethyl acetate. Collected washings and filtrate were combined and transferred to a rotavapor. The volatiles were evaporated and the residue was recrystallized from diethyl ether. The material obtained by suction filtrationwas 90% pure.
Purification was achieved by column chromatography: SiO₂ / a first ethyl acetate elution gave fraction 1 with starting material, consecutive elution with ethyl acetate/ MeOH 9:1 gave fraction 2: title compound (0.13 g, 54%) of high purity (> 99% HPLC area).

### Example 40

### {4-[5-(4-Fluorophenyl)-2-methanesulfinyl-3-(2-methoxyethyl)-3H-imidazol-4-yl]-pyridin-2-yl}-phenyl-amine

### A) {4-[5-(4-Fluorophenyl)-3-(2-methoxyethyl)-2-methylsulfanyl-3H-imidazol-4-yl]-pyridin-2-yl}-phenyl-amine

170 mg (3.9 mmol) NaH (55-65%) and 280 mg (3 mmol) aniline in 3 mL of diglyme were heated to 70°C while stirring. When gas evolution ceased a solution of 361 mg (1 mmol) 2-fluoro-4-[5-(4-fluorophenyl)-3-(2-methoxyethyl)-2-methylsulfanyl-3H-imidazol-4-yl]-pyridine in diglyme was added and further stirred. The reaction was monitored by TLC. The reaction mixture was cooled to room temperature and 40 mL of dichloromethane were added. The organic phase was washed six times with 25 mL of water, dried over Na₂SO₄ and evaporated. The oily residue was purified by column chromatography (SiO₂- EtOAc/Hexane=3/7). Yield: 275 mg (64.6%), mp: 107.6°C.
¹H-NMR: ppm (CDCl₃) 2.708 (s, 3H, S-CH₃); 3.204 (s, 3H, O-CH₃); 3.509 (t, 6.0 Hz, 2H, ethyl); 4.037 (t, 6.0 Hz, 2H, ethyl); 6.758-7.467 (m, 11H, arom, pyridine); 8.226 (d, 6.0 Hz, 1H, C6-H, Py);

### B) {4-[5-(4-Fluorophenyl)-2-methanesulfinyl-3-(2-methoxyethyl)-3H-imidazol-4-yl]-pyridin-2-yl}-phenyl-amine

260 mg (0.6 mmol) of {4-[5-(4-fluorophenyl)-3-(2-methoxyethyl)-2-methylsulfanyl-3H-imidazol-4-yl]-pyridin-2-yl}-phenyl-amine was dissolved in a mixture of 1.6 g tetrahydrofuran and 2.8 g acetone and 2.7 g water was added with stirring. A solution of 241 mg (1.1 mmol) of NalO₄ in 3.8 g of water was added and the mixture was stirred vigorously at 65°C for 26 h. The mixture was cooled to room temperature and 20 mL of dichloromethane were added. The phases were separated and the organic layer was dried over Na₂SO₄ and evaporated. The oily residue was purified by column chromatography (SiO₂ - dichloromethane/EtOH=97/3). Yield: 47 mg (17,4%), mp: 83-85 °C (sintering).
¹H-NMR: ppm (CDCl₃) 3.210 and 3.222 (2s, 3H and 3H, SO-CH₃ and O-CH₃); 3.478-3.660 (m, 2H, ethyl); 4.231-4.282 (m, 1H, ethyl); 4.465-4.462 (m, 1H, ethyl); 6.740 (d, 5.2 Hz, 1H, C5, pyridine), 6.818 (s, 1H, C3, pyridine), 6.908-7.092 (m, 4H, arom); 7.182-7.303 (m, 3H, arom); 7.447-7.482 (m, 2H, arom); 8.295 (d, 5.2 Hz, 1H, C6-H, Py).

### Example 41

### {4-[5-(4-Fluorophenyl)-2-methanesulfinyl-3-(2-methoxyethyl)-3H-imidazol-4-yl]-pyridin-2-yl}-(4-methoxyphenyl)-amine

### A) {4-[5-(4-Fluorophenyl)-3-(2-methoxyethyl)-2-methylsulfanyl-3H-imidazol-4-yl]-pyridin-2-yl}-(4-methoxyphenyl)-amine

To p-anisidine (2.32 g, 18.4 mmol) dissolved in diglyme (25 mL) NaH (1.07 g, 55% in white oil) was added at room temperature in portions and the temperature of the heating bath was set to 80 °C. As evolution of gas (H₂) ceased at this temperature (1h) 2-fluoro-4-[5-(4-fluorophenyl)-2-methanesulfanyl-3-(2-methoxyethyl)-3H-imidazol-4-yl]-pyridine (2.22 g, 6 mmol) was introduced. The reaction was monitored by HPLC. After 2 h at 80 °C the reaction was complete. The reaction mixture was poured into ice water (150 mL) and extracted twice with ethyl acetate. The ethyl acetate extracts were combined, washed with demineralised water, dried (Na₂SO₄ sicc.) and evaporated to leave a semi- solid residue. The white oil was extracted thoroughly with n-hexane and the crystals were collected.
The title compound was obtained by column chromatography: SiO₂/diisopropyl ether: ethanol 95:5 gave two fractions:
Fraction 1: 0.5 g (94% pure by HPLC)
Fraction2: 0.7 g (87% pure by HPLC)
Overall 1.2 g (42%)
GC-MS / 70 eV EI-MS: m/z (rel. Int. [%]): 464

### B) {4-[5-(4-Fluorophenyl)-2-methanesulfinyl-3-(2-methoxyethyl)-3H-imidazol-4-yl]-pyridin-2-yl}-(4-methoxyphenyl)-amine and {4-[5-(4-Fluorophenyl)-2-methanesulfinyl-3-(2-methoxyethyl)-3H-imidazol-4-yl]-pyridin-2-yl}-(4-methoxyphenyl)-amine hydrochloride

The sulfoxide was prepared from the above starting material (1.2 g, 88%, 2.3 mmol) according to the general oxidation method 1a with aqueous NalO₄ sol. (0.89 g, 4.1 mmol in 10 mL H₂O) in THF (21 mL) at reflux, reaction time 24 h (RT = 6.04 min.). After evaporation of the organic solvent the aqueous residue (suspension) was diluted with some water and the title compound was extracted with several aliquots of ethyl acetate. The combined extracts were washed with water, dried (Na₂SO₄ sicc.), filtered and evaporated to leave a viscous oil (1.2 g).
Purification was achieved by column chromatography: SiO₂/diisopropyl ether - ethanol 95:5
fraction 1: 0.2 g (38% yield) of purity 99% by HPLC
fraction 2: oily material recrystallized from diisopropyl ether: 0.05 g (4%).
This latter material was taken up in ethyl acetate and with HCl/ ethanol a hydrochloride salt was precipitated.
Recrystallization of the precipitate from diisopropyl ether gave 0.02 g of highly pure hydrochloride salt (>99%).
fraction 3: from the main fraction of poor quality, additional 0.12 g of the hydrochloride salt could be obtained in a high purity (>99% HPLC) by the same procedure.

### Example 42

### 2-Fluoro-4-[5-(4-fluorophenyl)-3-(2-methoxyethyl)-2-methylsulfanyl-imidazol-4-yl]-pyridine and 2-fluoro-4-[5-(4-fluorophenyl)-3-(2-methoxy-ethyl)-2-methylsulfinyl-imidazol-4-yl]-pyridine

### A) 2-(4-Fluorophenyl)-1-(2-fluoropyridin-4-yl)-ethanone

To Olah's reagent (58,0 g; 70% HF in pyridin) in a 100 mL FEP bottle (Perfluoro ethylen propylen) cooled to -10°C 1-(2-aminopyridin-4-yl)-2-(4-fluorophenyl)-ethanone (16.11 g) was added and stirred. Over a period of 1 h NaNO₂ (7.87 g) was added in small portions (15 ca. 0.5 g each). After each aliquot the reaction bottle was closed loosely. As the inner temperature was kept at about 0 °C, only little nitrous gases (with foaming) evolved. The reaction mixture turned yellow. After the last addition the stirring was continued for 1 h at 0°C and for 1 further hour at room temperature. Water (200mL) was poured into the mixture while stirring. CH₂Cl₂ (125 mL) was added and the layers were separated in a separatory funnel. The aqueous layer was extracted with CH₂Cl₂ (three times 75 mL). The CH₂Cl₂ fractions were combined and washed with CaCO₃-sol. (100 mL, 5 %) and water (100mL), dried over Na₂SO_{4 sicc.}, and the solvent was stripped off *in vacuo.* The residual obtained was treated with hot n-hexane several times. The title compound crystallized from the n-hexane extracts in the cold (refrigerator at 3-5°C) with 95% purity.

As an alternative the raw material obtained can be purified by column chromatography (cc): SiO₂/EtOAc-n-hexane=3:7.
Yield: 10,8 g (66,3%) Purity: 99% HPLC (after cc).
¹H NMR: (DMSO-d6) δ (ppm) = 4.503 (s, 2H, CH₂); 7.144-7.196 (m, 2H, C3/5, 4-F-Ph); 7.299-7.335 (m, 2H, C2/6, 4-F-Ph); 7.729 (s, 1H, C3-H, Pyr); 7.851-7.872 (m, 1H, C5-H, Pyr); 8.481-8.494 (m, 1H, C6-H, Pyr);

### B) 1-(4-Fluorophenyl)-2-(2-fluoropyridin-4-yl)-ethane-1,2-dione-1-oxime

2-(4-Fluorophenyl)-1-(2-fluoropyridin-4-yl)-ethanone (2.56 g; 11.0 mmol) was dissolved in glacial acetic acid (26 mL). An aqueous saturated solution of sodium nitrite (2.25 g, 32.0 mmol) was added dropwise at room temperature in such a rate that formation of nitrous gases was avoided. The slightely yellow solution was stirred overnight. Water (80 mL) was added and the suspension formed was stirred for at least 1 hour. The crystals were collected on a Büchner funnel by suction filtration and washed on the funnel with some aliquots of demineralized water and finally with hexane. Yield 2.8 g (98%), mp: 166 °C.
1H NMR: δ (ppm) (DMSO-d6) 7.291-7.344 (m, 2H, C3/5, 4-F-Ph); 7.537-7.588 (m, 3H, C2/6, 4-F-Ph;C3-H, Pyr);7.677 -7.697 (m, 1H, C5-H, Pyr); 8.402-8.417 (m, 1H, C6-H, Pyr); 13.105 (s, 1H, OH);

### C) (2-Fluoro-4-[5-(4-fluorophenyl)-3-(2-methoxyethyl)-1-oxy-3H-imidazol-4-yl]-pyridine

To a suspension of 1-(4-fluorophenyl)-2-(2-fluoropyridin-4-yl)-ethane-1,2-dione-1-oxim (10.48 g; 0.04 mol) in ethanol (180 mL) 1,3,5-tris-(2-methoxy-ethyl)-[1,3,5]triazinan (5.12 g, 0.0196 mol) dissolved in ethanol (20 mL) was added at once. The mixture was brought to reflux temperature (90°C) and refluxing conditions were held for 20 h. Work up started by stripping off the ethanol on a rotavapor and the residual solid was taken in diethyl ether (100 mL). After 12 h storage of the etheral suspension, the crystals were filtered from the mother liquor on a Buchner filter and dried at 45°C at 10 mbar.
C₁₇H₁₅F₂N₃O₂ (Mr 331,32): Yield 9,44 g (91%), purity (HPLC area method) > 99%

### (D) 4-(4-Fluorophenyl)-5-(2-fluoropyridin-4-yl)-1-(2-methoxyethyl)-1,3-dihydro-imidazole-2-thione

2-Fluoro-4-[5-(4-fluorophenyl)-3-(2-methoxyethyl)-1-oxy-imidazol-4-yl]-pyridine (9.44 g, 0.0285 mol), prepared according to step B, was suspended in CH₂Cl₂ (120 mL). While keeping the suspension at 0°C in a ice cooling bath a solution of 2,2,4,4-tetramethyl-cyclobutane-1,3-dithion (3.1 g, 0.018 mol) in CH₂Cl₂ (30 mL) was added dropwise. After about 15 min the clear solution was allowed to warm up to room remperature and stirring was continued for 2 hours. After this time the product, which crystallized from the solution, was filtered from the mother liquor. A second crop was obtained when the volume of the mother liquor was reduced to half of the initial volume and the reduced volume substituted by the same volume of diisopropyl ether. First and second crop were combined and dried.

C₁₇H₁₅F₂N₃OS (Mr 347,39): Yield 8,49 g (88%) Purity (HPLC area method) 95%; mp: 209°C,
GC-MS: 9,39 min m/z (%) 347 (22), 289 (100), 230 (5);
IR (λ[cm-1]): 3069, 2972, 2900, 1608, 1493, 1407, 1395, 122 (4-FPh), 1119 (=S), 881,844,815

### E) 2-Fluoro-4-[5-(4-fluorophenyl)-3-(2-methoxy-ethyl)-2-methylsulfanyl-imidazol-4-yl]-pyridine

A suspension of 4-(4-fluorophenyl)-5-(2-fluoropyridin-4-yl)-1-(2-methoxyethyl)-1,3-dihydro-imidazole-2-thion (8.42 g, 23.5 mmol) in methanol (150 ml) was prepared. After adding potassium carbonate (2.68 g, 19 mmol) a solution of methyl iodide (4.47 g; 32 mmol) in MeOH (30 mL) was added dropwise. The mixture was stirred for 20 h at room temperature. The volume of the suspension was reduced under vacuum to dryness. The residual solids were partitioned between a mixture of ethyl acetate and water (250 mL, 3:2). The aqueous layer was reextracted with ethyl acetate and removed. The combined organic layers were washed with water, dried over Na₂SO₄ sicc. and evaporated. The raw material was recrystallized from diisopropyl ether. This material is suitable to be used for fluorine-amine-replacement reaction. C₁₈H₁₇F₂N₃OS (MG 361.42) Yield 7.9 g (89%), 99% purity (HPLC area % ; RT=7.6 min.).
GC-MS: 7.81 min m/z (%) 361 (100),330 (19), 303 (21), 270 (81), 121 (14)
IR (λ[cm-1]): 3061, 2925, 2890, 1609, 1542, 1506, 1390, 1222 (4-FPh), 1121, 880, 851,828.

### F) 2-Fluoro-4-[5-(4-fluorophenyl)-2-methanesulfinyl-3-(2-methoxyethyl)-3H-imidazol-4-yl]-pyridine

A solution of 2-fluoro-4-[5-(4-fluorophenyl)-3-(2-methoxyethyl)-2-methylsulfanyl-3H-imidazol-4-yl]-pyridine (0.903 g; 0.0025 mol) in glacial acetic acid (10 mL) and solution of hydrogen peroxide 30% (0.3 g; 0.0026 mol) in glacial acetic acid (1 mL) were mixed and stirred at room temperature (according to general procedure 1); reaction time: 84 h (3,5 d). After completion (full conversion) the mixture was poured onto ice water (10 mL). The solution was made alkaline (pH 8-9) with ammonia (32%). The precipitated product was taken into ethyl acetate (40 mL), while the alkaline aqueous layer was extracted five times with ethyl acetate (15 mL). The combined ethyl acetate solution was washed with water (10 mL), dried over Na₂SO₄ sicc. and evaporated.
Purification was achieved by cc: Al₂O₃/Eluent: n-hexane= 2:1
After removal of the solvent the product crystallized from n-hexane.
C₁₈H₁₇F₂N₃O₂S (*Mr 377,42*): Yield: 704 mg (79%); Purity: from HPLC area: (RT=6,2 min.) 99%.
GC-MS: (RT=8.46 min.); m/z (rel. int. [%]) 377 (1), 361 (100), 330 (16), 303 (15), 270 (65), 121 (10).
IR (λ[cm⁻¹]): 2972, 2931, 2895, 1610, 1508, 1397, 1220 (4-FPh), 1051 (SO), 880, 840
¹H-NMR (DMSO-d6): δ (ppm) 3.1 (s, 1-H, SOCH₃); 3.13 (s, 3-H, OCH₃); 3.23-3.50 ( m, 2-H, N-CH₂-CH₂-OCH₃ near water resonance 3.3 ppm); 4.23-4.39 (m, 2-H, N-CH₂-CH₂-OCH₃); 7.13-7.17 (m, 2-H, C3-H pyr., C5-H pyr.); 7.37-7.41 (m, 4-H, C3/5-H 4-FPh +C2/6-H 4-FPh); 8.40 (d, 1-H, J= 5.2 Hz, C6-HPyr.)

### G) 2-Fluoro-4-[5-(4-fluorophenyl)-3-methyl-2-methanesulfinyl-3H-imidazol-4-yl]-pyridine

The title compound was prepared analogously from 2-fluoro-4-[5-(4-fluorophenyl)-3-methyl-2-methylsulfanyl-3H-imidazol-4-yl]-pyridine (0.952 g, 0.003 mol) in glacial acetic acid (10 mL) and a solution of hydrogen peroxide 30% (0.36 g; 0.0032 mol) in glacial acetic acid (1 mL) (according to general procedure 1): reaction time 168 h (7 d).
The white crystalline material is suitable to be used for fluorine-amine-replacement reaction without further purification.
C₁₆H₁₃F₂N₃OS (Mr 333,36): Yield: 870 mg (90%); Purity: from HPLC area: 5,66 min 84,35%.
GC-MS: 7.99 min m/z (%) 333 (27), 317 (100), 284 (82), 244 (47);
IR (λ[cm-1]):1617, 1541, 1509, 1407, 1221 (4-FPh), 1194, 1160, 1053, 950, 881, 847, 657.

Additional sulfanyl compounds of the present invention are given in table 6. The corresponding sulfinyl and sulfonyl compounds can be obtained according to the methods described herein.

**Table 6**

| | | | | |
|---|---|---|---|---|
| 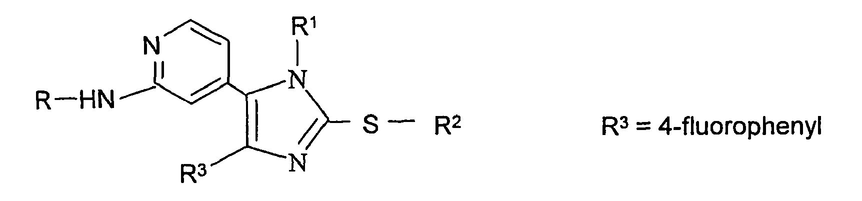 | | | | |

| **ex. no.** | **R¹** | **R²** | **R** | **m.p. (°C)** |
|---|---|---|---|---|
| 43 | CH₃ | CH₃ | iprop | |
| 44 | CH₃ | CH₃ | 3-methylbutyl | 160 |
| 45 | CH₃ | CH₃ | (±)-3-methyl-2-butyl | 163 |
| 46 | CH₃ | CH₃ | (R)-1-phenylethyl | |
| 47 | CH₃ | CH₃ | (S)-1-phenylethyl | |
| 48 | CH₃ | CH₃ | Ph-NH-CO | |
| 49 | CH₃ | CH₃ | Ph-N(CH₃)-CO | |
| 50 | CH₃ | 2,3-dihydroxypropyl | H | |
| 51 | CH₃ | 2,3-dihydroxypropyl | CH₃CO | |
| 52 | CH₃ | CH2CON(CH₂CH₂OH)₂ | H | |
| 53 | CH₃ | CH₂CON(CH₂CH₂OH)₂ | CH₃CO | |
| 54 | CH₃ | CH₂CH₂-COOEt | CH₃CO | |
| 55 | CH₃ | (R,S)-1-phenylethyl | H | |
| 56 | CH₃ | CH₂CH₂-CO₂H | H | |
| 57 | CH₃ | 4-CH₃CO-benzyl | CH₃CO | |
| 58 | Et | CH₃ | H | 174 |
| 59 | Et | CH₃ | (R,S)-1-phenylethyl | 175 |
| 60 | iprop | CH₃ | H | 193 |
| 61 | iprop | CH₃ | CH₃CO | 207 |
| 62 | iprop | CH₃ | (R,S)-1-phenylethyl | 160 |
| 63 | cprop-CH₂ | CH₃ | H | 154 |
| 64 | cprop-CH₂ | CH₃ | CH₃CO | 198 |
| 65 | cprop-CH₂ | CH₃ | (R,S)-1-phenylethyl | 111 |
| 66 | CH₃OCH₂CH₂ | CH₃ | H | 126 |
| 67 | CH₃OCH₂CH₂ | CH₃ | formyl | |
| 68 | CH₃OCH₂CH₂ | CH₃ | propionyl | 131 |
| 69 | CH₃OCH₂CH₂ | CH₃ | pivaloyl | |
| 70 | CH₃OCH₂CH₂ | CH₃ | isobutyryl | |
| 71 | CH₃OCH₂CH₂ | CH₃ | valerianyl | |
| 72 | CH₃OCH₂CH₂ | CH₃ | 3-methylbutyryl | |
| 73 | CH₃OCH₂CH₂ | CH₃ | 2-methylbutyryl | |
| 74 | CH₃OCH₂CH₂ | CH₃ | 4-methylpentanoyl | |
| 75 | CH₃OCH₂CH₂ | CH₃ | CF₃CO | 142 |
| 76 | CH₃OCH₂CH₂ | CH₃ | acryloyl | 138 |
| 77 | CH₃OCH₂CH₂ | CH₃ | benzoyl | |
| 78 | CH₃OCH₂CH₂ | CH₃ | 4-chlorobenzoyl | |
| 79 | CH₃OCH₂CH₂ | CH₃ | 4-methoxybenzoyl | |
| 80 | CH₃OCH₂CH₂ | CH₃ | PhCH₂CO | |
| 81 | CH₃OCH₂CH₂ | CH₃ | 3-Ph-propionyl | |
| 82 | CH₃OCH₂CH₂ | CH₃ | cinnamoyl | |
| 83 | CH₃OCH₂CH₂ | CH₃ | 4-phenylbutyryl | |
| 84 | CH₃OCH₂CH₂ | CH₃ | 2-furyl-CO | |
| 85 | CH₃OCH₂CH₂ | CH₃ | 2-thienyl-CO | 152 |
| 86 | CH₃OCH₂CH₂ | CH₃ | Ph-N(CH₃)-CO | |
| 87 | CH₃OCH₂CH₂ | CH₃ | (±)-3-methyl-2-butyl | |
| 88 | CH₃OCH₂CH₂ | CH₃ | (R)-1-phenylethyl | 103 |
| 89 | CH₃OCH₂CH₂ | CH₃ | (S,R)-1-phenylethyl | |
| 90 | CH₃OCH₂CH₂ | CH₃ | (R,R)-1-phenylethyl | |
| 91 | CH₃OCH₂CH₂ | CH₃ | (S)-1-phenylethyl | |
| 92 | CH₃OCH₂CH₂ | CH₃ | (S,S)-1-phenylethyl | |
| 93 | CH₃OCH₂CH₂ | CH₃ | 4-t-butylphenyl | |
| 94 | CH₃OCH₂CH₂ | CH₃ | 4-CH₃SO₂-phenyl | |
| 95 | CH₃OCH₂CH₂ | CH₃ | 3-CH₃SO₂-phenyl | |
| 96 | CH₃OCH₂CH₂ | 4-CH₃SO-benzyl | H | 119 |
| 97 | CH₃OCH₂CH₂ | 4-CH₃SO-benzyl | CH₃CO | 130 |
| 98 | CH₃OCH₂CH₂ | 4-CH₃SO-benzyl | (R,S)-1-phenylethyl | 105 |
| 99 | CH₃OCH₂CH₂ | morph-COCH₂CH₂ | CH₃CO | |
| 100 | CH₃OCH₂CH₂CH₂ | CH₃ | H | 141 |
| 101 | CH₃OCH₂CH₂CH₂ | CH₃ | acryloyl | 112 |
| 102 | CH₃OCH₂CH₂CH₂ | 4-CH₃SO-benzyl | CH₃CO | 149 |
| 103 | CH₃OCH₂CH₂CH₂ | morph-COCH₂CH₂ | CH₃CO | |
| 104 | HOCH₂CH₂ | CH₃ | H | |
| 105 | HOCH₂CH₂ | CH₃ | CH₃CO | 189 |
| 106 | HOCH₂CH₂ | morph-COCH₂CH₂ | H | |
| 107 | HOCH₂CH₂ | 4-CH₃SO-benzyl | CH₃CO | 160 |
| 108 | HOCH₂CH₂ | morph-COCH₂CH₂ | CH₃CO | |
| 109 | HOCH₂CH₂ | morph-COCH₂CH₂ | CH₃CO | |
| 110 | 2,2-dimethoxyethyl | CH₃ | H | 168 |
| 111 | 2,2-dimethoxyethyl | CH₃ | CH₃CO | 147 |
| 112 | HOCH₂CH₂CH₂ | CH₃ | H | 147 |
| 113 | HOCH₂CH₂CH₂ | CH₃ | CH₃CO | 189 |
| 114 | HOCH₂CH₂CH₂ | morph-COCH₂CH₂CH₂ | CH₃CO | |
| 115 | (R,S)-CH₃CH(OH)-CH₂ | CH₃ | H | 102 |
| 116 | (R,S)-CH₃CH(OH)-CH₂ | CH₃ | CH₃CO | 101 |
| 117 | (R,S)-CH₃CH(OH)-CH_{z} | 4-CH₃SO-benzyl | H | 123 |
| 118 | (R,S)-CH₃CH(OH)-CH₂ | 4-CH₃SO-benzyl | CH₃CO | 114 |
| 119 | 3-oxopropyl | CH₃ | CH₃CO | |
| 120 | 2-allyloxyethyl | CH₃ | CH₃CO | 128 |
| 121 | 2-allyloxyethyl | CH₃ | H | 112 |
| 122 | 2-allyloxyethyl | CH₃ | (R,S)-1-phenylethyl | 96 |
| 123 | 2-allyloxyethyl | 4-CH₃SO-benzyl | CH₃CO | 68 |
| 124 | 2-allyloxyethyl | 4-CH₃SO-benzyl | H | 93 |
| 125 | 2-allyloxyethyl | 4-CH₃SO-benzyl | (R,S)-1-phenylethyl | 80 |
| 126 | 2-propargyloxyethyl | CH₃ | (R,S)-1-phenylethyl | 81 |
| 127 | 2-propargyloxyethyl | CH₃ | CH₃CO | 163 |
| 128 | 2-propargyloxyethyl | CH₃ | H | |
| 129 | 2-propargyloxyethyl | 4-CH₃SO-benzyl | CH₃CO | 136 |
| 130 | 2-propargyloxyethyl | 4-CH₃SO-benzyl | H | 64 |
| 131 | HOCH₂CH₂OCH₂CH₂ | CH₃ | CH₃CO | 105 |
| 132 | HOCH₂CH₂OCH₂CH₂ | CH₃ | H | 150 |
| 133 | HOCH₂CH₂OCH₂CH₂ | 4-CH₃SO-benzyl | H | 114 |
| 134 | HOCH₂CH₂OCH₂CH₂ | 4-CH₃SO-benzyl | CH₃CO | 114 |
| 135 | 6-OH-hex-1-yl | CH₃ | CH₃CO | 138 |
| 136 | 2,2-dimethyldioxolan-4-yl-CH₂ | CH₃ | CH₃CO | 142 |
| 137 | EtOOCCH₂CH₂ | CH₃ | CH₃CO | |
| 138 | CH₃OOCCH₂CH₂ | CH₃ | CH₃CO | |
| 139 | HOOCCH₂CH₂ | CH₃ | CH₃CO | |
| 140 | HOOCCH₂CH₂ | CH₃ | H | |
| 141 | HOOCCH₂CH₂ | CH₃ | chex | |
| 142 | NCCH₂CH₂ | CH₃ | CH₃CO | |
| 143 | ClCH₂CH₂ | CH₃ | CH₃CO | |
| 144 | CH₃SO₃CH₂CH₂ | CH₃ | CH₃CO | |
| 145 | CH₃SCH₂CH₂ | CH₃ | CH₃CO | 161 |
| 146 | CH₃SCH₂CH₂ | 4-CH₃SO-benzyl | CH₃CO | 119 |
| 147 | CH₃SCH₂CH₂ | CH₃ | H | 180 |
| 148 | CH₃SCH₂CH₂ | 4-CH₃SO-benzyl | H | 71 |
| 149 | CH₃SCH₂CH₂ | CH₃ | (R,S)-1-phenylethyl | 128 |
| 150 | CH₃CONHCH₂CH₂ | 4-CH₃SO-benzyl | CH₃CO | 246 |
| 151 | CH₃CONHCH₂CH₂ | CH₃ | CH₃CO | 207 |
| 152 | CH₃CONHCH₂CH₂ | CH₃ | H | 155 |
| 153 | CH₃CONHCH₂CH₂ | 4-CH₃SO-benzyl | H | 246 |
| 154 | (CH₃)₂NCH₂CH₂ | CH₃ | CH₃CO | 159 |
| 155 | (CH₃)₂NCH₂CH₂ | CH₃ | H | 147 |
| 156 | [(CH₃)₃NCH₂CH₂]⁺ | CH₃ | CH₃CO | |
| 157 | piperidin-4-yl | CH₃ | H | 147 |
| 158 | N-COOEt-piperidin-4-yl | CH₃ | CH₃CO | 190 |
| 159 | N-COOEt-piperidin-4-yl | CH₃ | H | 201 |
| 160 | 2-N-morpholinylethyl | CH₃ | (R,S)-1-phenylethyl | 113 |
| 161 | 2-N-morpholinylethyl | CH₃ | iprop | 82 |
| 162 | 2-N-morpholinylethyl | CH₃ | CH₃CO | 218 |
| 163 | 2-N-morpholinylethyl | CH₃ | H | 105 |
| 164 | 2-N-piperidinylethyl | CH₃ | CH₃CO | 174 |
| 165 | 2-N-piperidinylethyl | CH₃ | (R,S)-1-phenylethyl | 111 |
| 166 | 2-N-piperidinylethyl | CH₃ | H | 176 |
| 167 | 2-N-piperidinylethyl | 4-CH₃SO-benzyl | CH₃CO | 121 |
| 168 | 2-N-piperidinylethyl | 4-CH₃SO-benzyl | H | 105 |
| 169 | 2,2,6,6,tetramethylpiperidin-4-yl | CH₃ | CH₃CO | 232 |
| 170 | 2,2,6,6,tetramethylpiperidin-4-yl | CH₃ | Et | 141 |
| 171 | 2,2,6,6,tetramethylpiperidin-4-yl | CH₃ | cpropCO | |
| 172 | 2,2,6,6,tetramethylpiperidin-4-yl | 4-CH₃SO-benzyl | CH₃CO | 219 |
| 173 | 3-N-morpholinylpropyl | CH₃ | CH₃CO | 215 |
| 174 | 3-N-morpholinylpropyl | 4-CH₃SO-benzyl | CH₃CO | 120 |
| 175 | R¹ + R² = ethylene | | H | |
| 176 | R¹ + R² = propylene | | H | |
| 177 | R¹ + R² = ethylene | | CH₃CO | 252 |
| 178 | R¹ + R² = ethylene | | phenyl | 299 |
| 179 | R¹ + R² = ethylene | | chex | |
| 180 | R¹ + R² = ethylene | | (±)-3-methyl-2-butyl | |
| 181 | R¹ + R² = propylene | | CH₃CO | 227 |
| 182 | CH₃ | CH₃ | H | 169 |
| 183 | CH₃ | CH₃ | (R,S)-1-phenyl-ethyl | 111 |
| 184 | CH₃ | CH₃ | cprop CH₃ | |
| 185 | CH₃ | 4-CH₃SO-benzyl | CH₃CO | 191 |
| 186 | nprop | CH₃ | H | 145 |
| 187 | nprop | CH₃ | Et | 110 |
| 188 | nprop | CH₃ | CH₃CO | 164 |
| 189 | 2,2,6,6,tetramethylpiperidin-4-yl | CH₃ | H | 232 |

Abbreviations used in tables 6 and 7:
- nprop: n-propyl
- iprop: isopropyl
- Et: ethyl
- cprop: cyclopropyl
- Ph: phenyl
- morph: morpholinyl (attached via its N atom)
- chex: cyclohexyl

Additional sulfinyl and sulfonyl compounds of the present invention are given in table 7:

**Table 7**

| | | | | |
|---|---|---|---|---|
| 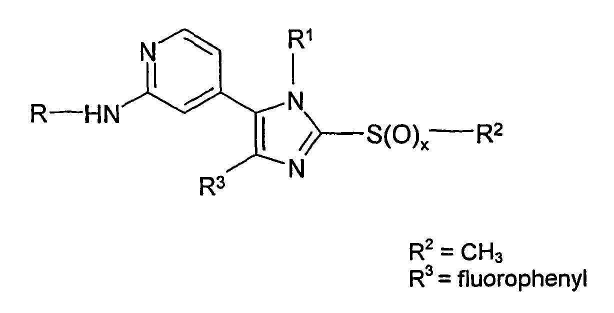 | | | | |

| **ex. no.** | **R¹** | **R** | **X** | **m.p. (°C)** |
|---|---|---|---|---|
| 190 | CH₃ | H | 1 | |
| 191 | CH₃ | cprop | 1 | |
| 192 | CH₃ | (±)-3-methyl-2-butyl | 1 | |
| 193 | CH₃ | phenyl-NHCO | 1 | |
| 194 | CH₃ | phenyl-N(CH₃)CO | 1 | |
| 195 | nprop | CH₃CO | 1 | 229 |
| 196 | nprop | CH₃CO | 2 | 211 |
| 197 | CH₃OCH₂CH₂ | H | 1 | |
| 198 | CH₃OCH₂CH₂ | (±)-3-methyl-2-butyl | 1 | |
| 199 | CH₃OCH₂CH₂ | (R,S)-1-phenylethyl | 1 | |
| 200 | CH₃OCH₂CH₂ | (R)-1-phenylethyl | 1 | |
| 201 | CH₃OCH₂CH₂ | (S)-1-phenylethyl | 1 | |
| 202 | CH₃OCH₂CH₂ | (S,R)-1-phenylethyl | 1 | |
| 203 | CH₃OCH₂CH₂ | (R,R)-1-phenylethyl | 1 | |
| 204 | CH₃OCH₂CH₂ | (S,S)-1-phenylethyl | 1 | |
| 205 | CH₃OCH₂CH₂ | chex | 2 | 132 |
| 206 | CH₃OCH₂CH₂ | 4-CH₃SO₂phenyl | 1 | 164 |
| 207 | CH₃OCH₂CH₂ | 4-CH₃SO₂phenyl | 2 | 155 |
| 208 | CH₃OCH₂CH₂ | 4-CH₃SO-phenyl | 1 | 199 |
| 209 | HOCH₃CH₂CH₂ | CH₃CO₂ | 1 | |
| 210 | R¹+R² = propylene | H | 1 | |
| 211 | R¹+R² = propylene | H | 1 | |
| 212 | CH₃OCH₂CH₂ | phenyl | 1 | |

### Example 213

### N-{4-[5-(4-Fluorophenyl)-3-(2-methoxyethyl)-2-methylsulfanyl-3H-imidazol-4-yl]-pyridin-2-yl}-benzamide

4-[5-(4-Fluorophenyl)-3-(2-methoxyethyl)-2-methylsulfanyl-3H-imidazol-4-yl]-pyri-din-2-ylamine (0.15 g, 0.4185 mmol) andt triethylamine (42.35 mg, 0.4185 mmol) were dissolved in abs. THF. The solution was cooled in an ice bath. Benzoic acid anhydride (94.67 mg, 0.4185 mg) was added and the reaction mixture was stirred in an ice bath for 2 h. Then the solvent was evaporated and the crude product was purified by column chromatography (silica gel 60, dichloromethane : ethanol = 95 : 5). Yield: 0.025 g (12.91 %)
¹H-NMR (CDCl₃):
δ (ppm) 2.74 (s, 3H, -S-CH₃), 3.26 (s, 3H, -O-CH₃), 3.54 (t, 2H, J = 5.9, -O-CH₂), 4.14 (t, 2H, J = 5.8, N-CH₂), 6.89-7.03 (m, 3H, 4-fluoro-Ph, Pyr), 7.41-7.61 (m, 5H, phenyl of benzamide), 7.91-7.96 (m, 2H, 4-F-Ph), 8.33 (d, 1H, J = 4.32 Hz, pyr), 8.48 (s, 1H. Pyr), 8.64 (s, 1H, NH, exchangeable)
IR (ATR) cm⁻¹ 1677, 1546, 1521, 1504, 1412, 1287, 1220, 1119, 839, 708

### Example 214

### 4-Chloro-N-{4-[5-(4-fluorophenyl)-3-(2-methoxyethyl)-2-methylsulfanyl-4H-imidazol-4-yl]-pyridin-2-yl}-benzamide

4-[5-(4-Fluorophenyl)-3-(2-methoxyethyl)-2-methylsulfanyl-3H-imidazol-4-yl]-pyridin-2-ylamine (132.3 mg, 0.3692 mmol) and triethylamine (37.36 mg, 0.3692 mmol) were dissolved in 15 ml abs. THF. The solution was cooled in an ice bath. p-Chlorobenzoic acid chloride (64.62 mg, 0.3692 mmol) was added and the reaction mixture was stirred in an ice bath for 2 h . Then the solvent was evaporated and the crude product was purified by column chromatography (RP-18, acetonitrile : water = 6 : 4)
Yield: 8,9 mg (4,83 %)
¹H-NMR (CDCl₃):
δ 2.74 (s, 3H, -S-CH₃), 3.26 (s. 3H, -O-CH₃), 3.52 (m, 2H, -O-CH₂), 4.13 (t, 2H, J = 5.98 Hz, -N-CH₂), 6.88-7.03 (m, 3H, 4-Fluor-Ph, pyr), 7.40-7.53 (m, 4H, phenyl of benzamide), 7.88 (dd, 2H, J₁ = 6.71, J₂ = 1.94, 4-Fluor-Ph), 8.33 (d, 1H, J= 5.1 Hz, pyr), 8.45 (s, 1H, Pyr), 8.61 (s, 1H, -NH- exchangeable)
IR (ATR) cm⁻¹:1545, 1523, 1504, 1487, 1412, 1289, 1220, 1118, 1096, 839

### Example 215

### N-{4-[5-(4-Fluorphenyl)-3-(2-methoxyethyl)-2-methylsulfanyl-3H-imidazol-4-yl]-pyridin-2-yl}-4-methoxybenzamide

4-[5-(4-Fluorphenyl)-3-(2-methoxyethyl)-2-methylsulfanyl-3H-imidazol-4-yl]-pyri-din-2-ylamin (1.67 g, 4.66 mmol) and triethylamine (0.47 g, 4.66 mmol) were dissolved in 120 ml abs. THF. The solution was cooled in an ice bath. 4-Methoxybenzoic acid chloride (0.71 g, 4.66 mmol) was added and the reaction mixture was stirred in an ice bath for 2 h . Then the solvent was evaporated and the crude product was purified by column chromatography (RP-18, acetonitrile : water = 6 : 4)
Yield: 0.1 g ( 4.36 %)
¹H-NMR (CDCl₃):
δ 2.82 (s, 3H, SCH₃), 3.26 (s, 3H, aliphat. OCH₃), 3.58 (t, 2H, J = 5.5 Hz, OCH₂), 3.90 (s, 3H, aromat. OCH₃), 4.23 (t, 2H, J = 5.6 Hz, NCH₂), 6.93-7.10 (m, 6H, 4-F-Ph, phenyl of benzamide), 7.41-7.48 (m, 2H, 4-F-Ph), 8.04-8.22 (m, pyr), 8.58 (s, 1H, - NH)
IR (ATR) cm⁻¹: 3316, 3182, 2930, 1606, 1541, 1507, 1432, 1219, 1117, 839

### Example 216

### N-{4-[5-(4-Fluorophenyl)-3-(2-methoxyethyl)-2-methylsulfanyl-3H-imidazol-4-yl]-pyridin-2-yl}-2-phenylacetamide

4-[5-(4-Fluorophenyl)-3-(2-methoxyethyl)-2-methylsulfanyl-3H-imidazol-4-yl]-pyridin-2-ylamine (0.53 g, 1.48 mmol) was dissolved in 40 ml dichloromethane and cooled in an ice bath. Phenylacetylchloride (0.23 g, 1.48 mmol) was added and the reaction mixture was stirred in an ice bath for 2 h . Then the solvent was evaporated and the crude product was purified by column chromatography (RP-18, acetonitrile : water = 6:4)
Yield: 0,09 g (12,76 %)
¹H-NMR (CDCl₃):
δ ppm 2.72 (s, 3H, -SCH₃), 3.22 (s, 3H, OCH₃), 3.45-3.51 (m, 2H, OCH₂), 3.78 (s, 2H, NCH₂), 4.08 (d, 2H, J = 5.9 Hz, CH₂ phenylacetamide), 6.86-6.95 (m, 3H, 4-F-Ph, pyr), 7.33-7.42 (m, 7H, 4-F-Ph, phenyl of phenylacetamide), 8.20-8.29 (m, 1 H, pyr), 8.29 (s, 1H, Pyr), 9.23 (s, 1H, -NH, exchangeable)
IR (ATR) cm⁻¹: 2929, 1545, 1503, 1411, 1261, 1219 1156, 1117, 838, 695

### Example 217

### N-{4-[5-(4-Fluorophenyl)-3-(2-methoxyethyl)-2-methylsulfanyl-3H-imidazol-4-yl]-pyridin-2-yl}-2,2-dimethylpropionamide

According to the general preparation method 5, the title compound was obtained from 4-[5-(4-fluorophenyl)-3-(2-methoxyethyl)-2-methylsulfanyl-3H-imidazol-4-yl]-pyridin-2-ylamine (1 g, 2.774 mmol), 50 mL abs. pyridine and pivaloylchloride (0.34 g, 2.8 mmol) after a reaction time of 3 h. The product was purified by column chromatography by means of MPLC (silica gel 60, n-hexane:acetone = 1:1).
Yield: 0,23 g (18,74 %)
¹H-NMR (CDCl₃): δ 1.34 (s, 9H, (CH₃)₃), 2,71 (s, 3H, SCH₃), 3.22 (s, 3H, OCH₃), 3.48 (t, 2H, J = 5.9 Hz, OCH₂), 4,11 (t, 2H, J = 6.0 Hz, NCH₂), 6.87-6.96 (m, 3H, 4-F-Ph), 7.37-7.45 (m, 2H, 4-F-Ph), 8.21-8.33 (m, 2H, pyr, NH (exchangeable)
¹³C-NMR (CDCl₃): δ 16.38 (SCH₃), 27.33 (C³/C⁴/C⁵-propionamide), 39.76 (C²-propionamide), 44.18 (NCH₂), 58.75 (OCH₃), 70.57 (OCH₂), 114.98 (Aryl-C), 115.01 (d, ²J (C,F) = 21.3 Hz, C³/C⁵ 4-F-Ph), 121,61 (pyr), 127.31 (C⁵-imidazole), 128.84 (d, ³J (C,F) = 7.9 Hz, C²/C⁶ 4-F-Ph), 129.81 (d, 4J = 3.3 Hz C¹ 4-F-Ph), 138.86 (aryl-C), 141.45 (aryl-C), 144.80 (aryl-C), 147.89 (aryl-C), 152.05 (aryl-C), 161.85 (d, ¹J (C,F) = 244.3 Hz, C⁴ 4-F-Ph), 177.02 (CO)
IR (ATR) cm⁻¹: 2964, 2931, 1545, 1516, 1501, 1410, 1220, 1155, 1119, 838

### Example 218

### N-{4-[5-(4-Fluoorphenyl)-3-(2-methoxyethyl)-2-methylsulfanyl-3H-imidazol-4-yl]-pyridin-2-yl}-isobutyramide

According to the general preparation method 5, the title compound was obtained from 4-[5-(4-fluorophenyl)-3-(2-methoxyethyl)-2-methylsulfanyl-3H-imidazol-4-yl]-pyridin-2-ylamine (0.75 g, 2.0801 mmol), 40 mL abs. pyridine and isobutyrylchloride (0.2238 g, 2.1 mmol). The product was purified by column chromatography (1x silica gel 60, DCM:EA = 7:3) and 2x MPLC (RP 18, acetonitrile:water = 6:4).
Yield: 0,09 g (10,1 %)
¹H-NMR (CDCl₃): δ 1.26 (d, J = 5.2 Hz, C³H₃/C⁴H₃ isobuturamide), 2.54-2.71 (m, 4H, SCH₃, C²H isobutyramide), 3.22 (s, 3H, OCH₃), 3.48 (t, 2H, J = 5.9 Hz, OCH₂), 4.1 (t, 2H, J = 6.0 Hz, NCH₂), 6.86 (m, 3H, 4-F-Ph, pyr), 7.37 (m, 2H, 4-F-Ph, 8.25-8.41 (m, 3H, pyr, NH)
¹³C-NMR (CDCl₃): δ 16.37 (SCH₃), 19.25 (C³/C⁴ isobutyramide, 36.67 (C² isobutyramide), 44.25 (NCH₂), 58.78 (OCH₂), 70.57 (OCH₂), 114.98 (aryl-C), 115.06 (d, ²J (C,F) = 21.3 Hz, C³/C⁵ 4-F-Ph), 115.47 (aryl-C), 121,54 (pyr), 127.25 (C⁵-imidazole), 128.92 (d, ³J (C,F) = 7.9 Hz, C²/C⁶ 4-F-Ph), 129.69 (d, 4J = 3.3 Hz C¹ 4-F-Ph), 139.00 (aryl-C), 141.67 (aryl-C), 145.00 (aryl-C), 147.49 (aryl-C), 151.82 (aryl-C), 161.90 (d, ¹J (C,F) = 244.5 Hz, C⁴ 4-F-Ph), 175.61 (CO)
IR (ATR) cm⁻¹ : 1546, 1519, 1503, 1411, 1219, 1188, 1156, 1118, 838, 815

### Example 219

### Pentanoic acid-{4-[5-(4-fluorophenyl)-3-(2-methoxy-ethyl)-2-methylsulfanyl-3H-imidazol-4-yl]-pyridin-2-yl}-amide

According to the general preparation method 5, the title compound was obtained from 4-[5-(4-fluorophenyl)-3-(2-methoxyethyl)-2-methylsulfanyl-3H-imidazol-4-yl]-pyridin-2-ylamine (1 g, 2.774 mmol), 50 mL abs. pyridine and pivaloylchlorid (0,34 g, 2,8 mmol) after a reaction time of 5 min. The crude product was purified by column chromatography (silica gel 60, DCM:EA = 7:3).
Yield: 0,45 g (36,45 %)
¹H-NMR (CDCl₃): δ 0.94 (t, 3H, J = 7.2 Hz, C⁵-pentanoic acid amide), 1.34-1.46 (m, 2H, C⁴-pentanoic acid amide) 1.67-1.75 (m, 2H, C³-pentanoic acid amide), 2.42 (t, 2H, J = 7.2 Hz, C²-pentanoic acid amide), 2.72 (s, 3H, SCH₃), 3.23 (s, 3H, OCH₃), 3.50 (t, 2H, J = 5.8 Hz, OCH₂), 4.10 (t, 2H, J = 6.0 Hz, NCH₂), 6.87-6.97 (m, 3H, 4-F-Ph, pyr), 7.37 (m, 2H, 4-F-Ph), 8.27 (dd, 2H, J₁ =5.2 Hz ,J₂ = 0.66 Hz, pyr), 8.31 (s, 1H, NH, exchangeable)
¹³C-NMR (CDCl₃): δ 13.66 (C⁵-Pentansäureamid), 16.33 (SCH₃), 22.21 (C⁴-pentanoic acid amide), 27.24 (C³- pentanoic acid amide), 37.28 (C²- pentanoic acid amide), 44.19 (NCH₂), 58.77 (OCH₃), 70.56 (OCH₂), 115.01 (d, ²J (C,F) = 21.3 Hz, C³/C⁵ 4-F-Ph), 115.06 (aryl), 121.53 (aryl), 127.32 (C⁵-imidazole), 128.86 (d, ³J (C,F) = 7.9 Hz, C²/C⁶ 4-F-Ph), 129.35 (d, 4J = 3.3 Hz C¹ 4-F-Ph), 138.91 (aryl), 141.40 (aryl), 144.84 (aryl), 147.97 (aryl), 152,01 (aryl), 159,40 (d, ¹J (C,F) = 244.3 Hz, C⁴ 4-F-Ph), 171.82 (CO)
IR (ATR) cm⁻¹: 1668, 1543,1502, 1416, 1405, 1360, 1225, 1214, 1121, 848

### Example 220

### N-{4-[5-(4-Fluorophenyl)-3-(2-methoxyethyl)-2-methylsulfanyl-3H-imidazol-4-yl]-pyridin-2-yl}-3-methylbutyramide

According to the general preparation method 5, the title compound was obtained from 4-[5-(4-fluorophenyl)-3-(2-methoxyethyl)-2-methylsulfanyl-3H-imidazol-4-yl]-pyridin-2-ylamine (1 g, 2.774 mmol), 50 mL abs. pyridine and isovalerylchloride (0.34 g, 2.8 mmol) erhalten. After purification by column chromatography (silica gel 60, DCM:EA = 7:3) the product was obtained as a viscous tan mass which was recrystallized from DCM/n-hexane to give a fine white powder.
Yield: 0,35g (28,35 %)
¹H-NMR (CDCl₃): δ 1.02 (d, 6H, J = 6.4 Hz, C⁴H₃/C⁵H₃ methylbutyramide), 2.21-2.30 (m, 3H, C²H₂/C³H methylbutyramide), 2.72 (s, 3H, SCH₃), 3.23 (s, 3H, OCH₃), 3.51 (t, 2H, J = 5.9 Hz, OCH₂), 4.10 (t, 2H, J = 6.0 Hz, NCH₂), 6.87-6.97 (m, 3 H, 4-F-Ph, pyr), 7.38-7.45 (m, 2H, 4-F-Ph), 8.25-8.32 (m, 3H, pyr, NH)
¹³C-NMR (CDCl₃): δ 16.33 (SCH₃), 22.34 (C⁴H₃/C⁵H₃ methylbutyramide), 25.99 (C³H methylbutyramide), 44.23 (C²H₂ methylbutyramide), 46.82 (NCH₂), 58.77 (OCH₃), 70.56 (OCH₂), 115.09 (pyr), 115.03 (d, ²J (C,F) = 21.4 Hz, C³/C⁵ 4-F-Ph), 121,48 (aryl), 127.29 (C⁵-imidazole), 128.92 (d, ³J (C,F) = 7.9 Hz, C²/C⁶ 4-F-Ph), 129.80 (d, 4J = 3.2 Hz C¹ 4-F-Ph), 139.00 (aryl), 141.58 (aryl),144.95 (aryl), 147.64 (aryl), 151.95 (aryl), 161.89 (d, ¹J (C,F) = 244.6 Hz, C⁴ 4-F-Ph), 171.35 (CO)
IR (ATR) cm⁻¹: 1663, 1545, 1502, 1451, 1439, 1415, 1295, 1221, 1119, 846

### Example 221

### N-{4-[5-(4-Fluorophenyl)-3-(2-methoxyethyl)-2-methylsulfanyl-3H-imidazol-4-yl]-pyridin-2-yl}-2-methylbutyramide

According to the general preparation method 5, the title compound was obtained from 4-[5-(4-fluorophenyl)-3-(2-methoxyethyl)-2-methylsulfanyl-3H-imidazol-4-yl]-pyridin-2-ylamine (1 g, 2.774 mmol), 50 mL abs. pyridine and 2-methylbuturylchloride (0.34 g, 2.8 mmol) after a reaction time of 3 h. The crude product was purified by column chromatography (silica gel 60, DCM:EA = 7:3).
Yield: 0,57 g (46,17%)
¹H-NMR (CDCl₃): δ 0.96 (t, 3H, J = 7.4 Hz, C⁴H₃ methylbutyramide), 1.24 (d, 3H, J = 6.9 Hz, C⁵H₃ methylbutyramide), 1.46-1.85 (m, 2H, C³H₂ methylbutyramide), 2.29-2.40 (m, 1H, C²H methylbutyramide), 2.71 (s, 3H, SCH₃), 3.22 (s, 3H, OCH₃), 3.49 (t, 2H, J = 6.1 Hz, OCH₂), 4.11 (t, 2H, J = 6.1 Hz, NCH₂), 6.86-6.97 (m, 3H, 4-F-Ph, pyr), 7.36-7.45 (m, 2H, 4-F-Ph), 8.25-8.40 (m, 3H, pyr, NH)
¹³C-NMR (CDCl₃): δ 11.69 (C⁴ methylbutyramide), 16.35 (SCH₃), 17.08 (C⁵ methylbutyramide), 27.11 (C³ methylbutyramide), 44.01 (C² methylbutyramide), 44.18 (NCH₂), 58.74 (OCH₃), 70.57 (OCH₂),114.99 (d, C³/C⁵ 4-F-Ph, ²J (C,F) = 21.3 Hz), 115.09 (aryl), 121,56 (aryl), 127.33 (d, ⁵J(C,F), 0.6 Hz, C5-imidazole), 128.87 (d, ³J (C,F) = 7.9 Hz, C²/C⁶ 4-F-Ph), 129.87 (d, ⁴J(C,F) = 3.2 Hz C¹ 4-F-Ph), 138.91 (aryl), 141.35(aryl), 144.83 (aryl), 148.04 (aryl), 152.03 (aryl), 161.85 (d, ¹J (C,F) = 244.6 Hz, C⁴ 4-F-Ph), 175.22 (CO)
IR (ATR) cm⁻¹: 1668, 1544, 1500, 1452, 1414, 1258, 1219, 1191, 1125, 845

### Example 222

### N-{4-[5-(4-Fluorphenyl)-3-(2-methoxyethyl)-2-methylsulfanyl-3H-imidazol-4-yl]-pyridin-2-yl}-3-phenylpropionamide

According to the general preparation method 5, the title compound was obtained from 4-[5-(4-fluorophenyl)-3-(2-methoxyethyl)-2-methylsulfanyl-3H-imidazol-4-yl]-pyridin-2-ylamine (1 g, 2.774 mmol), 50 mL abs. pyridine and 2-methylbuturylchloride (0.34 g, 2.8 mmol) after a reaction time of 3 h.nach 3 stündiger Reaktion erhalten. The crude product was purified by column chromatography (silica gel 60, DCM:EA = 7:3).
Yield: 0,35 g (25,57 %)
¹H-NMR (CDCl₃): δ 2.71 (s, 3H, SCH₃), 2.79-2.88 (m, 2H, C³H₂ phenylpropionamide), 2.96-3.01 (m, 2H, C²H₂ phenylpropionamide), 3.18 (s, 3H, OCH₃), 3.50 (t, 2H, J = 5.94 Hz, OCH₂), 4.06 (t, 2H, J = 5.82, NCH₂), 6.95-7.29 (m, 8H, 4-F-Ph, Pyr, phenyl), 7.46-7.54 (m, 2H, 4-F-Ph), 8.30-8.36 (m, 2H, pyr), 9.60 (s, 1H, NH, exchangeable)
¹³C-NMR (CDCl₃): δ 15.00 (SCH₃), 30.79 (C³ phenylpropionamide), 38.24 (C²phenylpropionamide), 43.93 (NCH₂),57.78 (OCH₃), 70.16 (OCH₂),114.64 (d, C³/C⁵ 4-F-Ph, ²J (C,F) = 21.5 Hz), 114.99 (aryl), 121,06 (aryl), 125.85 (aryl), 127.86 (aryl), 128.1 (aryl), 128.32 (d, ³J (C,F) = 7.5 Hz, C²/C⁶ 4-F-Ph),130.80 (d, ⁴J(C,F) = 3.1 Hz C¹ 4-F-Ph), 137.56 (aryl), 140.81 (aryl), 141.16 (aryl), 144.23 (aryl), 148.62 (aryl), 152.87 (aryl), 161.51 (d, ¹J (C,F) = 242.3 Hz, C⁴ 4-F-Ph), 171.07 (CO)
IR (ATR) cm⁻¹: 1667, 1548, 1503, 1417, 1431, 1262, 1116, 848, 694, 689

### Example 223

### 4-tert-Butyl-N-{4-[5-(4-fluorophenyl)-3-(2-methoxy-ethyl)-2-methylsulfanyl-3H-imidazol-4-yl]-pyridin-2-yl}-benzamide

According to the general preparation method 5, the title compound was obtained from 4-[5-(4-fluorophenyl)-3-(2-methoxyethyl)-2-methylsulfanyl-3H-imidazol-4-yl]-pyridin-2-ylamine (1 g, 2.774 mmol), 50 mL abs. pyridine and 4-tert-butylbenzoyl-chloride (0.55 ml, 2.8 mmol) after a reaction time of 5 min. The crude product was purified by column chromatography (silica gel 60, DCM:EA = 7:3).
Yield: 0,3 g (20,73 %)
¹H-NMR (CDCl₃): δ 1.36(s, 9H, tert-butyl), 2.73 (s, 3H, SCH₃), 3.25 (s, 3H, OCH₃), 3.53 (t, 2H, J = 5.9 Hz, OCH₂), 4.15 (t, 2H, J = 5.9 Hz, NCH₂), 6.88-6.99 (m, 3H, 4-F-Ph, pyr), 7.41-7.54 (m, 4H, 4-F-Ph), 7.88 (d, 2H, J = 8.32, phenyl), 8.24 (d, 1H, J = 5.14 Hz, pyr), 8.93 (s, 1H, NH, exchangeable)
¹³C-NMR (CDCl₃): δ 16.36 (SCH₃), 31.02 (C²/C³/C⁴ tert-butyl), 34.97 (C¹ tert-butyl), 44.24 (NCH₂), 58.81 (OCH₃), 70.61 (OCH₂), 115.04 (d, ²J (C,F) = 21.3 Hz, C³/C⁵ 4-F-Ph), 115.15 (aryl), 121.67 (aryl), 125.75 (aryl), 127.05 (aryl), 128.88 (d, ³J = 7.9 Hz, C²/C⁶ 4-F-Ph), 130.96 (aryl), 141.45 (aryl), 148.18 (aryl), 152.24 (aryl), 156.07 (aryl), 161.81 (d, ¹J = 249.5 Hz, C⁴ 4-F-Ph), 165.59 (CO)
IR (ATR) cm⁻¹: 1606, 1546, 1524, 1501, 1412, 1287, 1269, 1221, 1120, 839

### Example 224

### N-{4-[5-(4-Fluorophenyl)-3-(2-methoxyethyl)-2-methylsulfanyl-3H-imidazol-4-yl]-pyridin-2-yl}-3-phenylacrylamide

According to the general preparation method 5, the title compound was obtained from 4-[5-(4-fluorophenyl)-3-(2-methoxyethyl)-2-methylsulfanyl-3H-imidazol-4-yl]-pyridin-2-ylamine (1 g, 2.774 mmol), 50 mL abs. pyridine and 2-methylbuturylchloride (0.34 g, 2.8 mmol) after a reaction time of 3 h. The crude product was purified by column chromatography (silica gel 60, DCM : EA = 3 : 2).
Yield: 0,42 g (30,81 %)
¹H-NMR (CDCl₃): δ 2.74 (s, 3H, SCH₃), 3.25 (s, 3H, OCH₃), 3.53 (t, 2H, J = 5.8 Hz, OCH₂), 4.16 (t, 2H, J = 5.8 Hz, NCH₂), 6.64 (d, 1H, J = 15.6 Hz, alkene), 6.88-7.01 (m, 3H, 4-F-Ph, pyr), 7.36-7.53 (m, 7H, 4-F-Ph, phenyl), 7.79 (d, 1H, J = 15.6 Hz, alken), 8.33 (d, 1H, J = 5.1 Hz, pyr), 8.51 (s, 1H, pyr), 9.40 (s, 1H, NH)
IR (ATR) cm⁻¹: 1606, 1503, 1414, 1332, 1220, 1205, 1156, 1117, 842, 685

### Example 225

### N-{4-[5-(4-Fluorphenyl)-3-(2-methoxyethyl)-2-methylsulfanyl-3H-imidazol-4-yl]-pyridin-2-yl}-4-phenylbutyramid

According to the general preparation method 6, the title compound was obtained from 4-phenylbutyric acid (0.46 g, 2.8 mmol), CDI (0.45 g, 2.8 mmol) and aminopyridine (1.0 g, 2.774 mmol) and 4-[5-(4-fluorophenyl)-3-(2-methoxyethyl)-2-methylsulfanyl-3H-imidazol-4-yl]-pyri-din-2-ylamine after a reaction time of 1 h. The crude product was purified by column chromatography.
Yield: 0,21 g (15,0 %)
¹H-NMR (CDCl₃): δ 2.09 (quint, 2H, J = 7,40 Hz, C³H₂ phenylbutyramide), 2.43 (t, 2H, C²H₂ phenylbutyramide), 2.64-2.73 (m, 5H, SCH₃, C⁴H₂ phenylbutyramide), 3.24 (s, OCH₃), 3.51 (t, 2H, J = 5.8 Hz), 4.11 (t, 2H, J = 6.0 Hz, NCH₂), 6.88-7.17 (m, 3H, 4-F-Ph, pyr), 7.18-7.46 (m, 7H, 4-F-Ph, Ph), 7.82 (d, 1H, J = 5.2 Hz, pyr), 8.32 (s, 1H, pyr), 8.77 (s, 1H, NH)
¹³C-NMR (CDCl₃) δ 16.35 (SCH₃), 26.45 (CH₂), 34.93 (CH₂), 36.61 (CH₂), 44.25 (NCH₂), 58.80 (OCH₃), 70.55 (OCH₂), 115.05 (d, ²J (C,F) = 21.3 Hz, C³/C⁵ 4-F-Ph), 115.17 (aryl), 121.54 (aryl), 126.00 (aryl), 127.31 (d, J⁵ (C,F) = 0.6 Hz, C⁵ imidazole), 128.37 (aryl), 128.92 (d, ³J (C,F) = 7.9 Hz, C²/C⁶ 4-F-Ph), 129.78 (d, ⁴J(C,F) = 3.2 Hz, C¹ 4-F-Ph), 138.96 (aryl), 141.03 (aryl), 141.49 (aryl), 144.95 (aryl), 147.74 (aryl), 151.96 (aryl), 161.87 (d, ¹J (C,F) = 244.7 Hz, C⁴ 4-F-Ph), 171.46 (CO)
IR (ATR) cm⁻¹: 1546, 1502, 1433, 1417, 1262, 1204, 1115, 1099, 848, 695

### Example 226

### 4-Methylpentanoic acid{4-[5-(4-fluorophenyl)-3-(2-methoxyethyl)-2-methylsulfanyl-3H-imidazol-4-yl]-pyridin-2-yl}-amide

According to the general preparation method 6, the title compound was obtained from 4-Methylvaleric acid (0.33 g, 2.8 mmol), CDI (0.45 g ,2.8 mmol) and 4-[5-(4-fluorophenyl)-3-(2-methoxyethyl)-2-methylsulfanyl-3H-imidazol-4-yl]-pyri-din2-ylamine after a reaction time of 1 h. The product was purified by column chromatography and recrystallized from dichloromethane/n-hexane.
Yield: 0,34 g (26,84 %)
¹H-NMR (CDCl₃): δ 0.92 (d, 6H, J = 5.8 Hz, C⁵H₃ /C⁶H₃ 4-methylvaleric acid amide), 1.59-1.65 (m, 3H, C⁴H 4- methylvaleric acid amide, C³H 4- methylvaleric acid amide), 2.42 (t, 2H, J = 7.5 Hz, C²H₂ 4- methylvaleric acid amide), 2.72 (s, 3H, SCH₃), 3.23 (s, 3H, OCH₃), 3.50 (t, 2H, J = 5.8 Hz, OCH₂), 4.10 (t, 2H, J = 5.9 Hz, NCH₂), 6.87-6.97 (m, 3H, 4-F-Ph, pyr), 7.38-7.45 (m, 2H, 4-F-Ph), 8.24-8-31 (m, 2H, pyr), 8.67 (s, 1H, NH)
¹³C-NMR (CDCl₃): δ 16.34 (SCH₃), 22.17 (C⁵H₃/C⁶H₃ 4-methylvaleric acid amide), 27.62 (C⁴H 4- methylvaleric acid amide), 33.97 (CH₂), 35.68 (CH₂), 44.24 (NCH₂), 58.78 (OCH₃), 70.54 (OCH₂), 115.03 (d, ²J (C,F) = 21.4 Hz, C³/C⁵ 4-F-Ph), 115.10 (aryl), 121.49 (aryl), 127.30 (aryl), 128.92 (d, ³J (C,F) = 8.0 Hz, C²/C⁶ 4-F-Ph), 129.74 (d, ⁴J(C,F) = 3.1 Hz, C¹ 4-F-Ph), 138.98 (aryl), 141.55 (aryl), 144.97 (aryl), 147.64 (aryl), 151.98 (aryl), 161.88 (d, ¹J (C,F) = 244.5 Hz, C⁴ 4-F-Ph), 172.07 (CO)
IR (ATR) cm⁻¹ : 1668, 1545, 1503, 1455, 1416, 1363, 1260, 1215, 1121, 847

### Example 227

### N-{4-[5-(4-Fluorophenyl)-3-(2-methoxyethyl)-2-methylsulfanyl-3H-imidazol-4-yl]-pyridin-2-yl}-2-methyl-3-phenylpropionamide

According to the general preparation method 6, the title compound was obtained from α-Methylhydrocinnamic acid (0.46 g, 2.8 mmol), CDI (0.45 g ,2.8 mmol) and 4-[5-(4-fluorophenyl)-3-(2-methoxyethyl)-2-methylsulfanyl-3H-imidazol-4-yl]-pyridin-2-ylamine after a reaction time of 1 h. The product was purified by column chromatography.
Yield: 0,13 g (9,3%)
¹H-NMR (CDCl₃): δ 1.26 (d, 3H, J = 3.14 Hz, CH₃ 2-methyl-3-phenylpropionamide), 2.62-2.78 (m, 5H, SCH₃, CH₂ 2-methyl-3-phenylpropionamide), 3.05-3.16 (m, 1H, CH 2-methyl-3-phenylpropionamide), 3.23 (s, 3H, OCH₃), 3.50 (t, 2H, J = 6.0 Hz, OCH₂), 4.10 (t, 2H, J = 5.8 Hz, NCH₂),6.87-6.96 (m, 3H, 4-F-Ph, pyr), 7.19-7-26 (m, 5H, phenyl 2-methyl-3-phenylpropionamide), 7.38-7-45 (m, 4-F-Ph), 8.20-8.29 (m, 3H, pyr, NH)
¹³C-NMR (CDCl₃): δ 16.36 (SCH₃), 17.41 (CH₃ 2-methyl-3-phenylpropionamide), 40.01 (CH₂ 2-methyl-3-phenyl-propionamide), 44.17 (NCH₂), 44.55 (CH 2-methyl-3-phenylpropionamide), 58.77 (OCH₃), 70.57 (OCH₂), 115.01 (d, ²J (C,F) = 21.3 Hz, C³/C⁵ 4-F-Ph), 115.09 (aryl), 121.58 (aryl), 126.41 (aryl), 127.33 (aryl), 128.40 (aryl), 128.80 (aryl),128.94 (aryl), 129.88 (d, ⁴J (C,F) = 3.1 Hz, C²/C⁶ 4-F-Ph), 138.98 (d, ³J(C,F) = 7.8 Hz, C¹ 4-F-Ph), 141.27 (aryl), 144.81 (aryl), 148.13 (aryl), 151.86 (aryl), 161.85 (d, ¹J (C,F) = 244.4 H, C⁴ 4-F-Ph), 174.40 (CO)
IR (ATR) cm⁻¹ : 1605, 1545, 1519, 1502, 1412, 1219, 1156, 1118, 838, 699

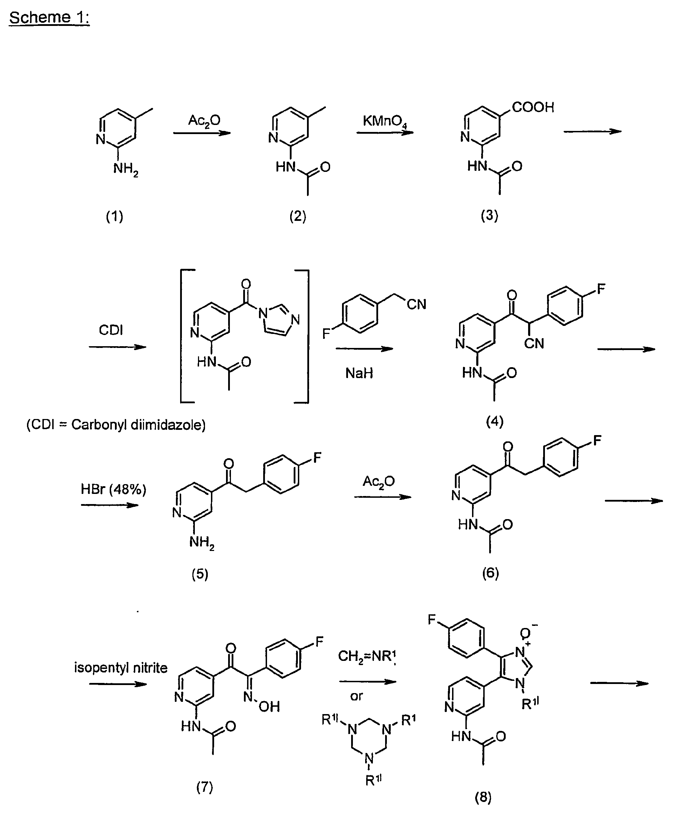

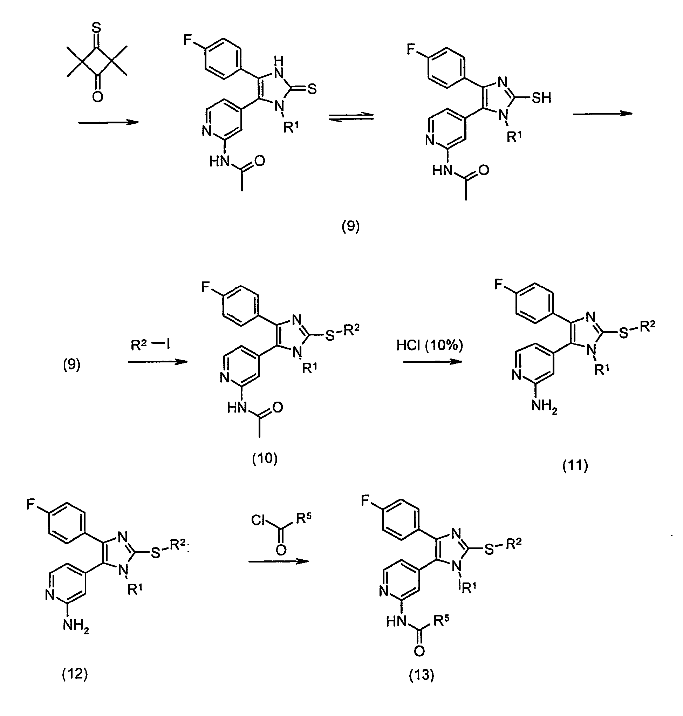

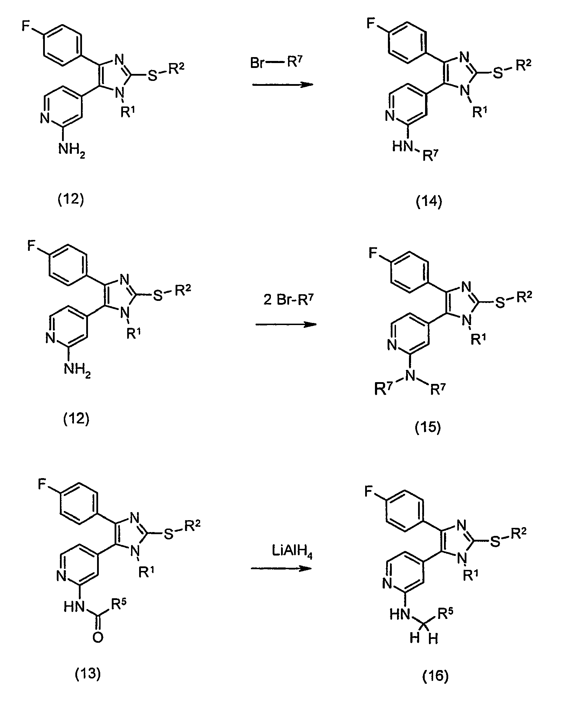

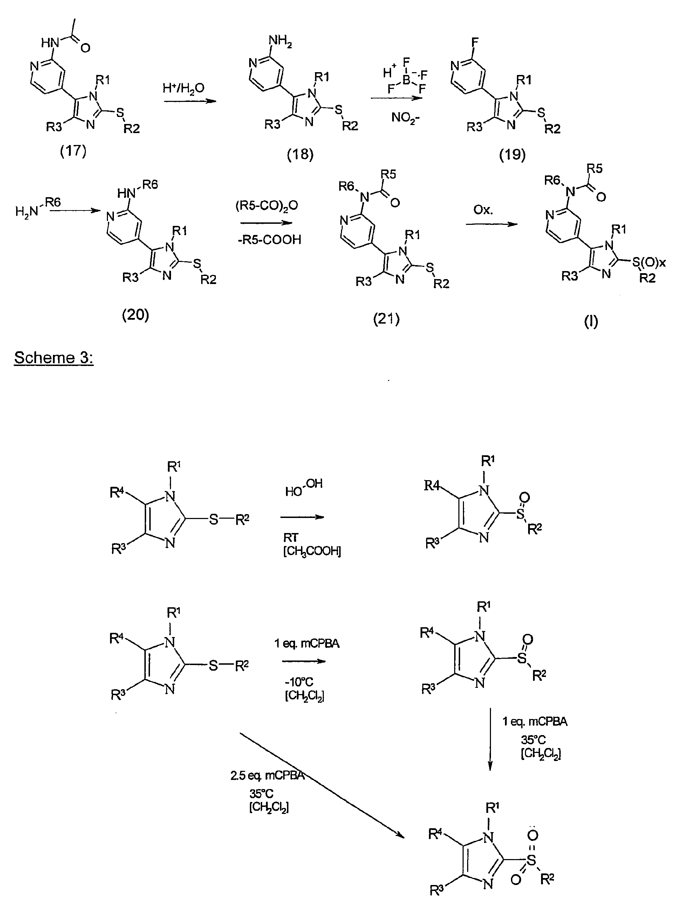

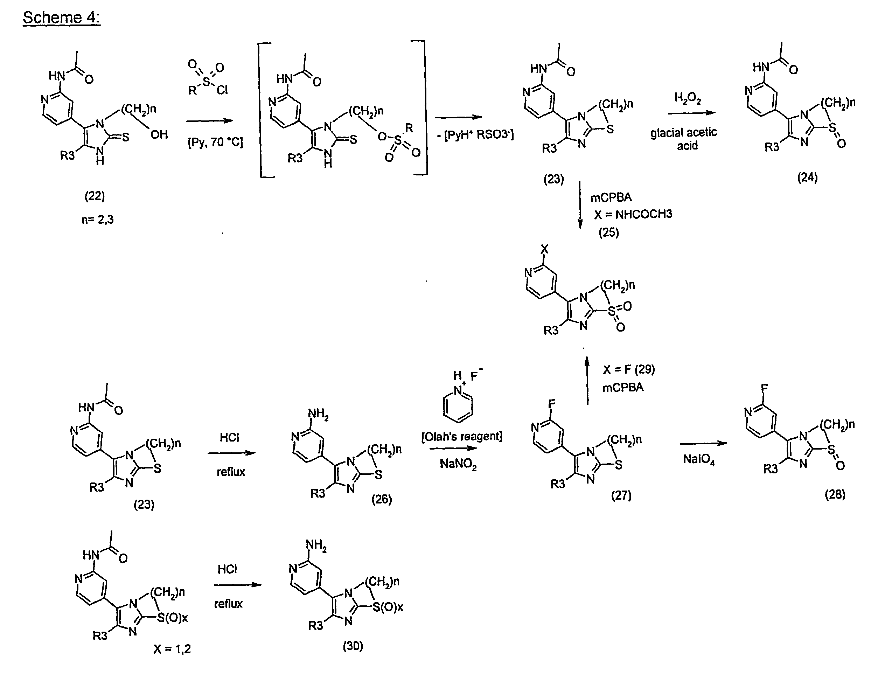

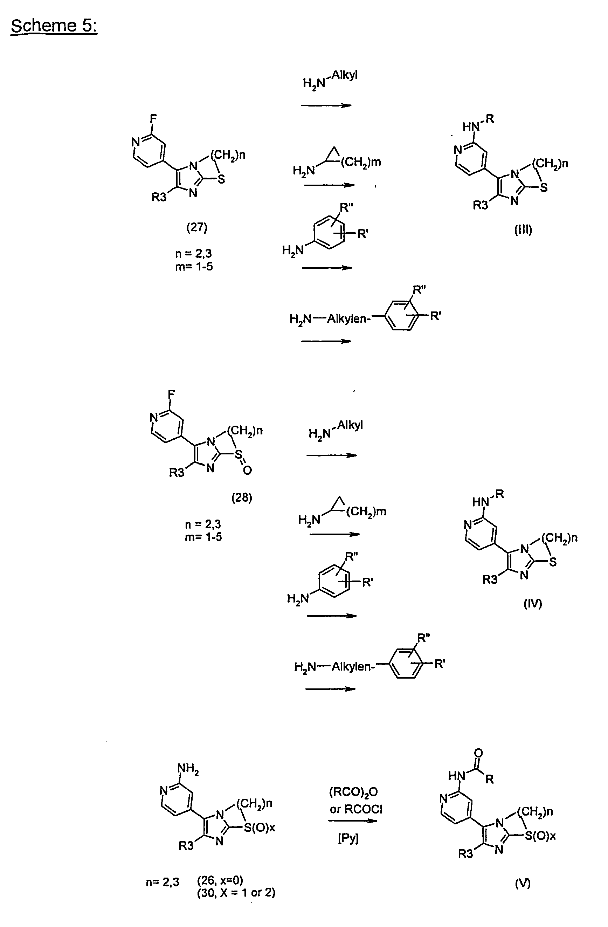

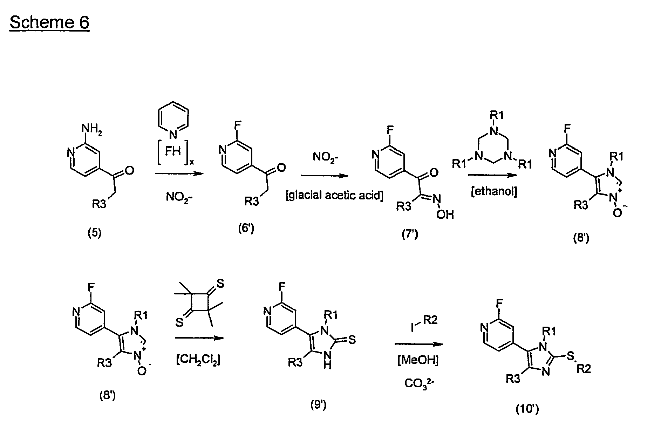

## Claims

1. A 2-sulfinyl-substituted or 2-sulfonyl-substituted imidazole compound of the formula I 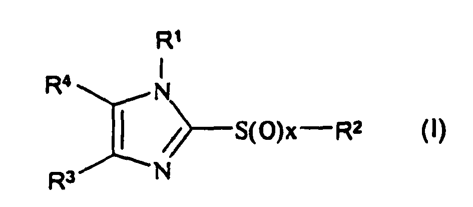 in which
R¹ is selected from:
a) C₁-C₆-alkyl which is optionally substituted by one or two groups independently of one another selected from
hydroxy;
C₁-C₄-alkoxy,
C₂-C₆-alkenyloxy;
C₂-C₆-alkynyloxy;
CO₂H;
CO₂-C₁-C₆-alkyl;
CN;
halogen;
C₁-C₆-alkyl- SO₃;
C₁-C₆-alkylthio;
NR⁷R⁸, wherein R⁷ and R⁸ are independently of one another H, C₁-C₆-alkyl or hydroxy-C₁-C₆-alkyl;
R⁹CONR¹⁰, R⁹ and R¹⁰ are independently of one another H or C₁-C₆-alkyl;
a nonaromatic heterocyclic radical having 5 or 6 ring atoms and 1 or 2 heteroatoms, selected independently of one another from N, O and S, which heterocyclic radical may be substituted by 1, 2, 3 or 4 C₁-C₆-alkyl groups;
b) 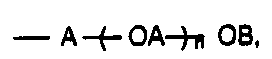 in which
A is -CH₂CH₂, -CH₂CH₂CH₂-, 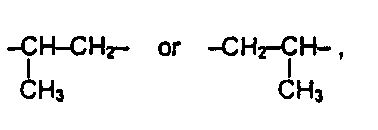 n is 1, 2, 3, 4 or 5, and B is H or C₁-C₄-alkyl;
c) C₁-C₆-oxoalkyl;
d) C₂-C₆-alkenyl
e) C₃-C₇-cycloalkyl;
f) (C₃-C₇-cycloalkyl)-C₁-C₆-alkyl;
g) aryl which is optionally substituted by one or more halogen atoms or a C₁-C₄-alkylsulfanyl group;
h) aminoaryl, where the amino group is optionally substituted by one or two C₁-C₄-alkyl groups,
i) aryl-C₁-C₆-alkyl or
j) an aromatic or nonaromatic heterocyclic radical having 5 or 6 ring atoms and 1 or 2 heteroatoms selected independently of one another from N, O and S, which heterocyclic radical is optionally substituted by 1, 2, 3 or 4 C₁-C₄-alkyl groups, an aryl or aryl-C₁-C₄-alkyl group;
R² is selected from:
a) C₁-C₆-alkyl,
b) phenyl-C₁-C₄-alkyl, where the phenyl group may have one or two substituents which are selected independently of one another from C₁-C₄-alkyl, halogen, C₁-C₄-alkylsulfanyl, C₁-C₄-alkylsulfinyl and C₁-C₄-alkylsulfonyl,
c) C₂-C₆-alkenyl,
d) C₂-C₆-alkenyl which is substituted by one or two halogen atoms and/or phenyl groups, where the phenyl group may be substituted independently by one or two C₁-C₄-alkyl or halogen atoms,
e) C₂-C₆-alkynyl,
f) C₂-C₆-alkynyl which is substituted by a phenyl group which may be optionally substituted by one or two C₁-C₄-alkyl or halogen atoms,
g) C₁-C₆-alkyl which is substituted by C₁-C₄-alkylsuffanyl, C₁-C₄-alkylsulfinyl or C₁-C₄-alkylsulfonyl;
h) C₁-C₆-alkyl which is substituted by -CO-Het wherein Het is a nonaromatic heterocyclic radical having 5 or 6 ring atoms and 1 or 2 heteroatoms which are selected independently of one another from N, O, and S;
i) phenyl; and
j) phenyl which has one or two substituents which are selected independently of one another from C₁-C₄-alkyl, halogen, C₁-C₄-alkylsuffanyl, C₁-C₄-alkylsulfinyl or C₁-C₄-alkylsulfonyl; or
R¹ and R² together are -CH₂CH₂- or -CH₂CH₂CH₂-.
x is 1 or 2,
R³ is phenyl which is substituted by 1 or 2 halogen atoms or trifluoromethyl groups,
R⁴ is 4-pyridyl which has one or two substituents which are selected independently of one another from
a) amino;
b) C₁-C₈-alkylamino;
c) phenylamino, where the phenyl group may be substituted by C₁-C₄-alkyl, C₁-C₄-alkoxy, halogen, or CF₃;
d) phenyl-C₁-C₄-alkylamino;
e) C₃-C₇-cycloalkylamino;
f) (C₃-C₇-cycloalkyl)-C₁-C₈-alkylamino; and
g) R⁵CONR⁶-, wherein R⁵ is selected from
H;
C₁-C₈-alkyl;
phenyl which may have one or two substituents which are selected independently of one another from C₁-C₄-alkyl, C₁-C₄-alkoxy and halogen;
C₃-C₇-cycloalkyl;
CF₃;
C₂-C₆-alkenyl;
phenyl-C₁-C₈-alkyl wherein the phenyl group may have one or two substituents which are selected independently of one another from C₁-C₄-alkyl, C₁-C₄-alkoxy or halogen;
phenyl-C₂-C₆-alkenyl wherein the phenyl group may have one or two substituents which are selected independently of one another from C₁-C₄-alkyl, C₁-C₄-alkoxy or halogen; and
phenyl-NR¹¹-, wherein R¹¹ is H or C₁-C₄-alkyl and the phenyl group may have one or two substituents which are selected independently of one another from C₁-C₄-alkyl, C₁-C₄-alkoxy or halogen;
R⁶ is H, C₁-C₄-alkyl, phenyl or benzyl, and
the optical isomers and physiologically tolerated salts thereof.

2. A compound as claimed in claim 1 of the formula I in which R¹ is selected from:
C₁-C₆-alkyl which is optionally substituted by one or two hydroxy or C₁-C₄-alkoxy groups or one nonaromatic heterocyclic radical having 5 or 6 ring atoms and 1 or 2 heteroatoms which are selected independently of one another from N, O and S, 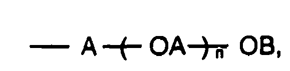 in which
A is -CH₂CH₂-, -CH₂CH₂CH₂-, 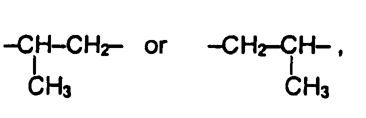
n is 1, 2, 3, 4 or 5, and B is H or C₁-C₄-alkyl,
C₂-C₆-alkenyl,
C₃-C₇-cycloalkyl,
amino-C₁-C₆-alkyl, where the amino group is optionally substituted by one or two C₁-C₄-alkyl groups,
an aromatic or nonaromatic heterocyclic radical having 5 or 6 ring atoms and 1 or 2 heteroatoms selected independently of one another from N, O and S, which heterocyclic radical is optionally substituted by 1, 2, 3 or 4 C₁-C₄-alkyl groups.

3. A compound as claimed in claim 1 of the formula I in which R¹ is selected from:
C₁-C₆-alkyl which is optionally substituted by one or two hydroxy or C₁-C₄-alkoxy groups or one nonaromatic heterocyclic radical having 5 or 6 ring atoms and 1 or 2 heteroatoms which are selected independently of one another from N, O and S, or 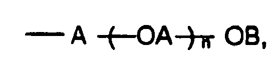 in which
A is -CH₂CH₂-, -CH₂CH₂CH₂-, 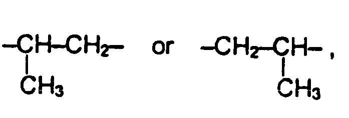
n is 1, 2, 3, 4 or 5 and B is H or C₁-C₄-alkyl.

4. A compound of the formula I as claimed in any of the preceding claims, where R¹ is C₁-C₄-alkyl, C₁-C₄-alkoxy-C₂-C₄-alkyl or hydroxy-C₂-C₄-alkyl.

5. A compound as claimed in claim 4, where R¹ is C₁-C₃-alkyl, hydroxy-C₂-C₃-alkyl or methoxy-C₂-C₃-alkyl.

6. A compound of the formula I as claimed in any of the preceding claims, in which R² is C₁-C₆-alkyl, phenyl-C₁-C₄-alkyl, phenyl or phenyl which has one or two substituents which are selected independently of one another from C₁-C₄-alkyl and halogen.

7. A compound of the formula I as claimed in any of the preceding claims, in which R² is C₁-C₆-alkyl or phenyl-C₁-C₄-alkyl.

8. A compound of the formula I as claimed in any of the preceding claims, in which R³ is 4-fluorophenyl or 3-trifluoromethylphenyl.

9. A compound as claimed in claim 1, where R⁴ is 4-pyridyl which is substituted by amino, C₁-C₈-alkylamino, phenylamino, phenyl-C₁-C₄-alkylamino, C₃-C₇-cycloalkylamino or R⁵CONR⁶-, where R⁵ and R⁶ have the meanings indicated in claim 1.

10. A compound of the formula I as claimed in any of the preceding claims, in which R⁴ is 4-pyridyl which is substituted by C₁-C₈-alkylamino, phenylamino, phenyl-C₁-C₄-alkylamino, C₃-C₇-cycloalkylamino or R⁵CONR⁶-, where R⁵ is C₁-C₈alkyl, C₃-C₇-cycloalkyl, phenyl-C₁-C₈-alkyl, vinyl or styryl, and R⁶ is H or C₁-C₄-alkyl.

11. A compound as claimed in any of the preceding claims, where the 4-pyridyl group is substituted in position 2.

12. A compound as claimed in claim 1, namely
cyclohexyl-{4-[5-(4-fluorophenyl)-2-methanesulfinyl-3-methyl-3H-imidazol-4-yl]pyridin-2-yl}amine
cyclopentyl-{4-[5-(4-fluorophenyl)-2-methanesulfinyl-3-methyl-3H-imidazol-4-yl]pyridin-2-yl}amine
cycloheptyl-{4-[5-(4-fluorophenyl)-2-methanesulfinyl-3-methyl-3H-imidazol-4-yl]pyridin-2-yl}amine
cyclopentyl-{4-[3-ethyl-5-(4-fluorophenyl)-2-methanesulfinyl-3H-imidazol-4-yl]pyridin-2-yl}amine
cyclohexyl-{4-[3-ethyl-5-(4-fluorophenyl)-2-methanesulfinyl-3H-imidazol-4-yl]pyridin-2-yl}amine
cycloheptyl-{4-[3-ethyl-5-(4-fluorophenyl)-2-methanesulfinyl-3H-imidazol-4-yl]pyridin-2-yl}amine
cyclohexyl-{4-[5-(4-fluorophenyl)-2-methanesulfinyl-3-(2-methoxyethyl)-3H-imidazol-4-yl]pyridin-2-yl}amine,
cyclohexyl-{4-[5-(4-fluorophenyl)-2-methanesulfinyl-3-(3-methoxypropyl)-3H-imidazol-4-yl]pyridin-2-yl}amine
cyclohexyl-{4-[5-(4-fluorophenyl)-2-methanesulfonyl-3-(2-methoxyethyl)-3H-imidazol-4-yl]pyridin-2-yl}amine
cyclopentyl-{4-[5-(4-fluorophenyl)-2-methanesulfonyl-3-(2-methoxyethyl)-3H-imidazol-4-yl]pyridin-2-yl}amine,
cyclopentyl-{4-[5-{4-fluorophenyl}-2-methanesulfinyl-3-(3-methoxypropyl)-3H-imidazol-4-yl]pyridin-2-yl}amine
3-[5-(2-cyclopentylamino-pyridin-4-yl)-4-(4-fluorophenyl)-2-methanesulfinyl-imidazol-1-yl]propan-1-ol
cycloheptyl-{4-[5-(4-fluorophenyl)-2-methanesulfinyl-3-(3-methoxypropyl)-3H-imidazol-4-yl]pyridin-2-yl}amine
3-[5-(2-cyclohexylamino-pyridin-4-yl)-4-(4-fluorophenyl)-2-methanesulfinyl-imidazol-1-yl]propan-1-ol
3-[5-(2-cycloheptylamino-pyridin-4-yl)-4-(4-fluorophenyl)-2-methanesulfinyl-imidazol-1-yl]propan-1-ol
cycloheptyl-{4-[5-(4-fluorophenyl)-2-methanesulfinyl-3-(2-methoxyethyl)-3H-imidazol-4-yl]pyridin-2-yl}amine
{4-[5-(4-fluorophenyl)-2-methanesulfinyl-3-(2-methoxyethyl)-3H-imidazol-4-yl]pyridin-2-yl}isopropylamine
{4-[5-(4-fluorophenyl)-2-methanesulfinyl-3-(3-methoxypropyl)-3H-imidazol-4-yl]pyridin-2-yl}isopropylamine
{4-[5-(4-fluorophenyl)-2-methanesulfonyl-3-(3-methoxypropyl)-3H-imidazol-4-yl]pyridin-2-yl}isopropylamine
{4-[5-(4-fluorophenyl)-2-methanesulfinyl-3-(3-methoxypropyl)-3H-imidazol-4-yl]pyridin-2-yl}(1-phenylethyl)amine
{4-[5-(4-fluorophenyl)-2-methanesulfinyl-3-(2-methoxyethyl)-3H-imidazol-4-yl]pyridin-2-yl}(1-phenylethyl)amine
N-{4-[5-(4-fluorophenyl)-2-methanesulfinyl-3-(3-methoxypropyl)-3H-imidazol-4-yl]pyridin-2-yl}acetamide
N-{4-[3-ethyl-5-(4-fluorophenyl)-2-methanesulfinyl-3H-imidazol-4-yl]pyridin-2-yl}acetamide
N-{4-[3-ethyl-5-(4-fluorophenyl)-2-methanesulfonyl-3H-imidazol-4-yl]pyridin-2-yl}acetamide
N-{4-[5-(4-fluoraphenyl)-2-methanesulfinyl-3-(2-methoxyethyl)-3H-imidazol-4-yl]pyridin-2-yl}acetamide
N-{4-[5-(4-fluorophenyl)-2-methanesulfonyl-3-(2-methoxyethyl)-3H-imidazol-4-yl]pyridin-2-yl}acetamide
{4-[5-(4-fluorophenyl)-2-methanesulfinyl-3-methyl-3H-imidazol-4-yl]pyridin-2-yl}(1-phenylethyl)amine
{4-[5-(4-fluorophenyl)-methanesulfonyl-3-methyl-3H-imidazol-4-yl]pyridin-2-yl}(1-phenylethyl)amine
N-{4-[5-(4-fluorophenyl)-2-methanesulfinyl-3-methyl-3H-imidazol-4-yl]pyridin-2-yl}acetamide
N-{4-[5-(4-fluorophenyl)-2-methanesulfonyl-3-methyl-3H-imidazol-4-yl]pyridin-2-yl}acetamide
cyclohexyl-{4-[5-(4-fluorophenyl)-2-methanesulfinyl-2-(2-methoxypropyl)-3H-imidazol-4-yl]pyridin-2-yl}amine
(+)-N-{4-[5-(4-fluorophenyl)-2-methanesulfinyl-3-methyl-3H-imidazol-4-yl]pyridin-2-yl}acetamide or
(-)-N-{4-[5-(4-fluorophenyl)-2-methanesulfinyl-3-methyl-3H-imidazol-4-yl]pyridin-2-yl}acetamide

13. A compound as claimed in claim 1, wherein R¹ is selected from:
a) C₁-C₆-alkyl which is optionally substituted by one or two hydroxy or C₁-C₄-alkoxy groups, or a nonaromatic heterocyclic radical having 5 or 6 ring atoms and 1 or 2 heteroatoms, which are selected independently of one another from N, O and S,
b) 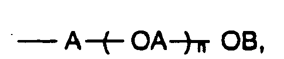 in which
A is -CH₂CH₂-, -CH₂CH₂CH₂-, 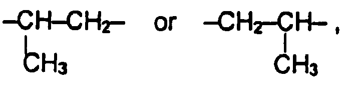 n is 1, 2, 3, 4 or 5, and B is H or C₁-C₄-alkyl,
c) C₂-C₆-alkenyl,
d) C₃-C₆-cycloalkyl,
e) aryl which is optionally substituted by one or more halogen atoms or one C₁-C₄-alkylsulfanyl group,
f) amino-C₁-C₄-alkyl, where the amino group is optionally substituted by one or two C₁-C₄-alkyl groups,
g) aminoaryl, where the amino group is optionally substituted by one or two C₁-C₄-alkyl groups,
h) aryl-C₁-C₄-alkyl or
i) an aromatic or nonaromatic heterocyclic radical having 5 or 6 ring atoms and 1 or 2 heteroatoms selected independently of one another from N, O and S, which heterocyclic radical is optionally substituted by 1, 2, 3 or 4 C₁-C₄-alkyl groups, one aryl oraryl-C₁-C₄-alkyl group,
R² is selected from:
a) C₁-C₆-alkyl,
b) phenyl-C₁-C₄-alkyl, where the phenyl group may have one or two substituents which are selected independently of one another from C₁-C₄-alkyl, halogen, C₁-C₄-alkylsulfanyl, C₁-C₄-alkylsuffinyl and C₁-C₄-alkylsulfonyl,
c) C₂-C₆-alkenyl,
d) C₂-C₆-alkenyl which is substituted by one or two halogen atoms and/or phenyl groups, where the phenyl group may be substituted independently by one or two C₁-C₄-alkyl or halogen atoms,
e) C₂-C₆-alkynyl,
f) C₂-C₆-alkynyl which is substituted by a phenyl group which may optionally be substituted by one or two C₁-C₄-alkyl or halogen atoms,
g) C₁-C₆-alkyl which is substituted by a nonaromatic heterocyclic radical having 5 or 6 ring atoms and 1 or 2 heteroatoms which are selected independently of one another from N, O, and S, C₁-C₄-alkylsulfanyl, C₁-C₄-alkylsulfinyl or C₁-C₄-alkylsulfonyl,
h) phenyl and
i) phenyl which has one or two substituents which are selected independently of one another from C₁-C₄-alkyl, halogen, C₁-C₄-alkylsulfanyl, C₁-C₄-alkylsulfinyl and C₁-C₄-alkylsulfonyl, or
R¹ and R² together are -CH₂CH₂- or -CH₂CH₂CH₂-,
x is 1 or 2,
R³ is phenyl which is substituted by 1 or 2 halogen atoms or trifluoromethyl groups,
R⁴ is 4-pyridyl which has one or two substituents which are selected independently of one another from
a) amino,
b) C₁-C₄-alkylamino,
c) phenylamino, where the phenyl group may be substituted by C₁-C₄-alkyl, C₁-C₄-alkoxy, halogen, or CF₃,
d) phenyl-C₁-C₄-alkylamino,
e) C₃-C₇-cycloalkylamino, and
f) R⁵CONR⁶-,
R⁵ is C₁-C₄-alkyl, phenyl which may have one or two substituents which are selected independently of one another from C₁-C₄-alkyl, C₁-C₄-alkoxy and halogen, or is C₃-C₇-cycloalkyl, and
R⁶ is H, C₁-C₄-alkyl, phenyl or benzyl, and
the optical isomers and physiologically tolerated salts thereof.

14. A compound as claimed in claim 1, wherein
R¹ is selected from C₁-C₆-alkyl which may be substituted with hydroxy or C₁-C₆-alkoxy groups;
R² is C₁-C₆-alkyl;
R³ is phenyl which is substituted by 1 or 2 halogen atoms; and
R⁴ is 4-pyridyl which has one or two substituents which are selected independently of one another from amino;
C₁-C₈-alkylamino;
phenylamino where the phenyl group may be substituted by C₁-C₄-alkyl or C₁-C₄-alkoxy;
phenyl-C₁-C₄-alkylamino where the phenyl group may be substituted by C₁-C₄-alkyl or C₁-C₄-alkoxy;
C₃-C₇-cycloalkylamino; and
R⁵CONR⁶-, wherein R⁵ is selected from
H;
C₁-C₈-alkyl;
C₃-C₇-cycloalkyl;
phenyl-C₁-C₈-alkyl, wherein the phenyl group may have one or two substituents which are selected independently of one another from C₁-C₄-alkyl, C₁-C₄-alkoxy or halogen; and
C₂-C₈-alkenyl; and
R⁸ is H, C₁-C₄-alkyl, phenyl or benzyl.

15. A 2-thio-substituted imidazole compound of the formula II 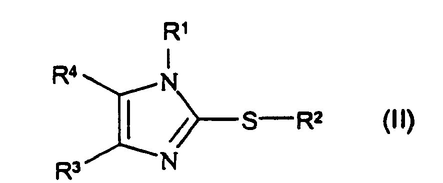 wherein R¹, R² and R³ are as defined in any one of claims 1 to 8 and R⁴ is 4-pyridyl which has one or two substituents which are selected independently of one another from C₅-C₈-alkylamino;
(C₃-C₇-cycloalkyl)-C₁-C₈-alkylamino;
C₃-C₇-cycloalkylamino; and
R⁵CONR⁶, wherein R⁵ is selected from
H;
C₅-C₈-alkyl;
cycloheptyl;
CF₃;
C₂-C₆-alkenyl;
phenyl-C₁-C₈-alkyl wherein the phenyl group may have one or two substituents which are selected independently of one another from C₁-C₄-alkyl, C₁-C₄-alkoxy or halogen;
phenyl-C₂-C₈-alkenyl wherein the phenyl group may have one or two substituents which are selected independently of one another from C₁-C₄-alkyl, C₁-C₄-alkoxy or halogen;
phenyl-NR¹¹-, wherein R¹¹ is H or C₁-C₄-alkyl and the phenyl group may have one or two substituents which are selected independently of one another from C₁-C₄-alkyl, C₁-C₄-alkoxy or halogen; and
R⁶ is H, C₁-C₄-alkyl, phenyl or benzyl, and
the optical isomers and physiologically tolerated salts thereof.

16. A 2-thiosubstituted imidazole compound of the formula II 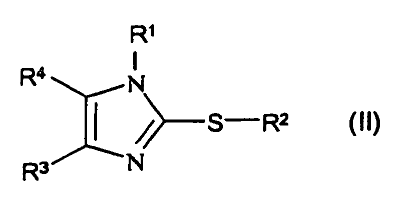 wherein R¹ is selected from:
a) C₁-C₆-alkyl which is substituted by one or two groups independently of one another selected from
CO₂H;
CO₂-C₁-C₆-alkyl;
CN;
halogen;
C₁-C₆-alkyl- SO₃;
NR⁷R⁸, wherein R⁷ is H and R⁸ is hydroxy-C₁-C₆-alkyl or R⁷ and R⁸ are hydroxyl-C₁-C₆-alkyl;
b) 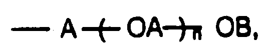 in which
A is -CH₂CH₂-, -CH₂CH₂CH₂-, 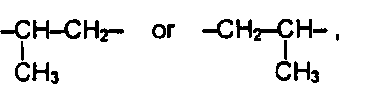 n is 1, 2, 3, 4 or 5, and B is C₁-C₄-alkyl;
c) C₁-C₆-oxoalkyl;
d) cycloheptyl; and
e) (C₃-C₇-cycloalkyl)-C₁-C₆-alkyl;
R² is selected from:
a) C₁-C₆-alkyl,
b) phenyl-C₁-C₄-alkyl, where the phenyl group may have one or two substituents which are selected independently of one another from C₁-C₄-alkyl, halogen, C₁-C₄-alkylsulfanyl, C₁-C₄-alkylsulfinyl and C₁-C₄-alkylsulfonyl,
c) C₂-C₆-alkenyl,
d) C₂-C₆-alkenyl which is substituted by one or two halogen atoms and/or phenyl groups, where the phenyl group may be substituted independently by one or two C₁-C₄-alkyl or halogen atoms,
e) C₂-C₆-alkynyl,
f) C₂-C₆-alkynyl which is substituted by a phenyl group which may be optionally substituted by one or two C₁-C₄-alkyl or halogen atoms,
g) C₁-C₆-alkyl which is substituted by C₁-C₄-alkylsulfanyl, C₁-C₄-alkylsulfinyl or C₁-C₄-alkylsulfonyl;
h) C₁-C₆-alkyl which is substituted by -CO-Het wherein Het is a nonaromatic heterocyclic radical having 5 or 6 ring atoms and 1 or 2 heteroatoms which are selected independently of one another from N, O, and S;
i) phenyl; and
j) phenyl which has one or two substituents which are selected independently of one another from C₁-C₄-alkyl, halogen, C₁-C₄-alkylsulfanyl, C₁-C₄-alkylsulfinyl or C₁-C₄-alkylsulfonyl; or
R¹ and R² together are -CH₂CH₂- or -CH₂CH₂CH₂-,
x is 1 or 2,
R³ is phenyl which is substituted by 1 or 2 halogen atoms or trifluoromethyl groups,
R⁴ is 4-pyridyl which has one or two substituents which are selected independently of one another from
a) amino;
b) C₁-C₈-alkylamino;
c) phenylamino, where the phenyl group may be substituted by C₁-C₄-alkyl, C₁-C₄-alkoxy, halogen, or CF₃;
d) phenyl-C₁-C₄-alkylamino;
e) C₃-C₇-cycloalkylamino;
f) (C₃-C₇-cycloalkyl)-C₁-C₆-alkylamino; and
g) R⁵CONR⁶-, wherein R⁵ is selected from
H;
C₁-C₈-alkyl;
phenyl which may have one or two substituents which are selected independently of one another from C₁-C₄-alkyl, C₁-C₄-alkoxy and halogen;
C₃-C₇-cycloalkyl;
CF₃;
C₂-C₆-alkenyl;
phenyl-C₁-C₈-alkyl wherein the phenyl group may have one or two substituents which are selected independently of one another from C₁-C₄-alkyl, C₁-C₄-alkoxy or halogen;
phenyl-C₂-C₆-alkenyl wherein the phenyl group may have one or two substituents which are selected independently of one another from C₁-C₄-alkyl, C₁-C₄-alkoxy or halogen; and
phenyl-NR¹¹-, wherein R¹¹ is H or C₁-C₄-alkyl and the phenyl group may have one or two substituents which are selected independently of one another from C₁-C₄-alkyl, C₁-C₄-alkoxy or halogen;
R⁶ is H, C₁-C₄-alkyl, phenyl or benzyl, and
R², R³ and R⁴ are as defined in any one of claims 1 or 6 to 11 and
the optical isomers and the physiologically tolerable salts thereof.

17. A pharmaceutical composition comprising at least one compound as claimed in any of claims 1 to 16, where appropriate together with one or more pharmaceutically acceptable carriers and/or additives.

18. The use of at least one of the compounds as claimed in any of claims 1 to 16 for producing a pharmaceutical composition for the treatment of disorders associated with an impairment of the immune system.

19. A compound as defined in any of claims 1 to 16 for use in a method for the treatment of disorders associated with an impairment of the immune system.

## Patentansprüche

1. 2-Sulfinyl-substituierte oder 2-sulfonyl-substituierte Imidazolverbindung der Formel I 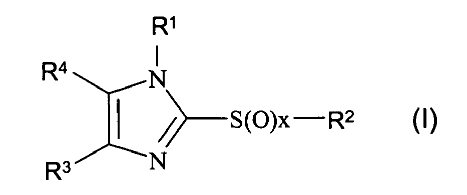 worin
R¹ ausgewählt ist unter:
a) C₁-C₆-Alkyl, welches gegebenenfalls mit einer oder zwei Gruppen substituiert ist, die unabhängig voneinander ausgewählt sind unter
Hydroxy;
C₁-C₄-Alkoxy;
C₂-C₆-Alkenyloxy;
C₂-C₆-Alkinyloxy;
CO₂H;
CO₂-C₁-C₆-Alkyl;
CN;
Halogen;
C₁-C₆-Alkyl- SO₃;
C₁-C₆-Alkylthio;
NR⁷R⁸, worin R⁷ und R⁸ unabhängig voneinander für H, C₁-C₆-Alkyl oder Hydroxy-C₁-C₆-Alkyl stehen;
R⁹CONR¹⁰, R⁹ und R¹⁰, die unabhängig voneinander für H oder C₁-C₆-Alkyl oder einen nichtaromatischen heterocyclischen Rest mit 5 oder 6 Ringatomen und 1 oder 2 Heteroatomen stehen, welche unabhängig voneinander ausgewählt sind unter N, O und S, wobei der heterocyclische Rest mit 1, 2, 3 oder 4 C₁-C₆-Alkylgruppen substituiert sein kann;
b) 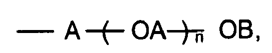 worin
A für -CH₂CH₂-, -CH₂CH₂CH₂-, 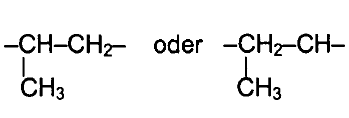 steht,
n 1, 2, 3, 4 oder 5 ist, und B für H oder C₁-C₄-Alkyl steht;
c) C₁-C₆-Oxoalkyl ;
d) C₂-C₆-Alkenyl
e) C₃-C₇-Cycloalkyl;
f) (C₃-C₇-Cycloalkyl)-C₁-C₆-alkyl;
g) Aryl, welches gegebenenfalls mit einem oder mehreren Halogenatomen oder einer C₁-C₄-Alkylsulfanylgruppe substituiert ist;
h) Aminoaryl, wobei die Aminogruppe gegebenenfalls mit einer oder zwei C₁-C₄-Alkylgruppen substituiert ist,
i) Aryl-C₁-C₆-alkyl, oder
j) einem aromatischen oder nichtaromatischen heterocyclischen Rest mit 5 oder 6 Ringatomen und 1 oder 2 Heteroatomen, welche unabhängig voneinander ausgewählt sind unter N, O und S, wobei der heterocyclische Rest gegebenenfalls mit 1, 2, 3 oder 4 C₁-C₄-Alkylgruppen, einer Aryl- oder einer Aryl-C₁-C₄-alkylgruppe substituiert ist;
R² ausgewählt ist unter:
a) C₁-C₆-Alkyl,
b) Phenyl-C₁-C₄-alkyl, wobei die Phenylgruppe ein oder zwei Substituenten besitzen kann, die unabhängig voneinander ausgewählt sind unter C₁-C₄-Alkyl, Halogen, C₁-C₄-Alkylsulfanyl, C₁-C₄-Alkylsulfinyl und C₁-C₄-Alkylsulfonyl,
c) C₂-C₆-Alkenyl,
d) C₂-C₆-Alkenyl, welches mit einem oder zwei Halogenatomen und/oder Phenylgruppen substituiert ist, wobei jede Phenylgruppe jeweils mit einem oder zwei C₁-C₄-Alkyl- oder Halogenatomen substituiert sein kann,
e) C₂-C₆-Alkinyl,
f) C₂-C₆-Alkinyl, welches mit einer Phenylgruppe substituiert ist, die gegebenenfalls mit einem oder zwei C₁-C₄-Alkyl oder Halogenatomen substituiert ist,
g) C₁-C₆-Alkyl, welches mit C₁-C₄-Alkylsulfanyl , C₁-C₄-Alkylsulfinyl oder C₁-C₄-Alkylsulfonyl substituiert ist;
h) C₁-C₆-Alkyl, welches mit -CO-Het substituiert ist, wobei Het für einen nichtaromatischen heterocyclischen Rest mit 5 oder 6 Ringatomen und 1 oder 2 Heteroatomen steht, die unabhängig voneinander ausgewählt sind unter N, O, und S;
i) Phenyl; und
j) Phenyl, welches einen oder zwei Substituenten besitzt, welche unabhängig voneinander ausgewählt sind unter C₁-C₄-Alkyl, Halogen, C₁-C₄-Alkylsulfanyl, C₁-C₄-Alkylsulfinyl oder C₁-C₄-Alkylsulfonyl; oder
R¹ und R² gemeinsam für -CH₂CH₂- oder -CH₂CH₂CH₂- stehen,
x 1 oder 2 ist,
R³ für Phenyl steht, welches mit 1 oder 2 Halogenatomen oder Trifluormethylgruppen substituiert ist,
R⁴ für 4-Pyridyl steht, welches einen oder zwei Substituenten besitzt, welche unabhängig voneinander ausgewählt sind unter
a) Amino;
b) C₁-C₈-Alkylamino;
c) Phenylamino, wobei die Phenylgruppe mi C₁-C₄-Alkyl, C₁-C₄-Alkoxy, Halogen oder CF₃ substituiert sein kann;
d) Phenyl-C₁-C₄-alkylamino;
e) C₃-C₇-Cycloalkylamino;
f) (C₃-C₇-Cycloalkyl)-C₁-C₈-alkylamino; und
g) R⁵CONR⁶-, wobei R⁵ ausgewählt ist unter
H;
C₁-C₈-Alkyl;
Phenyl, welches einen oder zwei Substituenten besitzen kann, welche unabhängig voneinander ausgewählt sind unter C₁-C₄-Alkyl, C₁-C₄-Alkoxy und Halogen;
C₃-C₇-Cycloalkyl;
CF₃;
C₂-C₆-Alkenyl;
Phenyl-C₁-C₈-alkyl, wobei die Phenylgruppe einen oder zwei Substituenten besitzen kann, welche unabhängig voneinander ausgewählt sind unter C₁-C₄-Alkyl, C₁-C₄-Alkoxy oder Halogen;
Phenyl-C₂-C₆-alkenyl, wobei die Phenylgruppe einen oder zwei Substituenten besitzen kann, welche unabhängig voneinander ausgewählt sind unter C₁-C₄-Alkyl, C₁-C₄-Alkoxy oder Halogen; und
Phenyl-NR¹¹-, wobei R¹¹ für H oder C₁-C₄-Alkyl steht und die Phenylgruppe einen oder zwei Substituenten besitzen kann, welche unabhängig voneinander ausgewählt sind unter C₁-C₄-Alkyl, C₁-C₄-Alkoxy oder Halogen;
R⁶ für H, C₁-C₄-Alkyl, Phenyl oder Benzyl steht, und
die optischen Isomeren und physiologisch verträglichen Salze davon.

2. Verbindung nach Anspruch 1 der Formel I, worin R¹ ausgewählt ist unter:
C₁-C₆-Alkyl, welches gegebenenfalls mit einem oder zwei Hydroxy- oder C₁-C₄-Alkoxygruppen oder einem nichtaromatischen heterocyclischen Rest mit 5 oder 6 Ringatomen und 1 oder 2 Heteroatomen substituiert ist, welche unabhängig voneinander ausgewählt sind unter N, O und S, 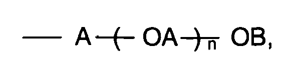 worin
A für -CH₂CH₂-, -CH₂CH₂CH₂-, 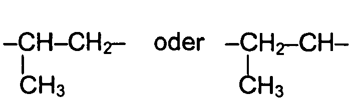 steht,
n 1, 2, 3, 4 oder 5 ist und B für H oder C₁-C₄-Alkyl steht,
C₂-C₆-Alkenyl,
C₃-C₇-Cycloalkyl,
Amino-C₁-C₆-alkyl, wobei die Aminogruppe gegebenenfalls mit einem oder zwei C₁-C₄-Alkylgruppen substituiert ist,
einem aromatischen oder nichtaromatischen heterocyclischen Radikal mit 5 oder 6 Ringatomen und 1 oder 2 Heteroatomen, welche unabhängig voneinander ausgewählt sind unter N, O und S, wobei das heterocyclische Radikal gegebenenfalls mit 1, 2, 3 oder 4 C₁-C₄-Alkylgruppen substituiert ist.

3. Verbindung nach Anspruch 1 der Formel I, worin R¹ ausgewählt ist unter:
C₁-C₆-Alkyl, welches gegebenenfalls mit einem oder zwei Hydroxy- oder C₁-C₄-Alkoxygruppen oder einem nichtaromatischen heterocyclischen Radikal mit 5 oder 6 Ringatomen und 1 oder 2 Heteroatomen substituiert ist, welche unabhängig voneinander ausgewählt sind unter N, O und S, oder 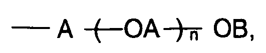 worin
A für -CH₂CH₂-, -CH₂CH₂CH₂-, 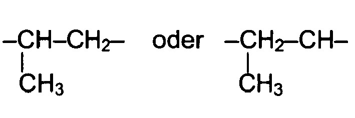 steht,
n 1, 2, 3, 4 oder 5 ist und B für H oder C₁-C₄-Alkyl steht.

4. Verbindung der Formel I nach einem der vorhergehenden Ansprüche, wobei R¹ für C₁-C₄-Alkyl, C₁-C₄-Alkoxy-C₂-C₄-alkyl oder Hydroxy-C₂-C₄-alkyl steht.

5. Verbindung nach Anspruch 4, wobei R¹ für C₁-C₃-Alkyl, Hydroxy-C₂-C₃-alkyl oder Methoxy-C₂-C₃-alkyl steht.

6. Verbindung der Formel I nach einem der vorhergehenden Ansprüche, wobei R² für C₁-C₆-Alkyl, Phenyl-C₁-C₄-alkyl, Phenyl oder Phenyl, welches ein oder zwei Substituenten besitzt, die unabhängig voneinander ausgewählt sind unter C₁-C₄-Alkyl und Halogen, steht.

7. Verbindung der Formel I nach einem der vorhergehenden Ansprüche, wobei R² für C₁-C₆-Alkyl oder Phenyl-C₁-C₄-alkyl steht.

8. Verbindung der Formel I nach einem der vorhergehenden Ansprüche, wobei R³ für 4-Fluorphenyl oder 3-Trifluormethylphenyl steht.

9. Verbindung nach Anspruch 1, wobei R⁴ für 4-Pyridyl steht, welches mit Amino, C₁-C₈-Alkylamino, Phenylamino, Phenyl-C₁-C₄-alkylamino, C₃-C₇-Cycloalkylamino oder R⁵CONR⁶- substituiert ist, wobei R⁵ und R⁶ die in Anspruch 1 beschriebenen Bedeutungen haben.

10. Verbindung der Formel I nach einem der vorhergehenden Ansprüche, wobei R⁴ für 4-Pyridyl steht, welches mit C₁-C₈-Alkylamino, Phenylamino, Phenyl-C₁-C₄-alkylamino, C₃-C₇-Cycloalkylamino oder R⁵CONR⁶- substituiert ist, wobei R⁵ für C₁-C₈-Alkyl, C₃-C₇-Cycloalkyl, Phenyl-C₁-C₈-alkyl, Vinyl oder Styryl und R⁶ für H oder C₁-C₄-Alkyl steht.

11. Verbindung nach einem der vorhergehenden Ansprüche, wobei die 4-Pyridylgruppe in Position 2 substituiert ist.

12. Verbindung nach Anspruch 1, nämlich
Cyclohexyl-{4-[5-(4-fluorphenyl)-2-methansulfinyl-3-methyl-3H-imidazol-4-yl]pyridin-2-yl}amin
Cyclopentyl-{4-[5-(4-fluorphenyl)-2-methansulfinyl-3-methyl-3H-imidazol-4-yl]pyridin-2-yl}amin
Cycloheptyl-{4-[5-(4-fluorphenyl)-2-methansulfinyl-3-methyl-3H-imidazol-4-yl]pyridin-2-yl}amin
Cyclopentyl-{4-[3-ethyl-5-(4-fluorphenyl)-2-methansulfinyl-3H-imidazol-4-yl]pyridin-2-yl}amin
Cyclohexyl-{4-[3-ethyl-5-(4-fluorphenyl)-2-methansulfinyl-3H-imidazol-4-yl]pyridin-2-yl}amin
Cycloheptyl-{4-[3-ethyl-5-(4-fluorphenyl)-2-methansulfinyl-3H-imidazol-4-yl]pyridin-2-yl}amin
Cyclohexyl-{4-[5-(4-fluorphenyl)-2-methansulfinyl-3-(2-methoxyethyl)-3H-imidazol-4-yl]pyridin-2-yl}amin,
Cyclohexyl-{4-[5-(4-fluorphenyl)-2-methansulfinyl-3-(3-methoxypropyl)-3H-imidazol-4-yl]pyridin-2-yl}amin
Cyclohexyl-{4-[5-(4-fluorphenyl)-2-methansulfonyl-3-(2-methoxyethyl)-3H-imidazol-4-yl]pyridin-2-yl}amin
Cyclopentyl-{4-[5-(4-fluorphenyl)-2-methansulfinyl-3-(2-methoxyethyl)-3H-imidazol-4-yl]pyridin-2-yl}amin,
Cyclopentyl-{4-[5-(4-fluorphenyl)-2-methansulfinyl-3-(3-methoxypropyl)-3H-imidazol-4-yl]pyridin-2-yl}amin
3-[5-(2-Cyclopentylamino-pyridin-4-yl)-4-(4-fluorphenyl)-2-methansulfinyl-imidazol-1-yl]propan-1-ol
Cycloheptyl-{4-[5-(4-fluorphenyl)-2-methansulfinyl-3-(3-methoxypropyl)-3H-imidazol-4-yl]pyridin-2-yl}amin
3-[5-(2-Cyclohexylamino-pyridin-4-yl)-4-(4-fluorphenyl)-2-methansulfinyl-imidazol-1-yl]propan-1-ol
3-[5-(2-Cycloheptylamino-pyridin-4-yl)-4-(4-fluorphenyl)-2-methansulfinyl-imidazol-1-yl]propan-1-ol
Cycloheptyl-{4-[5-(4-fluorphenyl)-2-methansulfinyl-3-(2-methoxyethyl)-3H-imidazol-4-yl]pyridin-2-yl}amin
{4-[5-(4-Fluorphenyl)-2-methansulfinyl-3-(2-methoxyethyl)-3H-imidazol-4-yl]pyridin-2-yl}isopropylamin
{4-[5-(4-Fluorphenyl)-2-methansulfinyl-3-(3-methoxypropyl)-3H-imidazol-4-yl]pyridin-2-yl}isopropylamin
{4-[5-(4-Fluorphenyl)-2-methansulfonyl-3-(3-methoxypropyl)-3H-imidazol-4-yl]pyridin-2-yl}isopropylamin
{4-[5-(4-Fluorphenyl)-2-methansulfinyl-3-(3-methoxypropyl)-3H-imidazol-4-yl]pyridin-2-yl}(1-phenylethyl)amin
{4-[5-(4-Fluorphenyl)-2-methansulfinyl-3-(2-methoxyethyl)-3H-imidazol-4-yl]pyridin-2-yl}(1-phenylethyl)amin
N-{4-[5-(4-Fluorphenyl)-2-methansulfinyl-3-(3-methoxypropyl)-3H-imidazol-4-yl]pyridin-2-yl}acetamid
N-{4-[3-Ethyl-5-(4-fluorphenyl)-2-methansulfinyl-3H-imidazol-4-yl]pyridin-2-yl}acetamid
N-{4-[3-Ethyl-5-(4-fluorphenyl)-2-methansulfonyl-3H-imidazol-4-yl]pyridin-2-yl}acetamid
N-{4-[5-(4-Fluorphenyl)-2-methansulfinyl-3-(2-methoxyethyl)-3H-imidazol-4-yl]pyridin-2-yl}acetamid
N-{4-[5-(4-Fluorphenyl)-2-methansulfonyl-3-(2-methoxyethyl)-3H-imidazol-4-yl]pyridin-2-yl}acetamid
{4-[5-(4-Fluorphenyl)-2-methansulfinyl-3-methyl-3H-imidazol-4-yl]pyridin-2-yl}(1-phenylethyl)amin
{4-[5-(4-Fluorphenyl)-2-methansulfonyl-3-methyl-3H-imidazol-4-yl]pyridin-2-yl}(1-phenylethyl)amin
N-{4-[5-(4-Fluorphenyl)-2-methansulfinyl-3-methyl-3H-imidazol-4-yl]pyridin-2-yl}acetamid
N-{4-[5-(4-Fluorphenyl)-2-methansulfonyl-3-methyl-3H-imidazol-4-yl]pyridin-2-yl}acetamid
Cyclohexyl-{4-[5-(4-fluorphenyl)-2-methansulfinyl-2-(2-methoxypropyl)-3H-imidazol-4-yl]pyridin-2-yl}amin
(+)-N-{4-[5-(4-Fluorphenyl)-2-methansulfinyl-3-methyl-3H-imidazol-4-yl]pyridin-2-yl}acetamid oder
(-)-N-{4-[5-(4-Fluorphenyl)-2-methansulfinyl-3-methyl-3H-imidazol-4-yl]pyridin-2-yl}acetamid.

13. Verbindung nach Anspruch 1, wobei R¹ ausgewählt ist unter:
a) C₁-C₆-Alkyl, welches gegebenenfalls mit einem oder zwei Hydroxy- oder C₁-C₄-Alkoxygruppen oder einem nichtaromatischen heterocyclischen Rest mit 5 oder 6 Ringatomen und 1 oder 2 Heteroatomen substituiert ist, welche unabhängig voneinander ausgewählt sind unter N, O und S,
b) 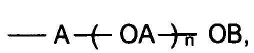 worin
A für -CH₂CH₂-, -CH₂CH₂CH₂-, 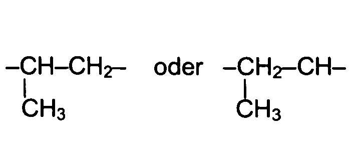 steht,
n 1, 2, 3, 4 oder 5 ist und B für H oder C₁-C₄-Alkyl steht,
c) C₂-C₆-Alkenyl,
d) C₃-C₆-Cycloalkyl,
e) Aryl, welches gegebenenfalls mit einem oder mehreren Halogenatomen oder einer C₁-C₄-Alkylsulfanylgruppe substituiert ist,
f) Amino-C₁-C₄-alkyl, wobei die Aminogruppe gegebenenfalls mit einem oder zwei C₁-C₄-Alkylgruppen substituiert ist,
g) Aminoaryl, wobei die Aminogruppe gegebenenfalls mit einem oder zwei C₁-C₄-Alkylgruppen substituiert ist,
h) Aryl-C₁-C₄-Alkyl oder
i) einem aromatischen oder nichtaromatischen heterocyclischen Rest mit 5 oder 6 Ringatomen und 1 oder 2 Heteroatomen, die unabhängig voneinander ausgewählt sind unter N, O und S, wobei der heterocyclische Rest gegebenenfalls mit 1, 2, 3 oder 4 C₁-C₄-Alkylgruppen, einer Aryl- oder Aryl-C₁-C₄-Alkylgruppe substituiert ist,
R² ausgewählt ist unter:
a) C₁-C₆-Alkyl,
b) Phenyl-C₁-C₄-alkyl, wobei die Phenylgruppe einen oder zwei Substituenten besitzen kann, welche unabhängig voneinander ausgewählt sind unter C₁-C₄-Alkyl, Halogen, C₁-C₄-Alkylsulfanyl, C₁-C₄-Alkylsulfinyl und C₁-C₄-Alkylsulfonyl,
c) C₂-C₆-Alkenyl,
d) C₂-C₆-Alkenyl, welches mit einem oder zwei Halogenatomen und/oder Phenylgruppen substituiert ist, wobei jede Phenylgruppe jeweils mit einem oder zwei C₁-C₄-Alkyl oder Halogenatomen substituiert sein kann,
e) C₂-C₆-Alkinyl,
f) C₂-C₆-Alkinyl, welches mit einer Phenylgruppe substituiert ist, die gegebenenfalls mit einem oder zwei C₁-C₄-Alkyl- oder Halogenatomen substituiert sein kann,
g) C₁-C₆-Alkyl, welches mit einem nichtaromatischen heterocyclischen Rest mit 5 oder 6 Ringatomen und 1 oder 2 Heteroatomen, die unabhängig voneinander ausgewählt sind unter N, O, und S, C₁-C₄-Alkylsulfanyl, C₁-C₄-Alkylsulfinyl oder C₁-C₄-Alkylsulfonyl , substituiert ist,
h) Phenyl, und
i) Phenyl, welches einen oder zwei Substituenten besitzt, welche unabhängig voneinander ausgewählt sind unter C₁-C₄-Alkyl, Halogen, C₁-C₄-Alkylsulfanyl, C₁-C₄-Alkylsulfinyl und C₁-C₄-Alkylsulfonyl, oder
R¹ und R² gemeinsam für -CH₂CH₂- oder -CH₂CH₂CH₂- stehen,
x 1 oder 2 ist,
R³ für Phenyl steht, welches mit 1 oder 2 Halogenatomen oder Trifluormethylgruppen substituiert ist,
R⁴ für 4-Pyridyl steht, welches einen oder zwei Substituenten besitzt, welche unabhängig voneinander ausgewählt sind unter
a) Amino,
b) C₁-C₄-Alkylamino,
c) Phenylamino, wobei die Phenylgruppe mit C₁-C₄-Alkyl, C₁-C₄-Alkoxy, Halogen oder CF₃ substituiert sein kann,
d) Phenyl-C₁-C₄-alkylamino,
e) C₃-C₇-Cycloalkylamino, und
f) R⁵CONR⁶-,
R⁵ für C₁-C₄-Alkyl, Phenyl, welches einen oder zwei Substituenten besitzt, die unabhängig voneinander ausgewählt sind unter C₁-C₄-Alkyl, C₁-C₄-Alkoxy und Halogen, oder für C₃-C₇-Cycloalkyl steht, und
R⁶ für H, C₁-C₄-Alkyl, Phenyl oder Benzyl steht, und
die optischen Isomeren und physiologisch verträglichen Salze davon.

14. Verbindung nach Anspruch 1, wobei
R¹ ausgewählt ist unter C₁-C₆-Alkyl, welches mit Hydroxy- oder C₁-C₆-Alkoxygruppen substituiert sein kann;
R² für C₁-C₆-Alkyl steht;
R³ für Phenyl steht, welches mit 1 oder 2 Halogenatomen substituiert ist; und
R⁴ für 4-Pyridyl steht, welches einen oder zwei Substituenten besitzt, welche unabhängig voneinander ausgewählt sind unter
Amino;
C₁-C₈-Alkylamino;
Phenylamino, wobei die Phenylgruppe mit C₁-C₄-Alkyl oder C₁-C₄-Alkoxy substituiert sein kann;
Phenyl-C₁-C₄-alkylamino, wobei die Phenylgruppe mit C₁-C₄-Alkyl oder C₁-C₄-Alkoxy substituiert sein kann;
C₃-C₇-Cycloalkylamino; und
R⁵CONR⁶-, wobei R⁵ ausgewählt ist unter
H;
C₁-C₈-Alkyl;
C₃-C₇-Cycloalkyl;
Phenyl-C₁-C₈-alkyl, wobei die Phenylgruppe einen oder zwei Substituenten besitzen kann, welche unabhängig voneinander ausgewählt sind unter C₁-C₄-Alkyl, C₁-C₄-Alkoxy oder Halogen; und
C₂-C₆-Alkenyl; und
R⁶ für H, C₁-C₄-Alkyl, Phenyl oder Benzyl steht.

15. 2-Thio-substituierte Imidazolverbindung der Formel II 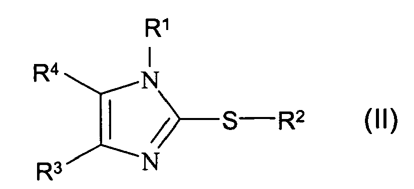 worin R¹, R² und R³ die in einem der Ansprüche 1 bis 8 definierten Bedeutungen besitzen und R⁴ für 4-Pyridyl steht, welches einen oder zwei Substituenten besitzt, welche unabhängig voneinander ausgewählt sind unter
C₅-C₈-Alkylamino;
(C₃-C₇-Cycloalkyl)-C₁-C₈-alkylamino;
C₃-C₇-Cycloalkylamino; und
R⁵CONR⁶, wobei R⁵ ausgewählt ist unter
H;
C₅-C₈-Alkyl;
Cycloheptyl;
CF₃;
C₂-C₆-Alkenyl;
Phenyl-C₁-C₈-alkyl, wobei die Phenylgruppe einen oder zwei Substituenten besitzen kann, welche unabhängig voneinander ausgewählt sind unter C₁-C₄-Alkyl, C₁-C₄-Alkoxy oder Halogen;
Phenyl-C₂-C₆-alkenyl, wobei die Phenylgruppe einen oder zwei Substituenten besitzen kann, welche unabhängig voneinander ausgewählt sind unter C₁-C₄-Alkyl, C₁-C₄-Alkoxy oder Halogen;
Phenyl-NR¹¹-, wobei R¹¹ für H oder C₁-C₄-Alkyl steht und die Phenylgruppe einen oder zwei Substituenten besitzen kann, welche unabhängig voneinander ausgewählt sind unter C₁-C₄-Alkyl, C₁-C₄-Alkoxy oder Halogen; und
R⁶ für H, C₁-C₄-Alkyl, Phenyl oder Benzyl steht, und
die optischen Isomere und physiologisch verträglichen Salze davon.

16. 2-Thiosubstituierte Imidazolverbindung der Formel II 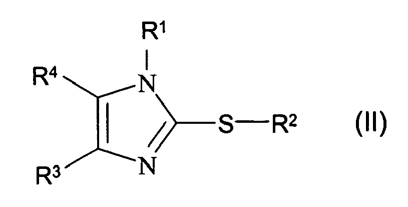 wobei R¹ ausgewählt ist unter:
a) C₁-C₆-Alkyl, welches mit einer oder zwei Gruppen substituiert ist, die unabhängig voneinander ausgewählt sind unter
CO₂H;
CO₂-C₁-C₆-Alkyl;
CN;
Halogen;
C₁-C₆-Alkyl- SO₃;
NR⁷R⁸, wobei R⁷ für H und R⁸ für Hydroxy-C₁-C₆-alkyl steht, oder R⁷ und
R⁸ für Hydroxyl-C₁-C₆-alkyl stehen;
b) 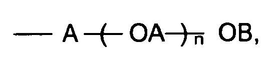 worin
A für -CH₂CH₂-, -CH₂CH₂CH₂-, 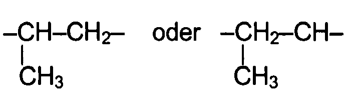 steht,
n 1, 2, 3, 4 oder 5 ist und B für C₁-C₄-Alkyl steht;
c) C₁-C₆-Oxoalkyl;
d) Cycloheptyl; und
e) (C₃-C₇-Cycloalkyl)-C₁-C₆-alkyl;
R² ausgewählt ist unter:
a) C₁-C₆-Alkyl,
b) Phenyl-C₁-C₄-alkyl, wobei die Phenylgruppe einen oder zwei Substituenten besitzen kann, welche unabhängig voneinander ausgewählt sind unter C₁-C₄-Alkyl, Halogen, C₁-C₄-Alkylsulfanyl, C₁-C₄-Alkylsulfinyl und C₁-C₄-Alkylsulfonyl,
c) C₂-C₆-Alkenyl,
d) C₂-C₆-Alkenyl, welches mit einem oder zwei Halogenatomen und/oder Phenylgruppen substituiert ist, wobei jede Phenylgruppe jeweils mit einem oder zwei C₁-C₄-Alkyl oder Halogenatomen substituiert sein kann,
e) C₂-C₆-Alkinyl,
f) C₂-C₆-Alkinyl, welches mit einer Phenylgruppe substituiert ist, welche gegebenenfalls mit einem oder zwei C₁-C₄-Alkyl- oder Halogenatomen substituiert sein kann,
g) C₁-C₆-Alkyl, welches mit C₁-C₄-Alkylsulfanyl, C₁-C₄-Alkylsulfinyl oder C₁-C₄-Alkylsulfonyl substituiert ist;
h) C₁-C₆-Alkyl, welches mit -CO-Het substituiert ist, wobei Het für einen nichtaromatischen heterocyclischen Rest mit 5 oder 6 Ringatomen und 1 oder 2 Heteroatomen steht, welche unabhängig voneinander ausgewählt sind unter N, O, und S;
i) Phenyl; und
j) Phenyl, welches einen oder zwei Substituenten besitzt, welche unabhängig voneinander ausgewählt sind unter C₁-C₄-Alkyl, Halogen, C₁-C₄-Alkylsulfanyl, C₁-C₄-Alkylsulfinyl oder C₁-C₄-Alkylsulfonyl; oder
R¹ und R² gemeinsam für -CH₂CH₂- oder -CH₂CH₂CH₂- stehen,
x 1 oder 2 ist,
R³ für Phenyl steht, welches mit 1 oder 2 Halogenatomen oder Trifluormethylgruppen substituiert ist,
R⁴ für 4-Pyridyl steht, welches einen oder zwei Substituenten besitzt, welche unabhängig voneinander ausgewählt sind unter
a) Amino;
b) C₁-C₈-Alkylamino;
c) Phenylamino, wobei die Phenylgruppe mit C₁-C₄-Alkyl, C₁-C₄-Alkoxy, Halogen oder CF₃ substituiert sein kann;
d) Phenyl-C₁-C₄-alkylamino;
e) C₃-C₇-Cycloalkylamino;
f) (C₃-C₇-Cycloalkyl)-C₁-C₈-alkylamino; und
g) R⁵CONR⁶-, wobei R⁵ ausgewählt ist unter
H;
C₁-C₈-Alkyl;
Phenyl, welches einen oder zwei Substituenten besitzen kann, welche unabhängig voneinander ausgewählt sind unter C₁-C₄-Alkyl, C₁-C₄-Alkoxy und Halogen;
C₃-C₇-Cycloalkyl;
CF₃;
C₂-C₆-Alkenyl;
Phenyl-C₁-C₈-Alkyl, wobei die Phenylgruppe einen oder zwei Substituenten besitzen kann, welche unabhängig voneinander ausgewählt sind unter C₁-C₄-Alkyl, C₁-C₄-Alkoxy oder Halogen;
Phenyl-C₂-C₆-Alkenyl, wobei die Phenylgruppe einen oder zwei Substituenten besitzen kann. welche unabhängig voneinander ausgewählt sind unter C₁-C₄-Alkyl, C₁-C₄-Alkoxy oder Halogen; und
Phenyl-NR¹¹-, wobei R¹¹ für H oder C₁-C₄-Alkyl steht und die Phenylgruppe eine oder zwei Substituenten besitzen kann, welche unabhängig voneinander ausgewählt sind unter C₁-C₄-Alkyl, C₁-C₄-Alkoxy oder Halogen;
R⁶ für H, C₁-C₄-Alkyl, Phenyl oder Benzyl steht, und
R², R³ und R⁴ die in einem der Ansprüche 1 oder 6 bis 11 definierten Bedeutungen besitzen, und
die optischen Isomeren und die physiologisch verträglichen Salze davon.

17. Pharmazeutische Zusammensetzung, umfassend wenigstens eine Verbindung nach einem der Ansprüche 1 bis 16, gegebenenfalls gemeinsam mit einem oder mehreren pharmazeutisch akzeptablen Trägern und/oder Additiven.

18. Verwendung von zumindest einer der Verbindungen nach einem der Ansprüche 1 bis 16 zur Herstellung einer pharmazeutischen Zusammensetzung für die Behandlung von Funktionsstörungen in Verbindung mit einer Immunsystemschwäche.

19. Verbindung nach einem der Ansprüche 1 bis 16 zur Verwendung in einem Verfahren zur Behandlung von Funktionsstörungen in Verbindung mit einer Immunsystemschwäche.

## Revendications

1. Composé imidazole 2-sulfinyl-substitué ou 2-sulfonyl-substitué de formule I 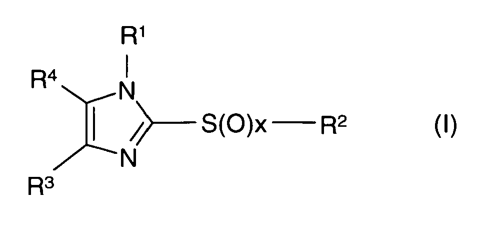 où
R¹ est sélectionné parmi :
a) alkyle en C₁-C₆ facultativement substitué par un ou deux groupes choisis indépendamment parmi
hydroxy ;
alcoxy en C₁-C₄ ;
(alcényle en C₂-C₆)oxy ;
(alcynyle en C₂-C₆)oxy ;
CO₂H ;
CO₂-alkyle en C₁-C₆ ;
CN ;
halogène ;
alkyle en C₁-C₆-SO₃ ;
(alkyle en C₁-C₆)thio ;
NR⁷R⁸, où R⁷ et R⁸ sont indépendamment l'un de l'autre H, alkyle en C₁-C₆ ou hydroxyalkyle en C₁-C₆ ;
R⁹CONR¹⁰, R⁹ et R¹⁰ sont indépendamment l'un de l'autre H ou alkyle en C₁-C₆ ;
un radical hétérocyclique non aromatique ayant 5 ou 6 atomes cycliques et un ou deux hétéroatomes, sélectionnés indépendamment l'un de l'autre parmi N, O et S, lequel radical hétérocyclique peut être substitué par 1, 2, 3 ou 4 groupes alkyle en C₁-C₆,
b) 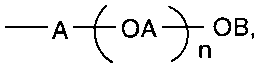 où
A est -CH₂CH₂- , -CH₂CH₂CH₂-, 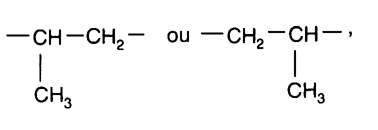 n est 1, 2, 3, 4 ou 5 et B est H ou alkyle en C₁-C₄ ;
c) oxoalkyle en C₁-C₆ ;
d) alcényle en C₂-C₆ ;
e) cycloalkyle en C₃-C₇ ;
f) (cycloalkyle en C₃-C₇)-alkyle en C₁-C₆ ;
g) aryle facultativement substitué par un ou plusieurs atomes d'halogène ou un groupe (alkyle en C₁-C₄)sulfanyle ;
h) aminoaryle, où le groupe amino est facultativement substitué par un ou deux groupes alkyle en C₁-C₄,
i) arylalkyle en C₁-C₆ ou
j) un radical hétérocyclique aromatique ou non aromatique ayant 5 ou 6 atomes cycliques et 1 ou 2 hétéroatomes sélectionnés indépendamment l'un de l'autre parmi N, O et S, lequel radical hétérocyclique est facultativement substitué par 1, 2, 3 ou 4 groupes alkyle en C₁-C₄, un groupe aryle ou un groupe arylalkyle en C₁-C₄ ;
R² est sélectionné parmi :
a) alkyle en C₁-C₆,
b) phénylalkyle en C₁-C₄, où le groupe phényle peut avoir un ou deux substituants sélectionnés indépendamment l'un de l'autre parmi alkyle en C₁-C₄, halogène, (alkyle en C₁-C₄)sulfanyle, (alkyle en C₁-C₄)sulfinyle et (alkyle en C₁-C₄)sulfonyle,
c) alcényle en C₂-C₆,
d) alcényle en C₂-C₆ substitué par un ou deux atomes d'halogène et/ou groupes phényle, où le groupe phényle peut être substitué indépendamment par un ou deux alkyles en C₁-C₄ ou atomes d'halogène,
e) alcynyle en C₂-C₆,
f) alcynyle en C₂-C₆ substitué par un groupe phényle qui peut être facultativement substitué par un ou deux alkyles en C₁-C₄ ou atomes d'halogène,
g) alkyle en C₁-C₆ substitué par (alkyle en C₁-C₄)sulfanyle, (alkyle en C₁-C₄)sulfinyle ou (alkyle en C₁-C₄)sulfonyle ;
h) alkyle en C₁-C₆ substitué par -CO-Het où Het est un radical hétérocyclique non aromatique ayant 5 ou 6 atomes cycliques et 1 ou 2 hétéroatomes sélectionnés indépendamment l'un de l'autre parmi N, O et S ;
i) phényle ; et
j) phényle qui a un ou deux substituants sélectionnés indépendamment l'un de l'autre parmi alkyle en C₁-C₄, halogène, (alkyle en C₁-C₄)-sulfanyle, (alkyle en C₁-C₄)sulfinyle ou (alkyle en C₁-C₄)sulfonyle ; ou
R¹ et R² ensemble sont -CH₂CH₂- ou -CH₂CH₂CH₂-,
x est 1 ou 2,
R³ est phényle substitué par un ou deux atomes d'halogène ou groupes trifluorométhyle,
R⁴ est 4-pyridyle qui a un ou deux substituants sélectionnés indépendamment l'un de l'autre parmi
a) amino ;
b) (alkyle en C₁-C₆)amino ;
c) phénylamino, où le groupe phényle peut être substitué par alkyle en C₁-C₄, alcoxy en C₁-C₄, halogène ou CF₃ ;
d) phényl(alkyle en C₁-C₄)amino ;
e) (cycloalkyle en C₃-C₇)amino ;
f) (cycloalkyle en C₃-C₇)-(alkyle en C₁-C₈)amino ; et
g) R⁵CONR⁶-, où R⁵ est sélectionné parmi
H ;
alkyle en C₁-C₈ ;
phényle qui peut avoir un ou deux substituants sélectionnés indépendamment l'un de l'autre parmi alkyle en C₁-C₄, alcoxy en C₁-C₄ et halogène ;
cycloalkyle en C₃-C₇ ;
CF₃ ;
alcényle en C₂-C₆ ;
phénylalkyle en C₁-C₈ où le groupe phényle peut avoir un ou deux substituants sélectionnés indépendamment l'un de l'autre parmi alkyle en C₁-C₄, alcoxy en C₁-C₄ ou halogène ;
phénylalcényle en C₂-C₆ où le groupe phényle peut avoir un ou deux substituants sélectionnés indépendamment l'un de l'autre parmi alkyle en C₁-C₄, alcoxy en C₁-C₄ ou halogène ; et
phényl-NR¹¹-, où R¹¹ est H ou alkyle en C₁-C₄ et le groupe phényle peut avoir un ou deux substituants sélectionnés indépendamment l'un de l'autre parmi alkyle en C₁-C₄, alcoxy en C₁-C₄ ou halogène ;
R⁶ est H, alkyle en C₁-C₄, phényle ou benzyle, et
isomères optiques et sels physiologiquement tolérés de celui-ci.

2. Composé selon la revendication 1 de formule I dans laquelle R¹ est sélectionné parmi :
alkyle en C₁-C₆ facultativement substitué par un ou deux groupes hydroxy ou alcoxy en C₁-C₄ ou un radical hétérocyclique non aromatique ayant 5 ou 6 atomes cycliques et un ou deux hétéroatomes sélectionnés indépendamment l'un de l'autre parmi N, O et S, 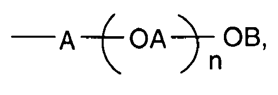 où
A est -CH₂CH₂- -CH₂CH₂CH₂-, 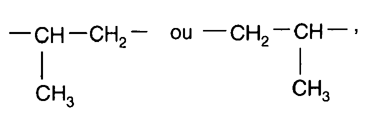
n est 1, 2, 3, 4 ou 5 et B est H ou alkyle en C₁-C₄,
alcényle en C₂-C₆,
cycloalkyle en C₃-C₇,
aminoalkyle en C₁-C₆, où le groupe amino est facultativement substitué par un ou deux groupes alkyle en C₁-C₄,
un radical hétérocyclique aromatique ou non aromatique ayant 5 ou 6 atomes cycliques et 1 ou 2 hétéroatomes sélectionnés indépendamment l'un de l'autre parmi N, O et S, lequel radical hétérocyclique est facultativement substitué par 1, 2, 3 ou 4 groupes alkyle en C₁-C₄.

3. Composé selon la revendication 1 de formule I dans laquelle R¹ est sélectionné parmi :
alkyle en C₁-C₆ facultativement substitué par un ou deux groupes hydroxy ou alcoxy en C₁-C₄ ou un radical hétérocyclique non aromatique ayant 5 ou 6 atomes cycliques et un ou deux hétéroatomes sélectionnés indépendamment l'un de l'autre parmi N, O et S,
ou 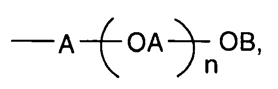 où
A est -CH₂CH₂-, -CH₂CH₂CH₂-, 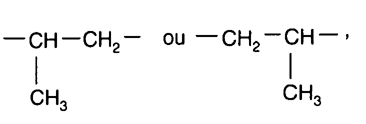
n est 1, 2, 3, 4 ou 5 et B est H ou alkyle en C₁-C₄.

4. Composé de formule I selon l'une quelconque des revendications précédentes, où R¹ est alkyle en C₁-C₄, (alcoxy en C₁-C₄)-alkyle en C₂-C₄ ou hydroxyalkyle en C₂-C_{4.}

5. Composé selon la revendication 4, où R¹ est alkyle en C¹-C³, hydroxyalkyle en C₂-C₃ ou méthoxyalkyle en C₂-C_{3.}

6. Composé de formule I selon l'une quelconque des revendications précédentes, où R² est acyle en C₁-C₆, phénylalkyle en C₁-C₄, phényle ou phényle qui a un ou deux substituants sélectionnés indépendamment l'un de l'autre parmi alkyle en C₁-C₄ et halogène.

7. Composé de formule I selon l'une quelconque des revendications précédentes, où R² est alkyle en C₁-C₆ ou phénylalkyle en C₁-C₄.

8. Composé de formule I selon l'une quelconque des revendications précédentes, où R³ est 4-fluorophényle ou 3-trifluorométhylphényle.

9. Composé selon la revendication 1, où R⁴ est 4-pyridyle substitué par amino, (alkyle en C₁-C₈)amino, phénylamino, phényl(alkyle en C₁-C₄)amino, (cycloalkyle en C₃-C₇)amino ou R⁵CONR⁶-, où R⁵ et R⁶ ont les significations indiquées à la revendication 1.

10. Composé de formule I selon l'une quelconque des revendications précédentes où R⁴ est 4-pyridyle substitué par (alkyle en C₁-C₈)amino, phénylamino, phényl(alkyle en C₁-C₄)amino, (cycloalkyle en C₃-C₇)amino ou R⁵CONR⁶-, où R⁵ est alkyle en C₁-C₈, cycloalkyle en C₃-C₇, phénylalkyle en C₁-C₈, vinyle ou styryle et R⁶ est H ou alkyle en C₁-C₄.

11. Composé selon l'une quelconque des revendications précédentes, où le groupe 4-pyridyle est substitué à la position 2.

12. Composé selon la revendication 1, à savoir
cyclohexyl-{4-[5-(4-fluorophényl)-2-méthanesulfinyl-3-méthyl-3H-imidazol-4-yl]pyridin-2-yl}amine,
cyclopentyl-{4-[5-(4-fluorophényl)-2-méthanesulfinyl-3-méthyl-3H-imidazol-4-yl]pyridin-2-yl}amine,
cycloheptyl-{4-[5-(4-fluorophényl)-2-méthanesulfinyl-3-méthyl-3H-imidazol-4-yl]pyridin-2-yl}amine,
cyclopentyl-{4-[3-éthyl-5-(4-fluorophényl)-2-méthanesulfinyl-3H-imidazol-4-yl]pyridin-2-yl}amine,
cyclohexyl-{4-[3-éthyl-5-(4-fluorophényl)-2-méthanesulfinyl-3H-imidazol-4-yl]pyridin-2-yl}amine,
cycloheptyl-{4-[3-éthyl-5-(4-fluorophényl)-2-méthanesulfinyl-3H-imidazol-4-yl]pyridin-2-yl}amine,
cyclohexyl-{4-[5-(4-fluorophényl)-2-méthanesulfinyl-3-(2-méthoxyéthyl)-3H-imidazol-4-yl]pyridin-2-yl}amine,
cyclohexyl-{4-[5-(4-fluorophényl)-2-méthanesulfinyl-3-(3-méthoxypropyl)-3H-imidazol-4-yl]pyridin-2-yl}amine,
cyclohexyl-{4-[5-(4-fluorophényl)-2-méthanesulfonyl-3-(2-méthoxyéthyl)-3H-imidazol-4-yl]pyridin-2-yl}amine,
cyclopentyl-{4-[5-(4-fluorophényl)-2-méthanesulfinyl-3-(2-méthoxyéthyl)-3H-imidazol-4-yl]pyridin-2-yl}amine,
cyclopentyl-{4-[5-(4-fluorophényl)-2-méthanesulfinyl-3-(3-méthoxypropyl)-3H-imidazol-4-yl]pyridin-2-yl}amine,
3-[5-(2-cyclopentylamino-pyridin-4-yl)-4-(4-fluorophényl)-2-méthanesulfinyl-imidazol-1-yl]propan-1-ol,
cycloheptyl-{4-[5-(4-fluorophényl)-2-méthanesulfinyl-3-(3-méthoxypropyl)-3H-imidazol-4-yl]pyridin-2-yl}amine,
3-[5-(2-cyclohexylamino-pyridin-4-yl)-4-(4-fluorophényl)-2-méthanesulfinyl-imidazol-1-yl]propan-1-ol,
3-[5-(2-cycloheptylamino-pyridin-4-yl)-4-(4-fluorophényl)-2-méthanesulfinyl-imidazol-1-yl]propan-1-ol,
cycloheptyl-{4-[5-(4-fluorophényl)-2-méthanesulfinyl-3-(2-méthoxyéthyl)-3H-imidazol-4-yl]pyridin-2-yl}amine,
{4-[5-(4-fluorophényl)-2-méthanesulfinyl-3-(2-méthoxyéthyl)-3H-imidazol-4-yl]pyridin-2-yl}isopropylamine,
{4-[5-(4-fluorophényl)-2-méthanesulfinyl-3-(3-méthoxypropyl)-3H-imidazol-4-yl]pyridin-2-yl}isopropylamine,
{4-[5-(4-fluorophényl)-2-méthanesulfonyl-3-(3-méthoxypropyl)-3H-imidazol-4-yl]pyridin-2-yl}isopropylamine,
{4-[5-(4-fluorophényl)-2-méthanesulfinyl-3-(3-méthoxypropyl)-3H-imidazol-4-yl]pyridin-2-yl}(1-phényléthyl)amine,
{4-[5-(4-fluorophényl)-2-méthanesulfinyl-3-(2-méthoxyéthyl)-3H-imidazol-4-yl]pyridin-2-yl}(1-phényléthyl)amine,
N-{4-[5-(4-fluorophényl)-2-méthanesulfinyl-3-(3-méthoxypropyl)-3H-imidazol-4-yl]pyridin-2-yl}acétamide,
N-{4-[3-éthyl-5-(4-fluorophényl)-2-méthanesulfinyl-3H-imidazol-4-yl]-pyridin-2-yl}acétamide,
N-{4-[3-éthyl-5-(4-fluorophényl)-2-méthanesulfonyl-3H-imidazol-4-yl]-pyridin-2-yl}acétamide,
N-{4-[5-(4-fluorophényl)-2-méthanesulfinyl-3-(2-méthoxyéthyl)-3H-imidazol-4-yl]pyridin-2-yl}acétamide,
N-{4-[5-(4-fluorophényl)-2-méthanesulfonyl-3-(2-méthoxyéthyl)-3H-imidazol-4-yl]pyridin-2-yl}acétamide,
{4-[5-(4-fluorophényl)-2-méthanesulfinyl-3-méthyl-3H-imidazol-4-yl]-pyidin-2-yl}(1-phényléthyl)amine,
{4-[5-(4-fluorophényl)-2-méthanesulfonyl-3-méthyl-3H-imidazol-4-yl]-pyridin-2-yl}(1-phényléthyl)amine,
N-{4-[5-(4-fluorophényl)-2-méthanesulfinyl-3-méthyl-3H-imidazol-4-yl]-pyridin-2-yl)acétamide,
N-{4-[5-(4-fluorophényl)-2-méthanesulfonyl-3-méthyl-3H-imidazol-4-yl]-pyridin-2-yl}acétamide,
Cyclohexyl-{4-[5-(4-fluorophényl)-2-méthanesulfinyl-2-(2-méthoxypropyl)-3H-imidazol-4-yl]pyridin-2-yl}amine,
(+)-N-{4-[5-(4-fluorophényl)-2-méthanesulfinyl-3-méthyl-3H-imidazol-4-yl]pyridin-2-yl}acétamide ou
(-)-N-{4-[5-(4-fluorophényl)-2-méthanesulfinyl-3-méthyl-3H-imidazol-4-yl]pyridin-2-yl}acétamide.

13. Composé selon la revendication 1, où R¹ est sélectionné parmi :
a) alkyle en C₁-C₆ facultativement substitué par un ou deux groupes hydroxy ou alcoxy en C₁-C₄ ou un radical hétérocyclique non aromatique ayant 5 ou 6 atomes cycliques et un 1 ou 2 hétéroatomes, sélectionnés indépendamment l'un de l'autre parmi N, O et S,
b) 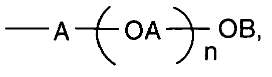 où
A est -CH₂CH₂ - , -CH₂CH₂CH₂ - , 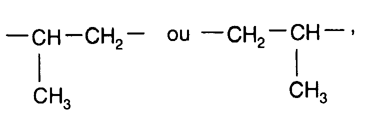 n est 1, 2, 3, 4 ou 5 et B est H ou alkyle en C₁-C₄;
c) alcényle en C₁-C₆ ;
d) cycloalkyle en C₃-C₆ ;
e) aryle facultativement substitué par un ou plusieurs atomes d'halogène ou un groupe (alkyle en C₁-C₄)sulfanyle,
f) aminoalkyle en C₁-C₄, où le groupe amino est facultativement substitué par un ou deux groupes alkyle en C₁-C₄,
g) aminoaryle, où le groupe amino est facultativement substitué par un ou deux groupes alkyle en C₁-C₄,
h) arylalkyle en C₁-C₄ ou
i) un radical hétérocyclique aromatique ou non aromatique ayant 5 ou 6 atomes cycliques et 1 ou 2 hétéroatomes sélectionnés indépendamment l'un de l'autre parmi N, O et S, lequel radical hétérocyclique est facultativement substitué par 1, 2, 3 ou 4 groupes alkyle en C₁-C₄, un groupe aryle ou arylalkyle en C₁-C₄ ;
R² est sélectionné parmi :
a) alkyle en C₁-C₆,
b) phénylalkyle en C₁-C₄, où le groupe phényle peut avoir un ou deux substituants sélectionnés indépendamment l'un de l'autre parmi alkyle en C₁-C₄, halogène, (alkyle en C₁-C₄)sulfanyle, (alkyle en C₁-C₄)sulfinyle et (alkyle en C₁-C₄)sulfonyle,
c) alcényle en C₂-C_{6,}
d) alcényle en C₂-C₆ substitué par un ou deux atomes d'halogène et/ou groupes phényle, où le groupe phényle peut être substitué indépendamment par un ou deux alkyles en C₁-C₄ ou atomes d'halogène,
e) alcynyle en C₂-C₆,
f) alcynyle en C₂-C₆ substitué par un groupe phényle qui peut être facultativement substitué par un ou deux alkyles en C₁-C₄ ou atomes d'halogène,
g) alkyle en C₁-C₆ substitué par un radical hétérocyclique non aromatique ayant 5 ou 6 atomes cycliques et 1 ou 2 hétéroatomes sélectionnés indépendamment l'un de l'autre parmi N, O et S ; et (alkyle en C₁-C₄)sulfanyle, (alkyle en C₁-C₄)sulflnyle ou (alkyle en C₁-C₄)sulfonyle ;
h) phényle ; et
i) phényle qui a un ou deux substituants sélectionnés indépendamment l'un de l'autre parmi alkyle en C₁-C₄, halogène, (alkyle en C₁-C₄)-sulfanyle, (alkyle en C₁-C₄)sulfinyle ou (alkyle en C₁-C₄)sulfonyle ; ou
R¹ et R² ensemble sont -CH₂CH₂- ou -CH₂CH₂CH₂-,
x est 1 ou 2,
R³ est phényle substitué par un ou deux atomes d'halogène ou groupes trifluorométhyle,
R⁴ est 4-pyridyle qui a un ou deux substituants sélectionnés indépendamment l'un de l'autre parmi
a) amino,
b) (alkyle en C₁-C₄)amino,
c) phénylamino, où le groupe phényle peut être substitué par alkyle en C₁-C₄, alcoxy en C₁-C₄, halogène ou CF₃,
d) phényl(alkyle en C₁-C₄)amino,
e) cycloalkyle en C₁-C₇-amino, et
f) R⁵CONR⁶-,
R⁵ est alkyle en C₁-C₄, phényle qui peut avoir un ou deux substituants sélectionnés indépendamment l'un de l'autre parmi alkyle en C₁-C₄, alcoxy en C₁-C₄ et halogène ou est cycloalkyle en C₃-C₇, et
R⁶ est H, alkyle en C₁-C₄, phényle ou benzyle, et
isomères optiques et sels physiologiquement tolérés de celui-ci.

14. Composé selon la revendication 1, où
R¹ est sélectionné parmi un alkyle en C₁-C₆ qui peut être substitué par des groupes hydroxy ou alcoxy en C₁-C₈ ;
R² est alkyle en C₁-C₆ ;
R³ est phényle substitué par 1 ou 2 atomes d'halogène ; et
R⁴ est 4-pyridyle qui a un ou deux substituants sélectionnés indépendamment l'un de l'autre parmi
amino ;
(alkyle en C₁-C₈)amino ;
phénylamino où le groupe phényle peut être substitué par alkyle en C₁-C₄ ou alcoxy en C₁-C₄ ;
phényl(alkyle en C₁-C₄)amino où le groupe phényle peut être substitué par alkyle en C₁-C₄ ou alcoxy en C₁-C₄ ;
(cycloalkyle en C₃-C₇)amino ; et
R⁵CONR⁶-, où R⁵ est sélectionné parmi
H;
alkyle en C₁-C₈ ;
cycloalkyle en C₃-C₇ ;
phénylalkyle en C₁-C₈, où le groupe phényle peut avoir un ou deux substituants sélectionnés indépendamment l'un de l'autre parmi alkyle en C₁-C₄, alcoxy en C₁-C₄ ou halogène ; et
alcényle en C₂-C₆ ; et
R⁸ est H, alkyle en C₁-C₄, phényle ou benzyle.

15. Composé imidazole 2-thio-substitué de formule II 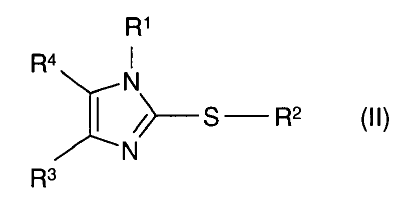 où R¹, R² et R³ sont tels que définis dans l'une quelconque des revendications 1 à 8 et R⁴ est 4-pyridyle qui a un ou deux substituants sélectionnés indépendamment l'un de l'autre parmi (alkyle en C₅-C₈)amino ;
(cycloalkyle en C₃-C₇)(alkyle en C₁-C₈)amino ;
(cycloalkyle en C₃-C₇)amino ; et
R⁵CONR⁶, où R⁵ est sélectionné parmi
H;
alkyle en C₁-C₈ ;
cycloheptyle ;
CF₃ ;
alcényle en C₂-C₆ ;
phénylalkyle en C₁-C₈ où le groupe phényle peut avoir un ou deux substituants sélectionnés indépendamment l'un de l'autre parmi alkyle en C₁-C₄, alcoxy en C₁-C₄ ou halogène ;
phénylalcényle en C₂-C₆ où le groupe phényle peut avoir un ou deux substituants sélectionnés indépendamment l'un de l'autre parmi alkyle en C₁-C₄, alcoxy en C₁-C₄ ou halogène ;
phényl-NR¹¹-, où R¹¹ est H ou alkyle en C₁-C₄ et le groupe phényle peut avoir un ou deux substituants sélectionnés indépendamment l'un de l'autre parmi alkyle en C₁-C₄, alcoxy en C₁-C₄ ou halogène ; et
R⁶ est H, alkyle en C₁-C₄, phényle ou benzyle, et
isomères optiques et sels physiologiquement tolérés de celui-ci.

16. Composé imidazole-thio-2-substitué de formule II 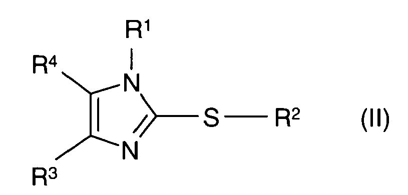 où R¹ est sélectionné parmi :
a) alkyle en C₁-C₆ substitué par un ou deux groupes sélectionnés indépendamment l'un de l'autre parmi
CO₂H;
CO₂-alkyle en C₁-C₆ ;
CN ;
halogène ;
alkyle en C₁-C₆-SO₃ ;
NR⁷R⁸, où R⁷ est H et R⁸ est hydroxyalkyle en C₁-C₆ ou R⁷ et R⁸ sont hydroxyalkyle en C₁-C₈ ;
b) 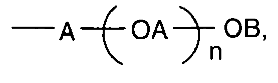 où
A est -CH₂CH₂ - , - CH₂CH₂CH₂ - , 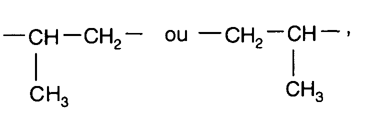 n est 1, 2, 3, 4 ou 5 et B est alkyle en C₁-C₄ ;
c) oxoalkyle en C₁-C₆ ;
d) cycloheptyle ; et
e) (cycloalkyle en C₃-C₇)alkyle en C₁-C₆ ;
R² est sélectionné parmi :
a) alkyle en C₁-C₆,
b) phénylalkyle en C₁-C₄, où le groupe phényle peut avoir un ou deux substituants sélectionnés indépendamment l'un de l'autre parmi alkyle en C₁-C₄, halogène, (alkyle en C₁-C₄)sulfanyle, (alkyle en C₁-C₄)sulfinyle et (alkyle en C₁-C₄)sulfonyle,
c) alcényle en C₂-C₆,
d) alcényle en C₂-C₆ substitué par un ou deux atomes d'halogène et/ou groupes phényle, où le groupe phényle peut être substitué indépendamment par un ou deux alkyles en C₁-C₄ ou atomes d'halogène,
e) alcynyle en C₂-C₆,
f) alcynyle en C₂-C₆ substitué par un groupe phényle qui peut être facultativement substitué par un ou deux alkyles en C₁-C₄ ou atomes d'halogène,
g) alkyle en C₁-C₆ substitué par (alkyle en C₁-C₄)sulfanyle, (alkyle en C₁-C₄)sulfinyle ou (alkyle en C₁-C₄)sulfonyle ;
h) alkyle en C₁-C₆ substitué par -CO-Het où Het est un radical hétérocyclique non aromatique ayant 5 ou 6 atomes cycliques et 1 ou 2 hétéroatomes sélectionnés indépendamment l'un de l'autre parmi N, O et S;
i) phényle ; et
j) phényle qui a un ou deux substituants sélectionnés indépendamment l'un de l'autre parmi alkyle en C₁-C₄, halogène, (alkyle en C₁-C₄)-sulfanyle, (alkyle en C₁-C₄)sulfinyle ou (alkyle en C₁-C₄)sulfonyle ; ou
R¹ et R² ensemble sont -CH₂CH₂- ou -CH₂CH₂CH₂-,
x est 1 ou 2,
R³ est phényle substitué par un ou deux atomes d'halogène ou groupes trifluorométhyle,
R⁴ est 4-pyridyle qui a un ou deux substituants sélectionnés indépendamment l'un de l'autre parmi
a) amino ;
b) (alkyle en C₁-C₆)amino ;
c) phénylamino, où le groupe phényle peut être substitué par alkyle en C₁-C₄, alcoxy en C₁-C₄, halogène ou CF₃ ;
d) phényl(alkyle en C₁-C₄)amino ;
e) (cycloalkyle en C₃-C₇)amino ;
f) (cycloalkyle en C₃-C₇)-(alkyle en C₁-C₈)amino ; et
g) R⁵CONR⁶-, où R⁵ est sélectionné parmi
H ;
alkyle en C₁-C₈ ;
phényle qui peut avoir un ou deux substituants sélectionnés indépendamment l'un de l'autre parmi alkyle en C₁-C₄, alcoxy en C₁-C₄ et halogène ;
cycloalkyle en C₃-C₇;
CF₃ ;
alcényle en C₂-C₆ ;
phénylalkyle en C₁-C₈ où le groupe phényle peut avoir un ou deux substituants sélectionnés indépendamment l'un de l'autre parmi alkyle en C₁-C₄, alcoxy en C₁-C₄ ou halogène ;
phénylalcényle en C₂-C₆ où le groupe phényle peut avoir un ou deux substituants sélectionnés indépendamment l'un de l'autre parmi alkyle en C₁-C₄, alcoxy en C₁-C₄ ou halogène ; et
phényl-NR¹¹-, où R¹¹ est H ou alkyle en C₁-C₄ et le groupe phényle peut avoir un ou deux substituants sélectionnés indépendamment l'un de l'autre parmi alkyle en C₁-C₄, alcoxy en C₁-C₄ ou halogène ;
R⁶ est H, alkyle en C₁-C₄, phényle ou benzyle, et
R², R³ et R⁴ sont tels que définis selon l'une quelconque des revendications 1 ou 6 à 11 et
isomères optiques et sels physiologiquement tolérables de celui-ci.

17. Composition pharmaceutique comprenant au moins un composé selon l'une quelconque des revendications 1 à 16, avec, si nécessaire un ou plusieurs véhicules et/ou additifs pharmaceutiquement acceptables.

18. Utilisation d'au moins un des composés selon l'une quelconque des revendications 1 à 16 pour la production d'une composition pharmaceutique destinée au traitement des troubles associés à un affaiblissement du système immunitaire.

19. Composé selon l'une quelconque des revendications 1 à 16 pour utilisation dans un procédé destiné au traitement des troubles associés à un affaiblissement du système immunitaire.
